# EUROPEAN PATENT SPECIFICATION

(11) **EP 3 999 053 B1**
(45) Date of publication and mention of the grant of the patent: **21.01.2026**
(21) Application number: 20839943.6
(22) Date of filing: 16.07.2020
(51) Int. Cl.: A61K 31/404, A61K 31/4985, A61P 35/00

(54) **METHOD OF TREATING NEOPLASTIC DISEASES WITH A CDC42-SPECIFIC INHIBITOR**
VERFAHREN ZUR BEHANDLUNG VON NEOPLASTISCHEN ERKRANKUNGEN MIT EINEM CDC42-SPEZIFISCHEN INHIBITOR
MÉTHODE DE TRAITEMENT DE MALADIES NÉOPLASIQUES À L'AIDE D'UN INHIBITEUR SPÉCIFIQUE DE CDC42

(30) Priority: 17.07.2019 US 201962875208 P
(43) Date of publication of application: 25.05.2022
(73) Proprietor: Children's Hospital Medical Center, Cincinnati, Ohio 45229 (US)
(72) Inventor: GUO, Fukun, Cincinnati, Ohio 45229-3039 (US); ZHENG, Yi, Cincinnati, Ohio 45229-3039 (US)
(74) Representative: Forresters IP LLP
(86) International application number: PCT/US2020/042376
(87) International publication number: WO 2021/011800

(56) References cited:
- EP-A1- 2 716 292
- WO-A1-2013/166043
- WO-A2-2009/114725
- US-A1- 2006 004 032
- US-A1- 2006 004 032
- US-A1- 2014 194 451
- LIU WEI; DU WEI; SHANG XUN; WANG LEI; EVELYN CHRIS; FLORIAN MARIA CAROLINA; A. RYAN MARNIE; RAYES AHMAD; ZHAO XUEHENG; SETCHELL KE: "Rational identification of a Cdc42 inhibitor presents a new regimen for long-term hematopoietic stem cell mobilization", LEUKEMIA, NATURE PUBLISHING GROUP UK, LONDON, vol. 33, no. 3, 25 September 2018 (2018-09-25), London, pages 749 - 761, XP036914448, ISSN: 0887-6924, DOI: 10.1038/s41375-018-0251-5
- HONG LIN, KENNEY S.RAY, PHILLIPS GENEVIEVE K., SIMPSON DENISE, SCHROEDER CHAD E., NÖTH JULICA, ROMERO ELSA, SWANSON SCARLETT, WALL: "Characterization of a Cdc42 Protein Inhibitor and Its Use as a Molecular Probe*", JOURNAL OF BIOLOGICAL CHEMISTRY, AMERICAN SOCIETY FOR BIOCHEMISTRY AND MOLECULAR BIOLOGY, vol. 288, no. 12, 1 March 2013 (2013-03-01), pages 8531 - 8543, XP055774146, ISSN: 0021-9258, DOI: 10.1074/jbc.M112.435941
- CARDAMA G.A., GONZALEZ N., MAGGIO J., MENNA P. LORENZANO, GOMEZ D.E.: "Rho GTPases as therapeutic targets in cancer (Review)", INTERNATIONAL JOURNAL OF ONCOLOGY, DEMETRIOS A. SPANDIDOS ED. & PUB, GR, vol. 51, no. 4, 1 October 2017 (2017-10-01), GR, pages 1025 - 1034, XP055774147, ISSN: 1019-6439, DOI: 10.3892/ijo.2017.4093

## Description

### BACKGROUND

### Technical Field

Provided are methods for treating a neoplastic disease in a subject by administration of at least one inhibitor of a GTPase, such as Cdc42 GTPase.

### Description of the Related Art

Rho family GTPases are molecular switches that control signaling pathways regulating cytoskeleton reorganization, gene expression, cell cycle progression, cell survival, and other cellular processes (Etienne-Manneville, 2002),

Rho family proteins constitute one of three major branches of the Ras superfamily. Development of inhibitors of Rho family GTPases may be a promising new avenue for new therapeutic compounds.
US 2006/004032 A1 discloses methods and compositions that affect the GTP-binding activity of members of the Rho family GTPases.
EP 2 716 292 A1 discloses a compound having the structure of formula I and its use as a Cdc42 inhibitor.
WO 2009/114725 A2 discloses methods, processes, uses and pharmaceutical compositions for mobilizing hematopoietic progenitor cells and/or cancer stem cells from bone marrow into peripheral blood, comprising the administration of an effective amount of a GTPase inhibitor.
WO 2013/166043 A1 discloses methods and pharmaceutical compositions for rejuvenating hematopoietic stem cells and progenitor cells by administering at least one GTPase inhibitor.

### SUMMARY OF THE INVENTION

Embodiments disclosed herein relate to methods for treating a neoplastic disease in a subject or prophylactically treating a subject for a neoplastic disease. In some embodiments, methods are provided for treating a neoplastic disease in a subject comprising, administering to a subject in need of treatment an effective amount of at least one Cdc42-specific inhibitor. In some embodiments, methods are provided for reducing the expected likelihood of a neoplastic disease in a subject comprising, administering to a subject in need of treatment an effective amount of at least one Cdc42-specific inhibitor. In some embodiments, the neoplastic disease is a tumor. In some embodiments, methods are provided for reducing tumor volume in a subject comprising, administering to a subject in need of treatment an effective amount of at least one Cdc42-specific inhibitor. In some embodiments, the methods further comprise identifying a subject as one that will benefit from treatment of a neoplastic disease or prophylactic treatment of a neoplastic disease. In some embodiments, the subject is identified on the basis of the subject's age, the subject's present medical condition, the subject's present medical treatment, or the subject's Cdc42 activity. Methods for treating a neoplastic disease also include, in some embodiments, co-administered drug therapies. In some embodiments, the subject is additionally administered an anticancer agent, an anti-neoplastic agent, or an apoptosis modulating agent that is an immune checkpoint inhibitor.

In some embodiments, the Cdc42-specific inhibitor is a small molecule. In some embodiments, the small molecule is Cdc42 Activity-Specific Inhibitor (CASIN). In the embodiments described herein, the chemical structure of CASIN is:

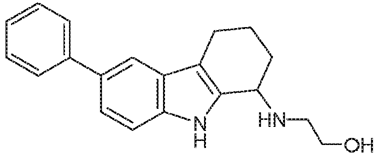

In some embodiments the small molecule comprises a compound of formula (I): 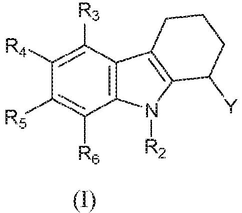 as a single enantiomer, a mixture of enantiomers, pharmaceutically acceptable salt, a solvate, or polymorph thereof, wherein:
**Y** is selected from the group consisting of **-OR₇,** -N**R₈R₉**, and -NN**R₈R₉**;
**R₇** is selected from the group consisting of C₁₋₆ alkyl, - (CH₂)**ᵤ**C₃₋₇cycloalₖyl, C₂₋₆ alkenyl, C₁₋₆ alkoxy, hydroxy-C₁₋₆ alkyl, phenyl, C₁₋₆ alkyl substituted with up to 5 fluoro, and C₁₋₆ alkoxy substituted with up to 5 fluoro, said C₁₋₆ alkyl, -(CH₂)**ᵤ**C₃₋₇cycloalkyl, C₂₋₆ alkenyl, C₁₋₆ alkoxy, hydroxy-C₁₋₆ alkyl, phenyl are each optionally substituted with one or more substitutents each independently selected from the group consisting of halo, -CN, -OH, C₁₋₆ alkoxyl, heteroaryl, **R₁₉**, and -O**R₂₀**;
**R₈** and **R₉** are each separately a hydrogen or **R₂₀**; or **R₈** and **R₉** are optionallly taken together with the nitrogen to which they are attached to form indolinyl, pyrrolidinyl, piperidinyl, piperazinyl, or morpholinyl, each optionally substituted with one or more substituents each independently selected from the group consisting of halo, cyano, nitro, hydroxy, C₁₋₆ alkyl, (CH₂)ᵤC₃₋₇cycloalₖyl, C₂₋₆ alkenyl, C₁₋₆ alkoxy, hydroxy-C₁₋₆ alkyl, phenyl, C₁₋₆ alkyl substituted with up to 5 fluoro, and C₁₋₆ alkoxy substituted with up to 5 fluoro; or **R₈** and **R₂** come together to be C₁₋₃ alkyl linking together as a ring;
each **R₂₀** separately selected from the group consisting of C₁₋₆ alkyl, C₃₋₇ cycloalkyl, and phenyl, said C₁₋₆ alkyl, C₃₋₇ cycloalkyl, and phenyl, each optionally substituted with one or more substituents each independently selected from the group consisting of **R₂₁** and **R₂₂**,
each **R₂₁** is separately selected from the group consisting of halo, cyano, nitro, and hydroxy,
each **R₂₂** is separately selected from the group consisting of C₁₋₆ alkyl, C₁₋₆ alkoxy -(CH₂)ᵤC₃₋₇cycloalkyl, C₂₋₆ alkenyl, hydroxy-C₁₋₆ alkyl, **R₁₉**, and -O**R₂₀**, each optionally substituted with one or more substituents each independently selected from the group consisting of halo, cyano, nitro, hydroxy, C₁₋₆ alkyl, and C₁₋₆ alkoxy;
each **u** is independently 0, 1, 2, 3, or 4;
**R₂** is a hydrogen, or selected from the group consisting of C₁₋₆ alkyl, C₃₋₇ cycloalkyl, and phenyl, said C₁₋₆ alkyl, C₃₋₇ cycloalkyl, and phenyl, each optionally substituted with one or more substituents each independently selected from the group consisting of halo, cyano, nitro, hydroxy, C₁₋₆ alkyl, -(CH₂)ᵤC₃₋₇cycloalkyl, C₂₋₆ alkenyl, C₁₋₆ alkoxy, hydroxy-C₁₋₆ alkyl, phenyl, C₁₋₆ alkyl substituted with up to 5 fluoro, C₁₋₆ alkoxy substituted with up to 5 fluoro, and -O(CH₂)ᵤphenyl optionally substituted with one or more substituents each independently selected from the group consisting of halo, cyano, nitro, hydroxy, C₁₋₆ alkyl, and C₁₋₆ alkoxy; or **R₈** and **R₂** come together to be C₁₋₃ alkyl linking together as a ring;
**R₃**, **R₄**, **R₈** and **R₆** are each independently selected from the group consisting of hydrogen, halo, cyano, nitro, hydroxy, C₁₋₆ alkyl, (CH₂)ᵤC₃₋₇cycloalₖyl, -O(CH₂)ᵤC₃-₇cycloalkyl, C₂₋₆ alkenyl, C₁₋₆ alkoxy, hydroxy-C₁₋₆ alkyl, phenyl, C₁₋₆ alkyl substituted with up to 5 fluoro, and C₁₋₆ alkoxy substituted with up to 5 fluoro, said C₁₋₆ alkyl, (CH₂)ᵤC₃₋₇cycloalₖyl, -O(CH₂)ᵤC₃₋₇cycloalkyl, C₂₋₆ alkenyl, C₁₋₆ alkoxy, hydroxy-C₁₋₆ alkyl, and phenyl, each optionally substituted with one or more **R₂₃**,
each **R₂₃** is independently selected from the group consisting of halo, cyano, nitro, hydroxy, C₁₋₆ alkyl, -(CH₂)**ᵤ**C₃₋₇cycloalkyl, C₂₋₆ alkenyl, C₁₋₆ alkoxy, hydroxy-C₁₋₆ alkyl, phenyl, C₁₋₆ alkyl substituted with up to 5 fluoro, and C₁₋₆ alkoxy substituted with up to 5 fluoro, said phenyl optionally substituted with one or more substituents each independently selected from the group consisting of halo, cyano, nitro, hydroxy, C₁₋₆ alkyl, -(CH₂)**ᵤ**C₃₋₇cycloalkyl, C₂₋₆ alkenyl, C₁₋₆ alkoxy, hydroxy-C₁₋₆ alkyl, phenyl, C₁₋₆ alkyl substituted with up to 5 fluoro, and C₁₋₆ alkoxy substituted with up to 5 fluoro;
each **R₁₉** is independently aryl optionally substituted with one or more substituents each independently selected from the group consisting of halo, cyano, nitro, hydroxy, C₁₋₆ alkyl optionally substituted with up to 5 fluoro, and C₁₋₆ alkoxy optionally substituted with up to 5 fluoro;
each **R₂₀** is independently hydrogen or aryl optionally substituted with one or more substituents each independently selected from the group consisting of halo, cyano, nitro, hydroxy, C₁₋₆ alkyl optionally substituted with up to 5 fluoro, and C₁₋₆ alkoxy optionally substituted with up to 5 fluoro; and
wherein when **Y** is N**R₈R₉** then **R₈** and **R₂** optionally come together to be C₁₋₃ alkyl linking together as a ring,
with the proviso when **R₈** comes together with **R₂** to be C₁₋₃ alkyl linking together as a ring then **R₄** is not substituted with hydroxyl.

### BRIEF DESCRIPTION OF THE DRAWINGS

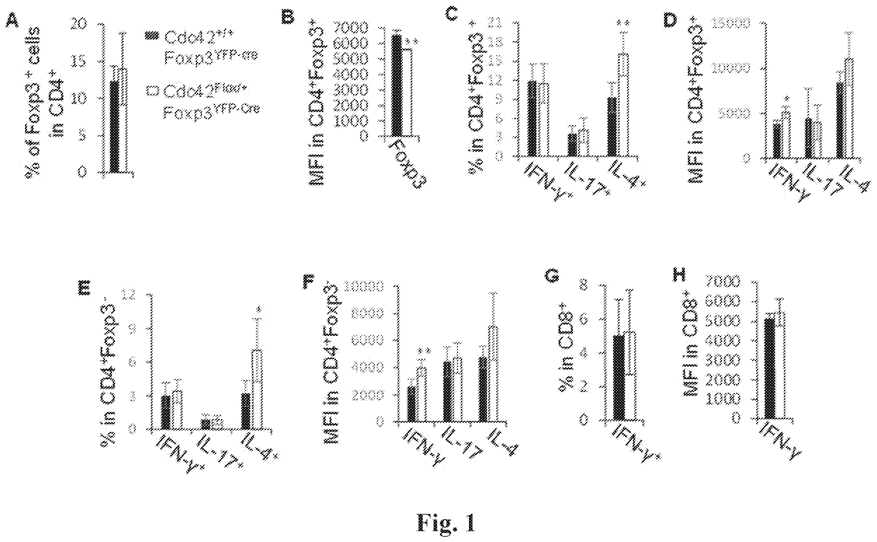

**Figure 1****. Treg-specific heterozygous deletion of CdC42 leads to impaired Treg stability.** (A) Percentages of splenic Tregs from Cdc42^{+/+}Foxp3^{YFP--Cre} (wild type, WT) and Cdc42^{fl/+}Foxp3^{YFP-Cre} (Cdc42^{+/-}) mice. (B) Mean fluorescence intensity (MFI) of Foxp3 in splenic WT and Cdc42^{+/-} Tregs. (C) Percentages of IFN-γ⁺, IL-17⁺and IL-4⁺ cells within splenic WT and Cdc42^{+/-} Tregs. (D) MFI of IFN-y, IL-17 and IL-4 in splenic WT and Cdc42^{+/-} Tregs. (E) Percentages of IFN-γ⁺, IL-17⁺ and IL-4⁺ cells within splenic CD4⁺ non-Tregs from WT and Cdc42^{+/-} mice. (F) MFI of IFN-γ, IL-17 and IL-4 in splenic CD4⁺ non-Tregs from WT and Cdc42^{+/-} mice. (G) Percentages of IFN-γ⁺ cells within splenic CD8⁺ cells from WT and Cdc42^{+/-} mice. (H) MFI of IFN-γ in splenic CD8+ cells from WT and Cdc42^{+/-} mice. n = 5. Data are representative of two independent experiments. Error bars indicate SD. *p < 0.05; **p < 0.01. In Figures 1(A)-(H), data for wild type (WT) mice is graphed in black/solid bars (left side of each data representation) and data for Cdc42^{+/-} mice is graphed in white/open bars (right side of each data representation).

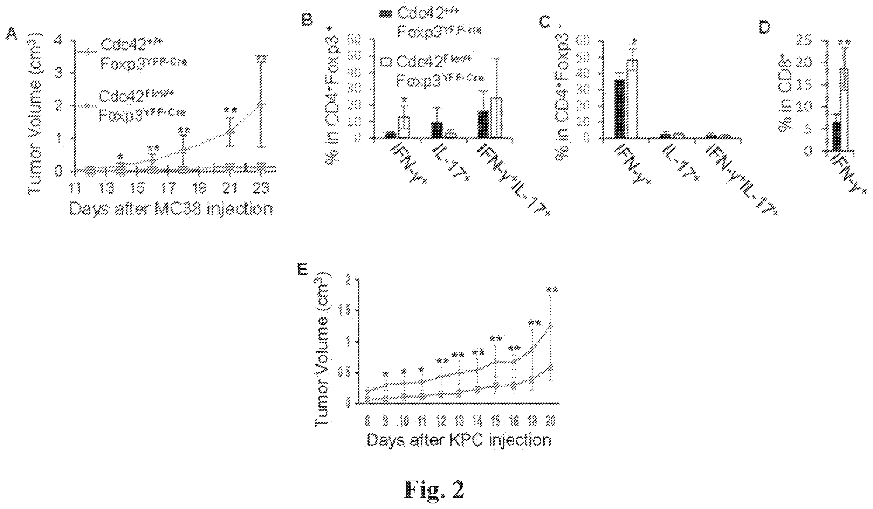

**Figure 2****. Treg-specific heterozygous deletion of Cdc42 leads to anti-tumor T cell immunity.** (A-D) MC38 mouse colon cancer cells (8 x 10⁵) were inoculated (s.c.) into WT and Cdc42^{+/-} mice. Tumor volume was monitored over 23 days (A). Tumors were dissected and the expression of IFN-γ⁺, IL-17⁺, and/or IFN-γ⁺IL-17⁺ cells in CD4⁺Foxp3⁺ (B), CD4⁺Foxp3⁻ (C), and CD8⁺ (D) was analyzed by flow cytometry. (E) KPC mouse pancreatic cancer cells (8 x 10⁵) were implanted (s.c.) into WT and Cdc42^{+/-} mice. Tumor volume was monitored over 20 days. n = 5-6. Data are representative of two independent experiments. Error bars indicate SD. **p < 0.01; *p < 0.05. In Figures 2(A)-(E), data for wild type (WT) mice is graphed in black/solid bars [left side of each data representation in Figures 2(B)-(D) and top line (furthest from the x-axis) in Figures 2(A) and (E)] and data for Cdc42^{+/-} mice is graphed in white/open bars [right side of each data representation in Figures 2(B)-(D) and bottom line of the graphed date (closest to the x-axis) in Figures 2(A) and (E)].

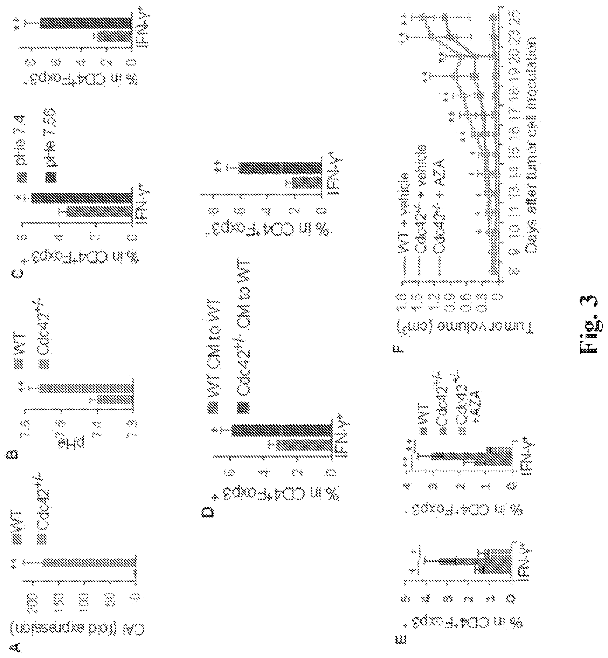

**Figure 3****. Cdc42 deficiency-induced Treg instability and tumor suppression is attributable to CAI-mediated cellular pH.** (A, B) Treg-specific heterozygous deletion of Cdc42 upregulates CAI and alkalizes extracellular pH of Tregs. Splenic CD4⁺ YFP⁺ Tregs were flow-sorted from WT and Cdc42^{+/-} mice. CAI expression was detected by Q-PCR (A). Another aliquot of the cells were cultured with Anti-CD3/CD28 and IL-2 for 36 hrs. pH of the culture medium (pHe) was measured by a pH meter. (C, D) Extracellular alkalization destabilizes Tregs. Splenic CD4⁺YFP⁺ Tregs from WT mice were cultured as described in B, with regular medium of pH 7.4 or medium of pH 7.56 (C). Alternatively, the cells were cultured with conditional medium (CM) from WT Treg culture or from Cdc42^{+/-} Treg culture (D). Percentages of IFN-γ⁺ cells within Tregs (left) and non-Tregs (right) were analyzed by flow cytometry. (E) Inhibition of CAI by acetazolamide (AZA) rescues instability of Cdc42^{+/-} Tregs. Splenic CD4+YFP⁺ Tregs from WT and Cdc42^{+/-} mice were cultured with or without AZA. Percentages of IFN-γ⁺ cells within Tregs (left) and non-Tregs (right) were analyzed by flow cytometry. (F) Inhibition of CAI by AZA restores tumor growth in Cdc42^{+/-} mice. Cdc42^{+/-} mice were treated (i.p.) with AZA (40 mg/kg) once a day throughout the experiments. Control WT and Cdc42^{+/-} mice were treated with vehicle. Three days after starting AZA injection, the mice were inoculated (s.c.) with MC38 (8 x 10⁵) and tumor volume was recorded. n = 4-7. Error bars indicate SD. *p < 0.05, **p < 0.01 (For F, WT + vehicle or Cdc42^{+/-} + AZA vs Cdc42^{-/-} + vehicle). In Figures 3(A)-(F), data for wild type (WT) mice is the left side of each data representation in Figures 3(A), (B), and (E) and middle line of the graphed data (between Cdc42^{+/-} + vehicle, which is the bottom line of graphed data closest to the x-axis, and Cdc42^{+/-} + AZA, which is the top line of the graphed data) in Figure 3(F); data for Cdc42^{+/-} mice is the right side of each data representation in Figures 3(A), (B), and bottom line of the graphed data in Figure 3(F) and the middle of each data representation in Figure 3(E). In Figure 3(C), data for "pHe 7.4" is located on the left side of each of the two bar graphs and data for "pHe 7.56" is located on the right side of each of the two bar graphs. And in Figure 3(D), data for "WT CM to WT" is located on the left side of each of the two bar graphs, while data for "Cdc42^{+/-} CM to WT" is located on the right side of each of the two bar graphs. To the extent not described above, in Figure 3(E), data for Cdc42^{+/-} + AZA is graphed in bars on the right side of each representation of data.

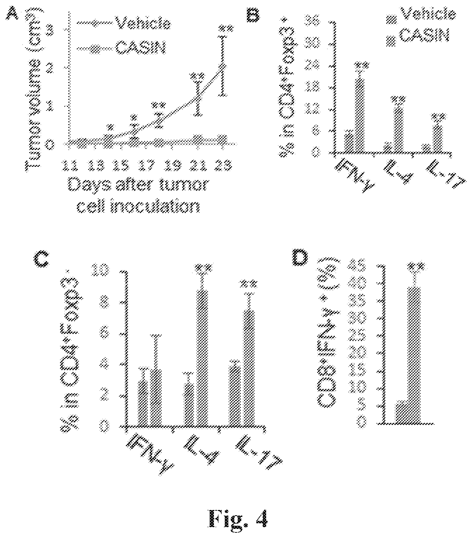

**Figure 4****. Prophylactic CASIN causes anti-tumor immunity.** C57BL/6 mice were injected (i.p.) with vehicle or 30 mg/kg body weight of CASIN twice a day for one week and then 40 mg/kg body weight of CASIN once a day until the end of the experiment. One day after the first CASIN treatment, the mice were injected (s.c.) with MC38 (8 x 10⁵). Tumor growth was monitored (A). Upon euthanasia, tumors were dissected and the expression of IFN-γ⁺, IL-4⁺ and/or IL-17⁺ within CD4⁺Foxp3⁺ (B), CD4⁺Foxp3⁻ (C), and CD8⁺ (D) cells was analyzed by flow cytometry. n = 7. Error bars indicate SD. **p < 0.01; *p < 0.05. In Figures 4(A)-(D), data for vehicle administration is the top line (furthest from the x-axis) in Figure 4A and left side of each data representation in Figures 4(B)-(D); and data for CASIN administration is the bottom line (closest to the x-axis) in Figure 4(A) and right side of each data representation in Figures 4(B)-(D).

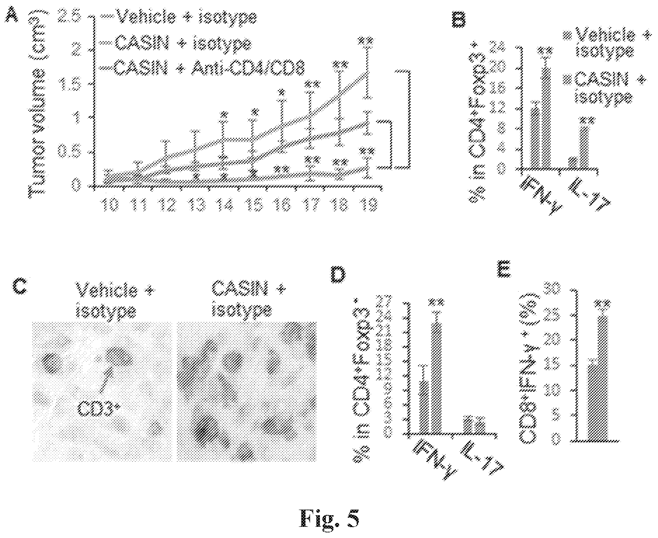

**Figure 5****. Therapeutic CASIN treatment causes anti-tumor T-cell immunity.** C57BL/6 mice were inoculated (s.c.) with MC38 (8 x 10⁵) at day 1 and injected (i.p.) with Anti-CD4/CD8 neutralizing antibodies (5 mg/kg body weight for each antibody) or isotype control antibody once every 4 days starting at day 1. Starting from day 10 when tumor onset was observed, the mice were treated with vehicle or CASIN as described for Figure 4 above. Tumor volume was monitored (A). At day 14, depletion of T-cells in peripheral blood from the mice treated with Anti-CD4/CD8 neutralizing antibodies was confirmed by flow cytometry (data not shown). Upon euthanasia, tumors were dissected from isotype control antibody-treated mice. The expression of IFN-γ⁺ and IL-17⁺ in CD4⁺Foxp3⁺ cells was analyzed by flow cytometry (B). CD3⁺ T-cells were visualized by immunohistochemistry using anti-CD3 antibody (C). The expression of IFN-γ⁺ and/or IL-17⁺ in CD4⁺Foxp3⁻ (D) and CD8⁺ cells (E) was analyzed by flow cytometry. n = 5. Error bars indicate SD. **p < 0.01; *p < 0.05. In Figure 5(A), data for "CASIN + isotype" control antibody administration is provided by the bottom-most line (closest to the x-axis), data for "Vehicle + isotype" control antibody administration is provided by the middle line (relative to the x-axis) and is below the CASIN + Anti-CD4/CD8 line and above the CASIN + isotype in the graph; and data for "CASIN + Anti-CD4/CD8" neutralizing antibodies is the top-most line (relative to the x-axis in graph). In Figure 5(B), (D), and (E), data for vehicle plus isotype control antibody administration is the left side of each data representation; and data for CASIN plus isotype control antibody administration is the right side of each data representation.

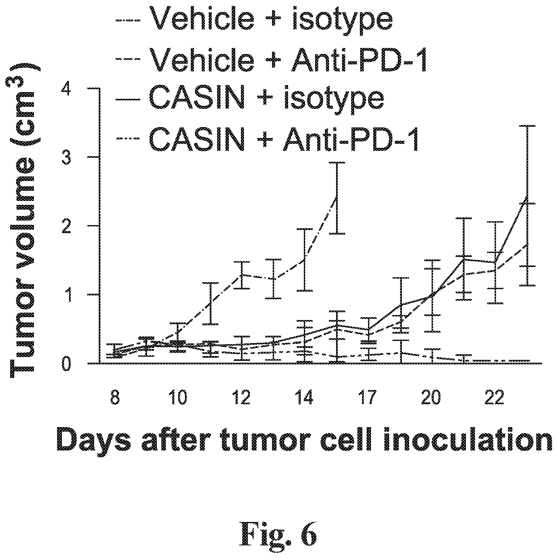

**Figure 6****. Therapeutic CASIN treatment synergizes with Anti-PD-1 in suppression of tumor growth.** C57BL/6 mice were inoculated (s.c.) with MC38 (8 x 10⁵) at day 1. Starting from day 8 when tumor onset was observed, the mice were treated (i.p.) with vehicle or CASIN as described in Figure 4. Anti-PD-1 (150 µg) or isotype control antibody was injected (i.p.) once every other day starting from day 8 until day 16. Tumor volume was monitored. n = 6-8. Error bars indicate SD. **p < 0.01 (Vehicle + isotype vs Vehicle + Anti-PD-1 or CASIN + isotype from day 11-16; Vehicle + Anti-PD-1 or CASIN + isotype vs CASIN + Anti-PD-1 from day 18-23). In Figure 6, data for vehicle plus isotype control antibody administration is provided by the "- . - -" line (terminating at day 16), data for vehicle plus Anti-PD-1 administration is provided by the "- - -" line (terminating at day 23 below the solid "-" line representing CASIN plus isotype control antibody administration and above the purple line representing CASIN plus Anti-PD-1 administration), data for CASIN plus isotype control antibody administration is provided by the solid "-" line (terminating at day 23 above the "- - -" line representing vehicle plus Anti-PD-1 administration), and data for CASIN plus Anti-PD-1 administration is provided by the "- - - -" line (terminating at day 23 below the "- - -" line representing vehicle plus Anti-PD-1 administration).

### DETAILED DESCRIPTION

Notable cancer immunotherapy drugs marketed today include immune checkpoint inhibitors and T-cells expressing chimeric antigen receptors (CAR-T). However, these immune checkpoint inhibitors demonstrate clinical efficacy in only a small proportion of cancer patients, with the majority of cancer patients demonstrating primary or acquired resistance. Thus, CAR-T therapies pose limited clinical efficacy, unacceptable toxicities (e.g. severe cytokine release syndrome, neurologic complications), difficulties in penetrating solid tumors, high cost and lengthy production. Therefore, alternative cancer immunotherapies are needed.

Cell division control protein 42 homolog, also known as Cdc42, is a protein involved in cell cycle regulation. Presented herein is the surprising discovery that exposing a subject to a Cdc42-specific inhibitor causes anti-tumor T-cell immunity in subjects with cancer. Moreover, presented herein is the surprising discovery that prophylactic administration of a Cdc42-specific inhibitor confers anti-tumor immunity in a subject. This class of small molecules have several advantages over biologic drugs (e.g. antibodies, CAR-T) because they can (1) target intracellular pathways, (2) induce acute anti-tumor effects while minimizing and/or avoiding systemic immune responses, thereby improving therapeutic index for the class, (3) easily penetrate solid tumors, (4) can be orally administered allowing for flexible, patient friendly dosing, and (5) are more cost-effective. Consequently, Cdc42-specific inhibitors represent a novel class of small molecule cancer immunotherapy drugs.

As described herein, it is intended that where a range of values is provided, it is understood that each intervening value, to the tenth of the unit of the lower limit unless the context clearly dictates otherwise, between the upper and lower limit of that range and any other stated or intervening value in that stated range is encompassed within the embodiments. The upper and lower limits of these smaller ranges may independently be included in the smaller ranges is also encompassed within the embodiments, subject to any specifically excluded limit in the stated range. Where the stated range includes one or both of the limits, ranges excluding either both of those included limits are also included in the embodiments. It is further intended that when a series of integers are reported for any particular value, e.g., 1, 2, 3, 4, 5, 6, etc., a range may be enumerated from any of the aforementioned integers, e.g., 1-6, 2-6, 3-5, 1-4, etc.

Unless defined otherwise, all technical and scientific terms used herein have the same meaning as commonly understood by one of ordinary skill in the art to which the embodiments belong. Although any methods and materials similar or equivalent to those described herein may also be used in the practice or testing of the embodiments, the preferred methods and materials are now described. All publications mentioned herein are expressly incorporated by reference in their entireties.

As used herein and in the appended claims, the singular forms "a," "and," and "the" include plural referents unless the context clearly dictates otherwise. Thus, for example, reference to "a method" includes a plurality of such methods and reference to "a dose" or "dosage" includes reference to one or more doses and equivalents thereof known to those skilled in the art, and so forth.

In some contexts, the terms "individual," "host," "subject," and "patient" are used interchangeably to refer to an animal that is the object of treatment, observation and/or experiment. "Animal" includes vertebrates and invertebrates, such as fish, shellfish, reptiles, birds, and, in particular, mammals. "Mammal" includes, without limitation, mice, rats, rabbits, guinea pigs, dogs, cats, sheep, goats, cows, horses, primates, such as monkeys, chimpanzees, and apes, and, in particular, humans.

In some contexts, the terms "ameliorating," "treating," "treatment," "therapeutic," or "therapy" do not necessarily mean total cure or abolition of the disease or condition. Any alleviation of any undesired signs or symptoms of a disease or condition, to any extent, can be considered amelioration, and in some respects a treatment and/or therapy.

The term "about" or "approximately" means within an acceptable error range for the particular value as determined by one of ordinary skill in the art, which will depend in part on how the value is measured or determined, e.g., the limitations of the measurement system. For example, "about" can mean within 1 or more than 1 standard deviations, per the practice in the art. Alternatively, "about" can mean a range of up to 20%, preferably up to 10%, more preferably up to 5%, and more preferably still up to 1% of a given value. Alternatively, particularly with respect to biological systems or processes, the term can mean within an order of magnitude, preferably within 5-fold, and more preferably within 2-fold, of a value. Where particular values are described in the application and claims, unless otherwise stated the term "about" meaning within an acceptable error range for the particular value should be assumed.

As used herein, the term "heterologous sequence or gene" means a nucleic acid (RNA or DNA) sequence, which is not naturally found in association with the nucleic acid sequences of the specified molecule. The sections below provides greater detail on some approaches that can be used to prepare inhibitors of Cdc42.

### Methods of Treating Neoplastic Diseases

Embodiments disclosed herein relate to administering to a subject in need of treatment an effective amount of at least one Cdc42-specific inhibitor. Specific methods described herein relate to methods for treating a neoplastic disease in a subject comprising, administering to a subject in need of treatment an effective amount of at least one Cdc42-specific inhibitor. In some embodiments, the aforementioned neoplastic disease may be benign. In other embodiments, the aforementioned neoplastic disease is malignant. In some embodiments, the neoplastic disease is a tumor. Prophylactic treatment of such neoplastic diseases, discussed infra, is specifically contemplated.

The methods described herein are particularly suited for the treatment of colon cancer and/or pancreatic cancer. Accordingly, in some embodiments, the neoplastic disease is selected from the group consisting of colon cancer and pancreatic cancer. The methods described herein are particularly suited for reducing tumor volume, including increases in tumor volume, and/or suppressing tumor growth, including increase in tumor growth, and/or delaying the onset of neoplastic disease, and/or delaying the progression of neoplastic disease, and/or prophylactically treating a subject for a neoplastic disease.

Tumors of the colon, such as non-neoplastic polyps, adenomas, familial syndromes, colorectal carcinogenesis, colorectal carcinoma, and carcinoid tumors are specifically contemplated for treatment according to the methods described herein (including prophylactic treatment as described infra).

Tumors of the pancreas, such as tumors of the exocrine gland and endocrine gland, adenocarcinomas, acinar cell carcinomas, intraductal papillary-mucinous neoplasm, mucinous cystic neoplasm with an invasive adenocarcinoma, gastrinomas, glucaganomas, insulinomas, somatostatinoma, VIPoma (vasoactive intestinal peptide), nonfunctional islet cell tumor, squamous tumors, pancreatic progenitor tumors, and immunogenic tumors are specifically contemplated for treatment according to the methods described herein (including prophylactic treatment as described infra).

In some embodiments, administering the Cdc42-specific inhibitor suppresses tumor growth and/or reduces tumor volume. In some embodiments, suppression of tumor growth and/or the reduction in tumor volume is suppressed/reduced by 1%, 2%, 3%, 4%, 5%, 6%, 7%, 8%, 9%, 10%, 11%, 12%, 113%, 14%, 15%, 16%, 17%, 18%, 19%, 20%, 21%, 22%, 23%, 24%, 25%, 26%, 27%, 28%, 29%, 30%, 35%, 40%, 45%, 50%, 55%, 60%, 65%, 70%, 75%, 80%, 85%, 90%, 95%, 100%, 150%, 200%, 250%, 300%, 350%, 400%, 450%, 500%, about any of the aforementioned percentages, or a range bounded by any of the aforementioned percentages (e.g., about 1%-30%, about 5%-25%, about 5%-20%, about 5%-15% or 1%-30%, 5%-25%, 5%-20%, 5%-15%), 1%-100%, 1%-90%, 1%-80%, 1%-70%, 1%-060%, 1%-50%, 1%-40%, 1%-30%, 1%-20%, 1%-10%, 10%-100%, 10%-90%, 10%-80%, 10%-70%, 10%-70%, 10%-60%, 10%-50%, 10%-40%, 10%-30%, 10%-20%, 20%-100%, 20%-90%, 20%-80%, 20%-70%, 20%-60%, 20%-50%, 20%-40%, 20%-30%, 30%-100%, 30%-90%, 30%-80%, 30%-70%, 30%-60%, 30%-50%, 30%-40%, 40%-100%, 40%-90%, 40%-80%, 40%-70%, 40%-60%, 40%-50%, 50%-100%, 50%-90%, 50%-80%, 50%-70%, 50%-60%, 60%-100%, 60%-90%, 60%-80%, 60%-70%, 70%-100%, 70%-90%, 70%-80%, 80%-100%, 80%-90%, 90%-100%, about any of the aforementioned range of percentages (e.g., about 10%-70%, about 30%-60%, or about 50%-70%), at least any of the aforementioned percentages or ranges of percentages (e.g., at least 10%, at least 20%, at least 30%, at least 40%, at least 50%, at least 60%, at least 70%, at least 80%, at least 90%, at least 100%, at least 150%, at least 200%, at least 250%, at least 300%, at least 350%, at least 400%, at least 450%, at least 500%, at least 50%-100%, at least 50%-300%), or at least about any of the aforementioned percentages or ranges of percentages (e.g., at least about 10%, at least about 20%, at least about 30%, at least about 40%, at least about 50%, at least about 60%, at least about 70%, at least about 80%, at least about 90%, at least about 100%, at least about 150%, at least about 200%, at least about 250%, at least about 300%, at least about 350%, at least about 400%, at least about 450%, at least about 500%, at least about 50%-100%, at least about 50%-300%) over the response in the absence of the first agent. In some embodiments, suppression of tumor growth and/or the reduction in tumor volume is suppressed/reduced by about 1%-200%, about 10%-190%, about 15%-180%, about 20%-170%, about 25%-150%, about 30%-125%, about 1%-100%, about 10%, about 20%, about 30%, about 40%, about 50%, about 60%, about 70%, about 80%, about 90%, or about 100% relative to tumor volume in an untreated subject or the expected tumor volume in an untreated subject. The "expected tumor volume" may be measured relative to a period of time, for example, over the period of 1 week, 2 weeks, 3 weeks, 1 month, 2 months, 3 months, 4 months, 5 months, 6 months, 7 months, 8 months, 9 months, 10 months, 11 months, 1 year, 1.1 years, 1.2 years, 1.3 years, 1.4 years, 1.5 years, 1.6 years, 1.7 years, 1.8 years, 1.9 years, 2 years, 2.5 years, 3 years, 3.5 years, 4 years, 4.5 yours, 5 years, 10 years, for the remaining life of the subject, or a range bounded by any of the aforementioned weeks, months or years, or about any of the aforementioned weeks, months or years. Expected suppression or reduction may be statistically significant or insignificant, though in preferred embodiments any expected increase is statistically significant. There are many methods known for calculating statistical significance, e.g., calculating a "p-value." In some embodiments, the threshold for statistical significance is a p-value ≤ 0.2, ≤ 0.15, ≤ 0.1, ≤ 0.05, ≤ 0.01, ≤ 0.005, about ≤ 0.2, about ≤ 0.15, about ≤ 0.1, about ≤ 0.05, about ≤ 0.01, or about ≤ 0.005. Sometimes, a result may not be statistically significant but yet the result is still informative or suggestive of some conferred benefit. It is understood that the degree of significance one would ascribe to a particular result is within the ken of the ordinarily skilled physician. Thus, embodiments described herein relate to the suppression of tumor growth, the reduction of tumor growth, and/or the reduction or suppression of tumor volume increase. Each of these methods may additionally relate to measurements and/or expectations occurring over time, including the aforementioned periods of time. Accordingly, the methods disclosed herein are particularly well-suited for combination therapy or treatment, especially therapy or treatment that includes additional chemotherapy and/or surgical intervention.

The compounds described herein can be administered as sole active ingredients and/or in a mixture and/or in a treatment regimen with one or more additional active ingredients or agents that are therapeutically or nutritionally useful, such as antibiotics, vitamins, herbal extracts, anti-inflammatories, glucose, antipyretics, analgesics, growth factors (e.g., granulocyte-macrophage colony stimulating factor (GM-CSF), Interleukins (IL-1, IL-2, IL-3, IL-4, IL-5, IL-6, IL-7, IL-8, IL-9, IL-10 IL-11, IL-12, IL-13, IL-14, or IL-15), TPO, or SCF), or other growth factors such as CSF-1, SF, EPO, leukemia inhibitory factor (LIF), or fibroblast growth factor (FGF), as well as C-KIT ligand, M-CSF and TNF-α, PIXY-321 (GM-CSF/IL-3 fusion protein), macrophage inflammatory protein, stem cell factor, thrombopoietin, growth related oncogene, G-CSF, VEGF, chemical agents (e.g., AMD3100) or chemotherapy and the like.

The term, "in conjunction with," as used herein, refers to concurrent administration of the active compound with and additional agent *(e.g.,* a growth factor or chemical agent), as well as administration of the active compound within several days (e.g., within approximately 1 to 7 days) of administration of the growth factor. Administration of the additional agent can be before, concurrent, or after administration of the active compound.

Some embodiments disclosed herein concern improved therapeutic approaches, wherein an effective amount of a Cdc42-specific inhibitor is combined or co-administered with at least one additional therapeutic agent (including, but not limited to, chemotherapeutic antineoplastics, apoptosis modulating agents, immunotherapeutics, antimicrobials, antivirals, antifungals, and anti-inflammatory agents) and/or therapeutic technique (e.g., surgical intervention, and/or radiotherapies). In some embodiments, compounds disclosed herein (e.g., a Cdc42-specific inhibitor) can sensitize a subject or cells within the subject to a second agent *(e.g.,* a chemotherapeutic agent) or therapeutic technique (e.g., radiotherapy). In some embodiments, the administering of the Cdc42-specific inhibitor enhances a therapy the subject is receiving, prescribed to receive, or about to receive for the treatment of a neoplastic disease. The subject may be receiving, prescribed to receive, or about to receive one or more of an anticancer agent, an anti-neoplastic agent, or an apoptosis modulating agent. The subject may also be receiving, prescribed to receive, or about to receive one or more of a chemotherapeutic compound, a radiation therapy, or a surgical inter^{v}ention.

The terms "sensitize" and "sensitizing," as used herein, refer to making, through the administration of a first agent *(e.g.,* a Cdc42-specific inhibitor), an animal or a cell within an animal more susceptible, or more responsive, to the biological effects (e.g., promotion or retardation of an aspect of cellular function including, but not limited to, cell growth, proliferation, invasion, angiogenesis, or apoptosis) of a second agent or therapeutic technique. The sensitizing effect of a first agent on a target cell can be measured as the difference in the intended biological effect (e.g., promotion or retardation of an aspect of cellular function including, but not limited to, cell growth, proliferation, invasion, angiogenesis, or apoptosis) observed upon the administration of a second agent or therapeutic technique with and without administration of the first agent. The response of the sensitized cell can be increased by 1%, 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, 35%, 40%, 45%, 50%, 55%, 60%, 65%, 70%, 75%, 80%, 85%, 90%, 95%, 100%, 150%, 200%, 250%, 300%, 350%, 400%, 450%, 500%, about any of the aforementioned percentages, or a range bounded by any of the aforementioned percentages (e.g., about 1%-30%, about 5%-25%, about 5%-20%, about 5%-15% or 1%-30%, 5%-25%, 5%-20%, 5%-15%), 1%-100%, 1%-90%, 1%-80%, 1%-70%, 1%-60%, 1%-50%, 1%-40%, 1%-30%, 1%-20%, 1%--10%, 10%-100%, 10%-90%, 10%-80%, 10%-70%, 10%-70%, 10%-60%, 10%-50%, 10%-40%, 10%-30%, 10%-20%, 20%-100%, 20%-90%, 20%-80%, 20%-70%, 20%-60%, 20%-50%, 20%-40%, 20%-30%, 30%-100%, 30%-90%, 30%-80%, 30%-70%, 30%-60%, 30%-50%, 30%-40%, 40%-100%, 40%-90%, 40%-80%, 40%-70%, 40%-60%, 40%-50%, 50%-100%, 50%-90%, 50%-80%, 50%-70%, 50%-60%, 60%-100%, 60%-90%, 60%-80%, 60%-70%, 70%-100%, 70%-90%, 70%-80%, 80%-100%, 80%-90%, 90%-100%, about any of the aforementioned range of percentages (e.g., about 10%-70%, about 30%-60%, or about 50%-70%), at least any of the aforementioned percentages or ranges of percentages (e.g., at least 10%, at least 20%, at least 30%, at least 40%, at least 50%, at least 60%, at least 70%, at least 80%, at least 90%, at least 100%, at least 150%, at least 200%, at least 250%, at least 300%, at least 350%, at least 400%, at least 450%, at least 500%, at least 50%-100%, at least 50%-300%), or at least about any of the aforementioned percentages or ranges of percentages (e.g., at least about 10%, at least about 20%, at least about 30%, at least about 40%, at least about 50%, at least about 60%, at least about 70%, at least about 80%, at least about 90%, at least about 100%, at least about 150%, at least about 200%, at least about 250%, at least about 300%, at least about 350%, at least about 400%, at least about 450%, at least about 500%, at least about 50%-100%, at least about 50%-300%) over the response in the absence of the first agent.

In some embodiments, the administering of the Cdc42-specific inhibitor and the one or more of the anticancer agent, the anti-neoplastic agent, or the apoptosis modulating agent affords a synergistic therapy for the treatment of the neoplastic disease. The synergistic response can be increased by 1%, 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, 35%, 40%, 45%, 50%, 55%, 60%, 65%, 70%, 75%, 80%, 85%, 90%, 95%, 100%, 150%, 200%, 250%, 300%, 350%, 400%, 450%, 500%, about any of the aforementioned percentages, or a range bounded by any of the aforementioned percentages (e.g., about 1%-30%, about 5%-25%, about 5%-20%, about 5%-15% or 1%-30%, 5%-25%, 5%-20%, 5%-15%), 1%-100%, 1%-90%, 1%-80%, 1%-70%, 1%-60%, 1%-50%, 1%-40%, 1%-30%, 1%-20%, 1%-10%, 10%-100%, 10%-90%, 10%-80%, 10%-70%, 10%-70%, 10%-60%, 10%-50%, 10%-40%, 10%-30%, 10%-20%, 20%-100%, 20%-90%, 20%-80%, 20%-70%, 20%-60%, 20%-50%, 20%-40%, 20%-30%, 30%-100%, 30%-90%, 30%-80%, 30%-70%, 30%-60%, 30%-50%, 30%-40%, 40%-100%, 40%-90%, 40%-80%, 40%-70%, 40%-60%, 40%-50%, 50%-100%, 50%-90%, 50%-80%, 50%-70%, 50%-60%, 60%-100%, 60%-90%, 60%-80%, 60%-70%, 70%-100%, 70%-90%, 70%-80%, 80%-100%, 80%-90%, 90%-100%, about any of the aforementioned range of percentages (e.g., about 10%-70%, about 30%-60%, or about 50%-70%), at least any of the aforementioned percentages or ranges of percentages (e.g., at least 10%, at least 20%, at least 30%, at least 40%, at least 50%, at least 60%, at least 70%, at least 80%, at least 90%, at least 100%, at least 150%, at least 200%, at least 250%, at least 300%, at least 350%, at least 400%, at least 450%, at least 500%, at least 50%-100%, at least 50%-300%), or at least about any of the aforementioned percentages or ranges of percentages (e.g., at least about 10%, at least about 20%, at least about 30%, at least about 40%, at least about 50%, at least about 60%, at least about 70%, at least about 80%, at least about 90%, at least about 100%, at least about 150%, at least about 200%, at least about 250%, at least about 300%, at least about 350%, at least about 400%, at least about 450%, at least about 500%, at least about 50%-100%, at least about 50%-300%) over the response in the absence of the first agent.

The term "hyperproliferative disease" or "hyperproliferative disorder" as used herein, refers to any condition in which a localized population of proliferating cells in an animal is not governed by the usual limitations of normal growth. Examples of hyperproliferative disorders include tumors, neoplasms and the like. A neoplasm is said to be benign if it does not undergo invasion or metastasis and malignant if it does either of these. A "metastatic" cell means that the cell can invade and destroy neighboring body structures. Hyperplasia is a form of cell proliferation involving an increase in cell number in a tissue or organ without significant alteration in structure or function. Metaplasia is a form of controlled cell growth in which one type of fully differentiated cell substitutes for another type of differentiated cell.

The term "neoplastic disease," as used herein, refers to any abnormal growth of cells being either benign (non-cancerous) or malignant (cancerous).

The terms "anticancer agent" and "anticancer drug," as used herein, refer to any therapeutic agents (e.g., chemotherapeutic compounds and/or molecular therapeutic compounds), radiation therapies, or surgical interventions, used in the treatment of hyperproliferative diseases such as cancer *(e.g.,* in mammals).

The term "anti-neoplastic agent," as used herein, refers to any compound that retards the proliferation, growth, or spread of a targeted (e.g., malignant) neoplasm.

The term "apoptosis modulating agents," as used herein, refers to agents which are involved in modulating (e.g., inhibiting, decreasing, increasing, promoting) apoptosis. Examples of apoptosis modulating agents include, but are not limited to, proteins and nucleic acids, which comprise a death domain or encode a death domain such as, but not limited to, Fas/CD95, TRAMP, TNF RI, DR1, DR2, DR3, DR4, DR5, DR6, FADD, and RIP. Small RNAs such as MIR RNAs can also be apoptosis modulating agents (e.g., MIR-34a). Other examples of apoptotic modulating agents include, but are not limited to, TNF-alpha, Fas ligand, antibodies to Fas/CD95 and other TNF family receptors, TRAIL, antibodies to TRAILR1 or TRAILR2, Bcl-2, p53, BAX, BAD, Akt, CAD, PI3 kinase, PP1, and caspase proteins. Modulating agents broadly include agonists and antagonists of TNF family receptors and TNF family ligands. Apoptosis modulating agents may be soluble or membrane bound (e.g. ligand or receptor). Preferred apoptosis modulating agents are inducers of apoptosis, such as TNF or a TNF-related ligand, particularly a TRAMP ligand, a Fas/CD95 ligand, a TNFR-1 ligand, or TRAIL.

A number of suitable anticancer agents are contemplated for combination or co-administration with a Cdc42-specific inhibitor to treat, prevent, or ameliorate any of the aforementioned diseases, maladies, conditions, or disorders. Indeed, some embodiments contemplate, but are not limited to, administration of a Cdc42-specific inhibitor in combination or co-administered with numerous anticancer agents such as: agents that induce apoptosis; polynucleotides (e.g., anti-sense, ribozymes, siRNA); polypeptides (e.g., enzymes and antibodies); biological mimetics (e.g., gossypol or BH3 mimetics); agents that bind (e.g., oligomerize or complex) Cdc42; alkaloids; alkylating agents; antitumor antibiotics; antimetabolites; hormones; platinum compounds; monoclonal or polyclonal antibodies (e.g., antibodies conjugated with anticancer drugs, toxins, defensins), toxins; radionuclides; biological response modifiers (e.g., interferons (e.g., IFN-alpha) and interleukins (e.g., IL-2)); adoptive immunotherapy agents; hematopoietic growth factors; agents that induce tumor cell differentiation (e.g., all-trans-retinoic acid); gene therapy reagents (e.g., antisense therapy reagents and nucleotides); tumor vaccines; angiogenesis inhibitors; proteasome inhibitors: NF-KB modulators; anti-CDK compounds; HDAC inhibitors; and the like. Numerous other examples of chemotherapeutic compounds and anticancer therapies suitable for mixture or co-administration with the disclosed inhibitors of Cdc42 are known to those skilled in the art.

In more embodiments, the Cdc42-specific inhibitors described herein and used in the methods disclosed are mixed or combined or co-administered with anticancer agents that induce or stimulate apoptosis. In some embodiments, the Cdc42-specific inhibitors described herein and used in the methods disclosed are mixed or combined or co-administered with an anticancer agent, an anti-neoplastic agent, or an apoptosis modulating agent that is an immune checkpoint inhibitor. Agents that induce apoptosis which are suitable in such compositions, mixtures, therapies and methods include, but are not limited to, radiation (e.g., X-rays, gamma rays, UV); tumor necrosis factor (TNF)-related factors (e.g., TNF family receptor proteins, TNF family ligands, TRAIL, antibodies to TRAILR1 or TRAILR2); kinase inhibitors (e.g., epidermal growth factor receptor (EGFR) kinase inhibitor, vascular growth factor receptor (VGFR) kinase inhibitor, fibroblast growth factor receptor (FGFR) kinase inhibitor, platelet-derived growth factor receptor (PDGFR) kinase inhibitor, and Bcr-Abl kinase inhibitors (such as GLEEVEC^{®})); antisense molecules; antibodies (e.g., HERCEPTIN^{®}, RITUXAN^{®}, ZEVALIN^{®}, and AVASTIN^{®}); anti-estrogens (e.g., raloxifene and tamoxifen); anti-androgens (e.g., flutamide, bicalutamide, finasteride, aminoglutethamide, ketoconazole, and corticosteroids); cyclooxygenase 2 (COX-2) inhibitors (e.g., celecoxib, meloxicam, NS-398, and non-steroidal anti-inflammatory drugs (NSAIDs)); anti-inflammatory drugs (e.g., butazolidin, DECADRON^{®}, DELTASONE^{®}, dexamethasone, dexamethasone intensol, DEXONE^{®}, HEXADROL^{®}, hydroxychloroquine, METICORTEN^{®}, oradexon, ORASONE^{®}, oxyphenbutazone, PEDIAPRED^{®}, phenylbutazone, PLAQUENIL^{®}, prednisolone, prednisone, PRELONE^{®}, and TANDEARIL^{®}); and cancer chemotherapeutic drugs (e.g., irinotecan (CAMPTOSAR^{®}), CPT-11, fludarabine (FLUDARA^{®}), dacarbazine (DTIC^{®}), dexamethasone, mitoxantrone, MYLOTARG^{®}, VP-16^{®}, cisplatin, carboplatin, oxaliplatin, 5-FU^{®}, doxorubicin, gemcitabine, bortezomib, gefitinib, bevacizumab, TAXOTERE^{®} or TAXOL^{®}); cellular signaling molecules; ceramides and cytokines; staurosporine, and the like.

In some embodiments, an immune checkpoint inhibitor is administered to the subject. In some embodiments, the immune checkpoint inhibitor is a small molecule or an antibody that targets PD-1, PD-L1, or CTLA-4. In some embodiments, the immune checkpoint inhibitor is a small molecule. In some embodiments, the immune checkpoint inhibitor is an antibody. In some embodiments, the immune checkpoint inhibitor that targets PD-1, PD-L1, or CTLA-4 is an antibody that is selected from the group consisting of pembrolizumab, nivolumad, cemiplimab, atezolizumab, avelumab, durvalumab, and ipitlimumab. In some embodiments, the immune checkpoint inhibitor is an anti-CD4/CD8 neutralizing antibody.

In still other embodiments, compositions and methods described provide a Cdc42-specific inhibitor and at least one anti-hyperproliferative or antineoplastic agent selected from alkylating agents, antimetabolites, and natural products (e.g., herbs and other plant and/or animal derived compounds).

Alkylating agents suitable for use in the present compositions, mixtures, therapies, and methods include, but are not limited to: 1) nitrogen mustards (e.g., mechlorethamine, cyclophosphamide, ifosfamide, melphalan (L-sarcolysin); and chlorambucil); 2) ethylenimines and methylmelamines (e.g., hexamethylmelamine and thiotepa); 3) alkyl sulfonates (e.g., busulfan); 4) nitrosoureas (e.g., carmustine (BCNU); lomustine (CCNU); semustine (methyl-CCNU); and streptozocin (streptozotocin)); and 5) triazenes (e.g., dacarbazine (DTIC^{®}; dimethyltriazenoimid-azolecarboxamide).

In some embodiments, antimetabolites suitable for use in the present compositions, mixtures, therapies, and methods include, but are not limited to: 1) folic acid analogs (e.g., methotrexate (amethopterin)); 2) pyrimidine analogs (e.g., fluorouracil (5-fluorouracil; 5-FU^{®}), floxuridine (fluorode-oxyuridine; FudR), and cytarabine (cytosine arabinoside)); and 3) purine analogs (e.g., mercaptopurine (6-mercaptopurine; 6-MP), thioguanine (6-thioguanine; TG), and pentostatin (2'-deoxycoformycin)).

In still further embodiments, chemotherapeutic agents suitable for use with the compositions, mixtures, therapies, and methods described herein include, but are not limited to: 1) vinca alkaloids (e.g., vinblastine (VLB), vincristine); 2) epipodophyllotoxins (e.g., etoposide and teniposide); 3) antibiotics (e.g., dactinomycin (actinomycin D), daunorubicin (daunomycin; rubidomycin), doxorubicin, bleomycin, plicamycin (mithramycin), and mitomycin (mitomycin C)); 4) enzymes (e.g., L-asparaginase); 5) biological response modifiers (e.g., interferon-alfa); 6) platinum coordinating complexes (e.g., cisplatin (cis-DDP) and carboplatin); 7) anthracenediones (e.g., mitoxantrone); 8) substituted ureas (e.g., hydroxyurea); 9) methylhydrazine derivatives (e.g., procarbazine (N-methylhydrazine; MIH)); 10) adrenocortical suppressants (e.g., mitotane (o,p'-DDD) and aminoglutethimide); 11) adrenocorticosteroids (e.g., prednisone); 12) progestins (e.g., hydroxyprogesterone caproate, medroxyprogesterone acetate, and megestrol acetate); 13) estrogens (e.g., diethylstilbestrol and ethinyl estradiol); 14) antiestrogens (e.g., tanioxifen); 15) androgens (e.g., testosterone propionate and fluoxymesterone); 16) antiandrogens (e.g., flutamide): and 17) gonadotropin-releasing hormone analogs (e.g., leuprolide).

Any oticolytic agent that is routinely used in a cancer therapy context finds use in the compositions and methods disclosed herein. For example, the U.S. Food and Drug Administration maintains a formulary of oncolytic agents approved for use in the United States. International counterpart agencies to the U.S.F.D.A. maintain similar formularies.

In some embodiments, conventional anticancer agents for use in administration with the present compounds include, but are not limited to, adriamycin, 5-fluorouracil, etoposide, camptothecin, actinomycin D, mitomycin C, cisplatin, docetaxel, gemcitabine, carboplatin, oxaliplatin, bortezomib, gefitinib, bevacizumab, demethylating agents, inhibitors of her-2, inhibitors of IGF-1R, VEGF, inhibitors of VEGFR, mTOR inhibitors, mitotic inhibitors, Smad inhibitors and taxanes. These agents can be prepared and used singularly, in combined therapeutic compositions, in kits, or in combination with immunotherapeutic agents, and the like.

For a more detailed description of anticancer agents and other therapeutic agents, those skilled in the art are referred to any number of instructive manuals including, but not limited to, the Physician's Desk Reference and to Goodman and Gilman's "Pharmaceutical Basis of Therapeutics" tenth edition, Eds. Hardman et al., 2002.

Some embodiments disclosed herein relate to an improved radiation therapy, wherein a Cdc42-specific inhibitor is provided before, during, or after a radiation therapy. Embodiments disclosed herein are not limited by the types, amounts, or delivery and administration systems used to deliver the therapeutic dose of radiation to a subject. For example, the subject may receive photon radiotherapy, particle beam radiation therapy, other types of radiotherapies, and combinations thereof. In some embodiments, the radiation is delivered to the subject using a linear accelerator. In still other embodiments, the radiation is delivered using a gamma knife, and in others, the radiation administered in the form of a radioactive implantable pellet.

The source of radiation can be external or internal to the subject. External radiation therapy is most common and involves directing a beam of high-energy radiation to a tumor site through the skin using, for instance, a linear accelerator. While the beam of radiation is localized to the tumor site, it is nearly impossible to avoid exposure of normal, healthy tissue. However, external radiation is usually well tolerated by patients. Internal radiation therapy involves implanting a radiation-emitting source, such as beads, wires, pellets, capsules, particles, and the like, inside the body at or near the tumor site including the use of delivery systems that specifically target cancer cells (e.g., using particles attached to cancer cell binding ligands). Such implants can be removed following treatment, or left in the body inactive. Types of internal radiation therapy include, but are not limited to, brachytherapy, interstitial irradiation, intracavity irradiation, radioimmunotherapy, and the like.

The subject may optionally receive radiosensitizers in addition to the Cdc42-specific inhibitor and radiation (e.g., metronidazole, misonidazole, intra-arterial Budr, intravenous iododeoxyuridine (ludR), nitroimidazole, 5-substituted-4-nitroimidazoles, 2H-isoindolediones, [[(2-bromoethyl)-amino]methyl]-nitro-1H-imidazole-1-ethanol, nitroaniline derivatives, DNA-affinic hypoxia selective cytotoxins, halogenated DNA ligand, 1,2,4 benzotriazine oxides, 2-nitroimidazole derivatives, fluorine-containing nitroazole derivatives, benzamide, nicotinamide, acridine-intercalator, 5-thiotretrazole derivative, 3-nitro-1,2,4-triazole, 4,5-dinitroimidazole derivative, hydroxylated texaphrins, cisplatin, mitomycin, tiripazamine, nitrosourea, mercaptopurine, methotrexate, fluorouracil, bleomycin, vincristine, carboplatin, epirubicin, doxorubicin, cyclophosphamide, vindesine, etoposide, paclitaxel, heat (hyperthermia), and the like), radioprotectors (e.g., cysteamine, aminoalkyl dihydrogen phosphorothioates, amifostine (WR 2721), IL-1, IL-6, and the like). Radiosensitizers enhance the killing of cancer cells. Radioprotectors protect healthy tissue from the harmful effects of radiation.

Any type of radiation can be administered to a patient, so long as the dose of radiation is tolerated by the patient without unacceptable negative side-effects. Suitable types of radiotherapy include, for example, ionizing (electromagnetic) radiotherapy (e.g., X-rays or gamma rays) or particle beam radiation therapy (e.g., high linear energy radiation). Ionizing radiation refers to radiation comprising particles or photons that have sufficient energy to produce ionization, e.g., gain or loss of electrons (as described in, for example, U.S. Pat. No. 5,770,581 incorporated herein by reference in its entirety). The effects of radiation can be at least partially controlled by the clinician. The dose of radiation can be fractionated for maximal target cell exposure and reduced toxicity.

### Methods for Prophylactically Treating Neoplastic Diseases

Embodiments disclosed herein relate to reducing the expected likelihood of a neoplastic disease in a subject comprising: administering to a subject in need of treatment an effective amount of at least one Cdc42-specific inhibitor. Embodiments disclosed herein also relate to methods of prophylactically treating a neoplastic disease in a subject comprising: administering to a subject in need of treatment an effective amount of at least one Cdc42-specific inhibitor. Surprisingly, direct pharmacological intervention of a Cdc42-specific inhibitor in a subject allows for prophylactic treatment of a neoplastic disease. Such direct administration obviates any need to obtain and purify blood cells from compatible donors, pre-treat blood cells with a Cdc42-specific inhibitor, and transplant the treated, donor blood cells into a transplant recipient.

In some embodiments, the expected likelihood of a neoplastic disease is reduced by 1%, 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, 35%, 40%, 45%, 50%, 55%, 60%, 65%, 70%, 75%, 80%, 85%, 90%, 95%, 100%, 150%, 200%, 250%, 300%, 350%, 400%, 450%, 500%, about any of the aforementioned percentages, or a range bounded by any of the aforementioned percentages (e.g., about 1%-30%, about 5%-25%, about 5%-20%, about 5%-15% or 1%-30%, 5%-25%, 5%-20%, 5%-15%), 1%-100%, 1%-90%, 1%-80%, 1%-70%, 1%-60%, 1%-50%, 1%-40%, 1%-30%, 1%-20%, 1%-10%, 10%-100%, 10%-90%, 10%-80%, 10%-70%, 10%-70%, 10%-60%, 10%-50%, 10%-40%, 10%-30%, 10%-20%, 20%-100%, 20%-90%, 20%-80%, 20%-70%, 20%-60%, 20%-50%, 20%-40%, 20%-30%, 30%-100%, 30%-90%, 30%-80%, 30%-70%, 30%-60%, 30%-50%, 30%-40%, 40%-100%, 40%-90%, 40%-80%, 40%-70%, 40%-60%, 40%-50%, 50%-100%, 50%-90%, 50%-80%, 50%-70%, 50%-60%, 60%-100%, 60%-90%, 60%-80%, 60%-70%, 70%-100%, 70%-90%, 70%-80%, 80%-100%, 80%-90%, 90%-100%, about any of the aforementioned range of percentages (e.g., about 10%-70%, about 30%-60%, or about 50%-70%), at least any of the aforementioned percentages or ranges of percentages (e.g., at least 10%, at least 20%, at least 30%, at least 40%, at least 50%, at least 60%, at least 70%, at least 80%, at least 90%, at least 100%, at least 150%, at least 200%, at least 250%, at least 300%, at least 350%, at least 400%, at least 450%, at least 500%, at least 50%-100%, at least 50%-300%), or at least about any of the aforementioned percentages or ranges of percentages (e.g., at least about 10%, at least about 20%, at least about 30%, at least about 40%, at least about 50%, at least about 60%, at least about 70%, at least about 80%, at least about 90%, at least about 100%, at least about 150%, at least about 200%, at least about 250%, at least about 300%, at least about 350%, at least about 400%, at least about 450%, at least about 500%, at least about 50%-100%, at least about 50%-300%) over the response in the absence of the first agent. In some embodiments, the expected likelihood of a neoplastic disease is reduced by about 1%-200%, about 10%-190%, about 15%-180%, about 20%-170%, about 25%-150%, about 30%-125%, about 1%-100%, about 10%, about 20%, about 30%, about 40%, about 50%, about 60%, about 70%, about 80%, about 90%, or about 100% relative to the expected likelihood of a neoplastic disease in a subject that is not administered a Cdc42-specific inhibitor.

The "likelihood" referred to in the embodiments disclosed above refers to an "expected likelihood" for the subject as opposed to the actual likelihood any particular subject experiences. Thus, one does not need to wait for a subject to develop a neoplastic disease or expire in order to practice the disclosed embodiments. The "expected likelihood" may be measured relative to a period of time, for example, over the period of 1 month, 2 months, 3 months, 4 months, 5 months, 6 months, 7 months, 8 months, 9 months, 10 months, 11 months, 1 year, 1.1 years, 1.2 years, 1.3 years, 1.4 years, 1.5 years, 1.6 years, 1.7 years, 1.8 years, 1.9 years, 2 years, 2.5 years, 3 years, 3.5 years, 4 years, 4.5 yours, 5 years, 10 years, for the remaining life of the subject, or a range bounded by any of the aforementioned months or years, or about any of the aforementioned months or years. Expected likelihoods may be statistically significant or insignificant, though in preferred embodiments any expected increase is statistically significant. There are many methods known for calculating statistical significance, e.g., calculating a "p-value." In some embodiments, the threshold for statistical significance is a p-value ≤ 0.2, ≤ 0.15, ≤ 0.1, ≤ 0.05, ≤ 0.01, ≤ 0.005, about ≤ 0.2, about ≤ 0.15, about ≤ 0.1, about ≤ 0.05, about ≤ 0.01, or about ≤ 0.005. Sometimes, a result may not be statistically significant but yet the result is still informative or suggestive of some conferred benefit. It is understood that the degree of significance one would ascribe to a particular result is within the ken of the ordinarily skilled physician.

### Subject Identification

Embodiments disclosed herein relate to administering to a subject in need of treatment an effective amount of at least one Cdc42-specific inhibitor, which includes modulators of Cdc42-specific activity. In some embodiments, not every subject is a candidate for such administration and identification of treatment subjects may be desirable. It is understood that patient selection depends upon a number of factors within the ken of the ordinarily skilled physician. Thus, some embodiments disclosed herein further comprise identifying a subject as one that will benefit from administering an effective amount of at least one Cdc42-specific inhibitor to treat a neoplastic disease. Some embodiments disclosed herein further comprise identifying a subject as a candidate for prophylactic treatment of a neoplastic disease.

Subjects may be identified on the basis of physiological factors specific to the subject according to the subject's age, present medical condition, present medical treatment, prescribed medical treatment, family history (e.g., a history of neoplastic disease or susceptibility to neoplastic disease), genetic markers indicative of neoplastic disease or susceptibility to neoplastic disease, or in preferred embodiments, the subject's Cdc42 activity. In some embodiments, a subject in need of treatment can comprise a subject having a population of blood cells that exhibit a phenotype typical of an aging cell. Assays for determining a subject's Cdc42 activity, particularly in measuring such activity in a subject's blood sample, are known in the art. *See, e.g.,* Mizukawa et al., Blood (2017) 130:1336-46.

Subjects may also be identified by a screening test for a neoplastic disease. Such tests are known in the art, and include one or more of a blood test, a stool test, a sigmoidoscopy, a colonoscopy, a biopsy, or by visualizing the colon. Subjects may also be identified by imaging an internal organ, by an endoscopic ultrasound, by a biopsy, or by a blood test for a tumor marker. It is within the ken of a physician to order or conduct such screening tests.

In some embodiments, a physician may rely on a combination of physiological factors for a given subject to identify a subject for treatment with an effective amount of at least one Cdc42-specific inhibitor. As noted above, the subject's age may be a factor in identifying a subject in need of treatment. For example, the subject may be elderly (e.g., an elderly human subject). In some embodiments, a subject in need of treatment is an elderly subject, as is understood in the art. An elderly human subject, in some embodiments described herein, is a subject having an age that is equal to or older than 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80 years old, or a range bounded by any of the aforementioned ages (e.g., an age that is equal to or older than 50-80, 50-70, 50-60, 55-75, 55-75, 55-70, 55-65, 60-70, 52-71, 60-79, or 73-78 years old).

Given the extraordinary benefits conferred by a Cdc42-specific inhibitor in the treatment or a neoplastic disease and the benefits conferred by a Cdc42-specific inhibitor in prophylaxis, a subject need not receive a screening test for a neoplastic disease prior to the subject being administered a Cdc42-specific inhibitor. In some embodiments, the Cdc42-specific inhibitor is administered to the subject prior to said subject receiving a screening test for a neoplastic disease. In some embodiments, factors other than the result of a screening test identify the subject for treatment with a Cdc42-specific inhibitor. In some embodiments, however, the Cdc42-specific inhibitor is administered to the subject after said subject receives a screening test for a neoplastic disease. Thus, in some embodiments, the results from the screening test for a neoplastic disease is a determinative factor for patient identification.

In some embodiments, as discussed supra, the subject is a human. However, the methods are not limited to the treatment of humans and are equally applicable to the treatment of non-human subjects, including mammals. In such instances of treating non-human mammals, patient selection depends upon a number of factors within the ken of the ordinarily skilled veterinarian or research scientist. The non-human subject can be elderly, as determined by an equivalent age in comparison to a human life-span. For example, the non-human subject can be a canine subject older than about 8, 9, 10, 11, 12, 13, 14, 15, 16, or older than 17 years old. Similarly, the non-human subject can be a mammalian organism, such as primate, bovine, equine, porcine, ovine, murine, canine or feline. In some embodiments the non-human subject can be a non-mammalian organism, such as avian or zebrafish. In some embodiments, the subject is not elderly, but exhibits a premature phenotype associated with an aging immune system, blood cell, T-cell, or regulatory T-cell. For example, in some embodiments, the subject is a young subject with a genetic disruption to one or more alleles of the gene encoding the 50RhoGAP protein, which causes premature aging-like phenotypes in multiple tissues and cell types.

As used herein, the terms "aging-like phenotype" and "phenotype typical of an aging cell" and like terms refer to any phenotype of a cell that is typically seen in cells in an elderly subject, but not typically seen in a young subject. Phenotypes typical of an aging immune system, blood cell, T-cell, or regulatory T-cell are known to those of skill in the art, as exemplified by the disclosure of Wang et al., Proc. Natl. Acad. Sci. USA (2007) 104:1248-1253, which is incorporated by reference in its entirety. As one example, phenotypes indicative of an aging immune system, blood cell, T-cell, or regulatory T-cell can include an increase in myeloid cell frequency and a decrease in T-cell frequency in peripheral blood, as well as an overall decrease of B-cell frequency and an increase in myeloid cell frequency in bone marrow. As another example, an aging immune system, blood cell, T-cell, or regulatory T-cell can exhibit a reduction in the polar distribution of microtubules in the cell.

Methods for measuring relative levels Cdc42 activity are known in the art, and include measuring the relative level of GTP-bound Cdc42 in a cell or cell population. Methods and reagents for measuring the relative level of GTP-bound Cdc42 are known in the art, as exemplified by the Active Cdc42 pull-down and detection kit available from Thermo Fisher Scientific Inc. (Rockford, IL), as described in the Example section below, and by the disclosure of Asnaghi et al., Oncogene (2010) 29:2760-2771, which is incorporated by reference in its entirety.

### Cdc42-Specific Inhibitors

Embodiments disclosed herein relate to compounds, compositions, pharmaceutical compositions, methods, uses, and kits that comprise at least one Cdc42-specific inhibitor. In some embodiments, the Cdc42-specific inhibitor can be a chemical inhibitor such as a small molecule (e.g., CASIN). Small molecules include, for example, chemical molecules with a low molecular weight (e.g. a molecular weight below 2000 daltons). Additionally, the Cdc42-specific inhibitor can be an siRNA molecule, an antisense molecule, a small RNA (e.g., a micro RNA) or modified nucleic acid, a ribozyme, an antibody (such as a neutralizing antibody), or a polypeptide (e.g., a dominant negative peptide). Any type of inhibitor which is known to one of skill in the art may be used.

Another aspect of the embodiments relates to the regulation of biological pathways in which a GTPase is involved. Thus, some embodiments relate to all aspects of modulating an activity of a Cdc42 GTPase comprising, administering an effective amount of an active agent, an effective amount of a compound which specifically and/or selectively modulates the activity of a Cdc42 GTPase, or combination thereof. The activity of Cdc42 which is modulated can include: GTP binding, GDP binding, GEF binding, GTPase activity, integrin binding, coupling or binding of Cdc42 to receptor or effector-like molecules (such as integrins, growth factor receptors, tyrosine kinases, PI-3K, PIP-5K, etc.). Increasing, reducing, antagonizing, or promoting Cdc42 can modulate the activity. The modulation of Cdc42 can be measured by assay for GTP hydrolysis, binding to GEF, etc. An effective amount is any amount which, when administered, modulates the Cdc42 activity. The activity can be modulated in a cell, a tissue, a whole organism, in situ, in vitro (test tube, a solid support, etc.), in vivo, or in any desired environment. In some embodiments, the effective amount of a Cdcd2-specific inhibitor is one that restores Cdc42 activity to a normal level in the subject. In some embodiments, the effective amount of a Cdc42-specific inhibitor is one that reverses tubulin apolarity in a cell, inhibits Cdc42 activity in a blood cell, T-cell, or regulatory T-cell. In some embodiments, the effective amount of the Cdc42-specific inhibitor does not mobilize a blood precursor cell. In some embodiments, the Cdc42-specific inhibitor is in a pharmaceutically acceptable composition that comprises the Cdc42-specific inhibitor in a dosage formulated to not lower Cdc42 activity below normal levels. In some embodiments, the effective amount of a Cdc42-specific inhibitor is one in which Cdc42 is inhibited in a regulatory T-cell of the subject. In some embodiments, administering the Cdc42-specific inhibitor stabilizes a regulatory T-cell in the subject.

Other assays for Cdc42-mediated signal transduction can be accomplished according to procedures known in the art, e.g., as described in U.S. Pat. Nos. 5,141,851; 5,420,334; 5,436,128; and 5,482,954, all of which are incorporated herein by reference in their entirety where permitted. In addition, peptides that inhibit the interaction, e.g., binding, between an active agent and a G-protein, such as Cdc42, can be identified.

Methods for detecting inhibition of Cdc42 activity are known in the art, as exemplified by the Active Cdc42 pull-down and detection kit available from Thermo Fisher Scientific Inc. (Rockford, IL), as described in the Example section below, and by the incorporated materials of Asnaghi et al., Oncogene (2010) 29:2760-2771. Detecting inhibition may include comparing the inhibitory properties of a compound being tested to the inhibitory properties of one or more reference compounds. Such a reference compound can be, for example, CASIN or other compounds described herein.

By modulating, it is meant that addition of the agent affects the activity or binding. The binding or activity modulation can be affected in various ways, including inhibiting, blocking, preventing, increasing, enhancing, or promoting it. The binding or activity effect does not have to be achieved in a specific way, e.g., it can be competitive, noncompetitive, allosteric, sterically hindered, via cross-linking between the agent and the GEF or GTPase, etc. The agent can act on either the active agent or GTPase. The agent can be an agonist, an antagonist, or a partial agonist or antagonist. The presence or amount of binding can be determined in various ways, e.g., by assaying for an activity promoted or inhibited by the active agent, such as guanine nucleotide exchange, GTP hydrolysis, oncogenic transformation, etc. Such assays are described above and below, and are also known in the art. The agent can be obtained and/or prepared from a variety of sources, including natural and synthetic. It can comprise, e.g., amino acids, lipids, carbohydrates, organic molecules, nucleic acids, inorganic molecules, or mixtures thereof.

Detecting modulation can be performed in vitro or in vivo as will be understood in the art. Examples of in vitro and in vivo methods are provided herein. The results from evaluating the inhibitory properties of the compounds provided herein can be reported in terms understood in the art including, for example, IC₅₀, EC₅₀, Kᵢ, or other standard terms known in the art. Thus, the evaluation provided herein can include evaluating the results where evaluating the results includes determining the inhibitory properties of the compound(s) being tested. In some instances evaluating the results also includes comparing the inhibitory properties of a compound being tested to the inhibitory properties of one or more reference compounds. Such a reference compound can be, for example, CASIN or other compounds described herein.

### Small Molecules

Small molecule inhibitors can be used to specifically inhibit and/or modulate Cdc42 as disclosed herein. Any type of small molecule inhibitor which is known to one of skill in the art may be used. Many methods are known to identify small molecule inhibitors and commercial laboratories are available to screen for small molecule inhibitors. For example, chemicals can be obtained from the compound collection at Merck^{®} Research Laboratories (Rahway, N.J.) or a like company. The compounds can be screened for inhibition of a Cdc42 by automated robotic screening in a 96-well plate format. For example, the compounds can be dissolved at an initial concentration of about 50 µM in DMSO and dispensed into the 96-well plate. The 96-well plate assay may contain an appropriate number of units of Cdc42 and target (a substrate). Compounds that cause greater than a 50% inhibition of Cdc42 activity can be further diluted and tested to establish the concentration necessary for a 50% inhibition of activity. In some embodiments, the screen will include Cdc42 protein and one or more of its binding proteins and candidate inhibitors. The inhibitory effect of screened compound to disrupt Cdc42 target binding can be monitored using, for example, an ELISA-type test with Cdc42 or the target immobilized on the surface and residual binding can be detected, for example, using antibodies of Cdc42 target (binding)-molecule conjugated to a reporter (e.g., alkaline phosphate). Binding assays can also be performed using surface plasmon resonance (SPR) based interaction screening including Cdc42 and it's binding target and inhibitor or any other assay screening protein interactions (e.g. yeast two hybrid systems, immunoprecipitation, immunocapture experiments coupled to enymatic or FACS detection etc.). In some embodiments, the candidate Cdc42 inhibitor can be tested for its ability to inhibit Cdc42 GTPase activity using assays known in the art. In other embodiments, the Cdc42 inhibitor can be tested for its ability to reduce the quantity of GTP-bound Cdc42, for example, relative to the quantity GDP-bound Cdc42, using assays known in the art.

Information disclosed herein (*e.g.,* polypeptide or nucleic acid sequences, data from assays, etc.) can be stored, recorded, and manipulated on any medium that can be read and accessed by a computer. As used herein, the words "recorded" and "stored" refer to a process for storing information on computer readable medium. A skilled artisan can readily adopt any of the presently known methods for recording information on a computer readable medium to generate manufactures comprising the information of this embodiment. A variety of data storage structures are available to a skilled artisan for creating a computer readable medium having recorded thereon information of this embodiment. The choice of the data storage structure will generally be based on the component chosen to access the stored information. Computer readable media include magnetically readable media, optically readable media, or electronically readable media. For example, the computer readable media can be a hard disc, a floppy disc, a magnetic tape, zip disk, CD-ROM, DVD-ROM, RAM, or ROM as well as other types of other media known to those skilled in the art The computer readable media on which the information is stored can be in a personal computer, a network, a server or other computer systems known to those skilled in the art.

Embodiments of the invention utilize computer-based systems that contain the information described herein and convert this information into other types of usable information (e.g., models for rational drug design). The term "a computer-based system" refers to the hardware, software, and any database used to analyze information (*e.g*., a Cdc42-specific inhibitor that enhances neoplasm therapy or prophylactic treatment for neoplasms) so as to construct models or to conduct rational drug design. The computer-based system preferably includes the storage media described above, and a processor for accessing and manipulating the sequence data. The hardware of the computer-based systems of this embodiment comprise a central processing unit (CPU) and a database. A skilled artisan can readily appreciate that any one of the currently available computer-based systems are suitable.

In some embodiments, the computer system includes a processor connected to a bus that is connected to a main memory (preferably implemented as RAM) and a variety of secondary storage devices, such as a hard drive and removable medium storage device. The removable medium storage device can represent, for example, a floppy disk drive, a DVD drive, an optical disk drive, a compact disk drive, a magnetic tape drive, etc. A removable storage medium, such as a floppy disk, a compact disk, a magnetic tape, etc. containing control logic and/or data recorded therein can be inserted into the removable storage device. The computer system includes appropriate software for reading the control logic and/or the data from the removable medium storage device once inserted in the removable medium storage device. Information described herein can be stored in a well-known manner in the main memory, any of the secondary storage devices, and/or a removable storage medium. Software for accessing and processing these sequences (such as search tools, compare tools, and modeling tools etc.) reside in main memory during execution.

As used herein, "a database" refers to memory that can store an information described herein (*e.g*., levels of cell rejuvenation, neoplasm inhibition, and values, levels or results from functional assays). Additionally, a "database" refers to a memory access component that can access manufactures having recorded thereon information described herein. In other embodiments, a database stores a "functional profile" comprising the values or levels and results (e.g., a Cdc42-specific inhibition or enhancement of neoplasm therapy or prophylactic reduction of neoplasms) from one or more functional assays, as described herein or known in the art, and relationships between these values or results. The data and values or results from functional assays can be stored and manipulated in a variety of data processor programs in a variety of formats. For example, the sequence data can be stored as text in a word processing file, a html file, or a pdf file in a variety of database programs familiar to those of skill in the art.

A "search program" refers to one or more programs that are implemented on the computer-based system to compare information (*e.g*., a Cdc42-specific inhibition or enhancement of neoplasm therapy or prophylactic reduction of neoplasms). A search program also refers to one or more programs that compare one or more protein models to several protein models that exist in a database and one or more protein models to several peptides, peptidomimetics, and chemicals that exist in a database. A search program is used, for example, to compare levels of a Cdc42-specific inhibition or enhancement of neoplasm therapy or prophylactic reduction of neoplasms by providing a therapy to a neoplasm with or without a compound (*e.g*., a Cdc42-specific inhibitor) that are present in one or more databases. Still further, a search program can be used to compare values, levels or results from functional assays.

A "retrieval program" refers to one or more programs that can be implemented on the computer-based system to identify a homologous nucleic acid sequence, a homologous protein sequence, or a homologous protein model. A retrieval program can also used to identify, for example, a Cdc42-specific inhibitors or enhancement of neoplasm therapy by Cdc42-specific inhibitors or prophylactic reduction of neoplasms by Cdc42-specific inhibitors. That is, a retrieval program can also be used to obtain a functional profile. Further, a functional profile can have one or more symbols that represent these molecules and/or models, an identifier that represents one or more inhibitors including, but not limited to values, levels, or results from a functional assay.

In any of the embodiments described herein, said Cdc42-specific inhibitor, said inhibitor of Cdc42, said inhibitor of GTPase Cdc42, said GTPase Cdc42 inhibitor, said agent capable of inhibiting GTPase Cdc42, or said agent that specifically inhibits Cdc42 comprises a compound of formula (1): 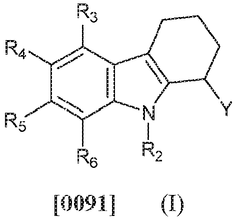 as a single enantiomer, a mixture of enantiomers, pharmaceutically acceptable salt, a solvate, or polymorph thereof, wherein:
**Y** is selected from the group consisting of -O**R7**, -N**R₈R₉**, and -NN**R₈R₉**;
**R₇** is selected from the group consisting of C₁₋₆ alkyl, -(CH₂)**ᵤ**C₃₋₇cycloalkyl, C₂₋₆ alkenyl, C₁₋₆ alkoxy, hydroxy-C₁₋₆ alkyl, phenyl, C₁₋₆ alkyl substituted with up to 5 fluoro, and C₁₋₆ alkoxy substituted with up to 5 fluoro, said C₁₋₆ alkyl, -(CH₂)**ᵤ**C₃₋₇cycloalkyl, C₂₋₆ alkenyl, C₁₋₆ alkoxy, hydroxy-C₁₋₆ alkyl, phenyl, C₁₋₆ alkyl substituted with up to 5 fluoro, and C₁₋₆ alkoxy substituted with up to 5 fluoro are each optionally substituted with one or more substituents each independently selected from the group consisting of halo, -CN, -OH, C₁₋₆ alkoxyl, heteroaryl, **R₁₉**, and -O**R₂₀**;
**R₈** and **R₉** are each separately a hydrogen or **R₂₀;** or **R₈** and **R₉** are optionally taken together with the nitrogen to which they are attached to form indolinyl, pyrrolidinyl, piperidinyl, piperazinyl, or morpholinyl, each optionally substituted with one or more substituents each independently selected from the group consisting of halo, cyano, nitro, hydroxy, C₁₋₆ alkyl, -(CH₂)**ᵤ**C₃₋₇cycloalkyl, C₂₋₆ alkenyl, C₁₋₆ alkoxy, hydroxy-C₁₋₆ alkyl, phenyl, C₁₋₆ alkyl substituted with up to 5 fluoro, and C₁₋₆ alkoxy substituted with up to 5 fluoro; or **R₈** and **R₂** come together to be C₁₋₃ alkyl linking together as a ring;
each **R₂₀** is separately selected from the group consisting of C₁₋₆ alkyl, C₃₋₇ cycloalkyl, and phenyl, said C₁₋₆ alkyl, C₃₋₇ cycloalkyl, and phenyl, each optionally substituted with one or more substituents each independently selected from the group consisting of **R₂₁** and **R₂₂**,
each **R₂₁** is separately selected from the group consisting of halo, cyano, nitro, and hydroxy,
each **R₂₂** is separately selected from the group consisting of C₁₋₆ alkyl, C₁₋₆ alkoxy, -(CH₂)**ᵤ**C₃₋₇cycloalkyl, C₂₋₆ alkenyl, hydroxy-C₁₋₆ alkyl, **R₁₉**, and O**R₂₀**, each optionally substituted with one or more substituents each independently selected from the group consisting of halo, cyano, nitro, hydroxy, C₁₋₆ alkyl, and C₁₋₆ alkoxy;
each **u** is independently 0, 1, 2, 3, or 4;
**R₂** is a hydrogen, or selected from the group consisting of C₁₋₆ alkyl, C₃₋₇ cycloalkyl, and phenyl, said C₁₋₆ alkyl, C₃₋₇ cycloalkyl, and phenyl, each optionally substituted with one or more substituents each independently selected from the group consisting of halo, cyano, nitro, hydroxy, C₁₋₆ alkyl, -(CH₂)**ᵤ**C₃₋₇cycloalkyl, C₂₋₆ alkenyl, C₁₋₆ alkoxy, hydroxy-C₁₋₆ alkyl, phenyl, C₁₋₆ alkyl substituted with up to 5 fluoro, C₁₋₆ alkoxy substituted with up to 5 fluoro, and -O(CH₂)**ᵤ**phenyl optionally substituted with one or more substituents each independently selected from the group consisting of halo, cyano, nitro, hydroxy, C₁₋₆ alkyl, and C₁₋₆ alkoxy; or **R₈** and **R₂** come together to be C₁₋₃ alkyl linking together as a ring;
**R₃, R₄, R₅** and **R₆** are each independently selected from the group consisting of hydrogen, halo, cyano, nitro, hydroxy, C₁₋₆ alkyl, -(CH₂)**ᵤ**C₃₋₇cycloalkyl, -O(CH₂)**ᵤ**C₃₋₇cycloalkyl, C₂₋₆ alkenyl, C₁₋₆ alkoxy, hydroxy-C₁₋₆ alkyl, phenyl, C₁₋₆ alkyl substituted with up to 5 fluoro, and C₁₋₆ alkoxy substituted with up to 5 fluoro, said C₁₋₆ alkyl, -(CH₂)**ᵤ**C₃-₇cycloalkyl, -O(CH₂)**ᵤ**C₃₋₇cycloalkyl, C₂₋₆ alkenyl, C₁₋₆ alkoxy, hydroxy-C₁₋₆ alkyl, and phenyl, each optionally substituted with one or more **R₂₃**,
each **R₂₃** is independently selected from the group consisting of halo, cyano, nitro, hydroxy, C₁₋₆ alkyl, -(CH₂)**ᵤ**C₃₋₇cycloalkyl, C₂₋₆ alkenyl, C₁₋₆ alkoxy, hydroxy-C₁₋₆ alkyl, phenyl, C₁₋₆ alkyl substituted with up to 5 fluoro, and C₁₋₆ alkoxy substituted with up to 5 fluoro, said phenyl optionally substituted with one or more substituents each independently selected from the group consisting of halo, cyano, nitro, hydroxy, C₁₋₆ alkyl, -(CH₂)**ᵤ**C₃₋₇cycloalkyl, C₂₋₆ alkenyl, C₁₋₆ alkoxy, hydroxy-C₁₋₆ alkyl, phenyl, C₁₋₆ alkyl substituted with up to 5 fluoro, and C₁₋₆ alkoxy substituted with up to 5 fluoro;
each **R₁₉** is independently aryl optionally substituted with one or more substituents each independently selected from the group consisting of halo, cyano, nitro, hydroxy, C₁₋₆ alkyl optionally substituted with up to 5 fluoro, and C₁₋₆ alkoxy optionally substituted with up to 5 fluoro;
each **R₂₀** is independently hydrogen or aryl optionally substituted with one or more substituents each independently selected from the group consisting of halo, cyano, nitro, hydroxy, C₁₋₆ alkyl optionally substituted with up to 5 fluoro, and C₁₋₆ alkoxy optionally substituted with up to 5 fluoro; and
wherein when **Y** is N**R₈R₉** then **R₈** and **R₂** optionally come together to be C₁₋₃ alkyl linking together as a ring,
with the proviso when **R₈** comes together with **R₂** to be C₁₋₃ alkyl linking together as a ring then **R₄** is not substituted with hydroxyl.

In some embodiments, one, two or three of **R₃, R₄, R₅** and **R₆** are not hydrogen.

In some embodiments, **R₄** is selected from the group consisting of C₁₋₆ alkyl, -(CH₂)**ᵤ**C₃₋₇cycloalkyl, -O(CH₂)**ᵤ**C₃₋₇cycloalkyl, C₂₋₆ alkenyl, C₁₋₆ alkoxy, phenyl, C₁₋₆ alkyl substituted with up to 5 fluoro, and C₁₋₆ alkoxy substituted with up to 5 fluoro, said C₁₋₆ alkyl, -(CH₂)**ᵤ**C₃-₇ycloalkyl, -O(CH₂)**ᵤ**C₃₋₇ cycloalkyl, C₂₋₆ alkenyl, C₁₋₆ alkoxy, and phenyl, each optionally substituted with one or more substituents each independently selected from the group consisting of haloC₁₋₆ alkyl, -(CH₂)**ᵤ**C₃₋₇cycloalkyl, C₂₋₆ alkenyl, C₁₋₆ alkoxy, hydroxy-C₁₋₆ alkyl, phenyl, C₁₋₆ alkyl substituted with up to 5 fluoro, and C₁₋₆ alkoxy substituted with up to 5 fluoro.

In some embodiments: **Y** is -N**R₈R₉**; **R₈** is hydrogen; and **R₉** is C₁₋₆ alkyl optionally substituted with one or more substituents each independently selected from the group consisting of hydroxy, **R₁₉** and -O**R₂₀**; each **R₁₉** is independently phenyl optionally substituted with one or more substituents each independently selected from the group consisting of halo, cyano, C₁₋₆ alkyl optionally substituted with up to 5 fluoro, and C₁₋₆ alkoxy optionally substituted with up to 5 fluoro; and each **R₂₀** is independently hydrogen or phenyl optionally substituted with one or more substituents each independently selected from the group consisting of halo, cyano, nitro, hydroxy, C₁₋₆ alkyl optionally substituted with up to 5 fluoro, and C₁₋₆ alkoxy optionally substituted with up to 5 fluoro.

In some embodiments: each **R₁₉** is independently phenyl optionally substituted with one or more substituents each independently selected from the group consisting of halo, C₁₋₆ alkyl, and C₁₋₆ alkoxy; and each **R₂₀** is independently phenyl optionally substituted with one or more substituents each independently selected from the group consisting of halo, C₁₋₆ alkyl, and C₁₋₆ alkoxy,

In some embodiments, **R₂** and **R₈** are hydrogen.

In some embodiments, **Y** is -N**R₈R₉** and **R₈** and **R₂** come together to be C₁₋₃ alkyl linking together as a ring.

In some embodiments, **R₉** is hydrogen.

In some embodiments, **R₉** is C₁₋₆ alkyl optionally substituted with one or more substituents each independently selected from the group consisting of hydroxy, **R₁₉** or - O**R₂₀**;
each **R₁₉** is independently phenyl optionally substituted with one or more substituents each independently selected from the group consisting of halo, cyano, C₁₋₆ alkyl optionally substituted with up to 5 fluoro, and C₁₋₆ alkoxy optionally substituted with up to 5 fluoro; and
each **R₂₀** is independently hydrogen or phenyl optionally substituted with one or more substituents each independently selected from the group consisting of halo, cyano, nitro, hydroxy, C₁₋₆ alkyl optionally substituted with up to 5 fluoro, and C₁₋₆ alkoxy optionally substituted with up to 5 fluoro.
In some embodiments, **R₉** is hydrogen or C₁₋₆ alkyl, optionally substituted with one or more substituents each independently selected from the group consisting of hydroxyl, **R₁₉** and -O**R₂₀**;
each **R₁₉** is independently phenyl optionally substituted with one or more substituents each independently selected from the group consisting of halo, cyano, C₁₋₆ alkyl optionally substituted with up to 5 fluoro, and C₁₋₆ alkoxy optionally substituted with up to 5 fluoro; and
each **R₂₀** is independently hydrogen or phenyl optionally substituted with one or more substituents each independently selected from the group consisting of halo, cyano, nitro, hydroxy, C₁₋₆ alkyl optionally substituted with up to 5 fluoro, and C₁₋₆ alkoxy optionally substituted with up to 5 fluoro.

In some embodiments, **R₄** is selected from the group consisting of C₁₋₆ alkyl, -(CH₂)**ᵤ**C₃₋₇cycloalkyl, -O(CH₂)**ᵤ**C₃₋₇cycloalkyl, C₂₋₆ alkenyl, C₁₋₆ alkoxy, phenyl, C₁₋₆ alkyl substituted with up to 5 fluoro, and C₁₋₆ alkoxy substituted with up to 5 fluoro, said C₁₋₆ alkyl, -(CH₂)**ᵤ**C₃₋₇cycloalkyl, -O(CH₂)**ᵤ**C₃₋₇cycloalkyl, C₂₋₆ alkenyl, C₁₋₆ alkoxy, and phenyl, each optionally substituted with one or more **R₂₃,** each **R₂₃** is independently selected from the group consisting of halo, C₁₋₆ alkyl, -(CH₂)**ᵤ**C₃₋₇cycloalkyl, C₂₋₆ alkenyl, C₁₋₆ alkoxy, phenyl, C₁₋₆ alkyl substituted with up to 5 fluoro, and C₁₋₆ alkoxy substituted with up to 5 fluoro, said phenyl optionally substituted with one or more substituents each independently selected from the group consisting of halo, C₁₋₆ alkyl, -(CH₂)**ᵤ**C₃₋₇cycloalkyl, C₂₋₆ alkenyl, C₁₋₆ alkoxy, C₁₋₆ alkyl substituted with up to 5 fluoro, and C₁₋₆ alkoxy substituted with up to 5 fluoro.

In some embodiments, **R₄** is selected from the group consisting of C₁₋₆ alkyl, C₃₋₇cycloalkyl, -OC₃₋₇cycloalkyl, phenyl, C₁₋₆ alkyl substituted with up to 5 fluoro, and C₁₋₆ alkoxy substituted with up to 5 fluoro, said phenyl optionally substituted with one or more substituents each independently selected from the group consisting of halo, C₁₋₆ alkyl, C₁₋₆ alkoxy, C₁₋₆ alkyl substituted with up to 5 fluoro, and C₁₋₆ alkoxy substituted with up to 5 fluoro.

In some embodiments, **Y** may be -N**R₈R₉** and **R₈** and **R₂** come together to be C₁₋₃ alkyl linking together as a ring.

In some embodiments, **R₂** is a hydrogen or selected from the group consisting of C₁₋₆ alkyl, C₃₋₇ cycloalkyl, and phenyl, said C₁₋₆ alkyl optionally substituted with one or more halo.

In some embodiments, **R₂** is a hydrogen.

In some embodiments, **R₉** is hydrogen, or C₁₋₆ alkyl, optionally substituted with one or more substituents each independently selected from the group consisting of hydroxyl, **R₁₉** and -O**R₂₀**;
each **R₁₉** is independently phenyl optionally substituted with one or more substituents each independently selected from the group consisting of halo, cyano, C₁₋₆ alkyl optionally substituted with up to 5 fluoro, and C₁₋₆ alkoxy optionally substituted with up to 5 fluoro; and
each **R₂₀** is independently hydrogen or phenyl optionally substituted with one or more substituents each independently selected from the group consisting of halo, cyano, nitro, hydroxy, C₁₋₆ alkyl optionally substituted with up to 5 fluoro, and C₁₋₆ alkoxy optionally substituted with up to 5 fluoro.

In some embodiments, the compound of formula (I) is selected from the group consisting of:

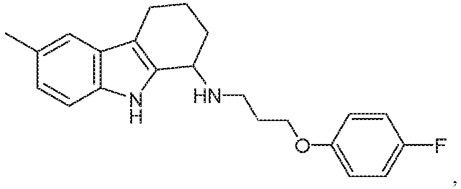

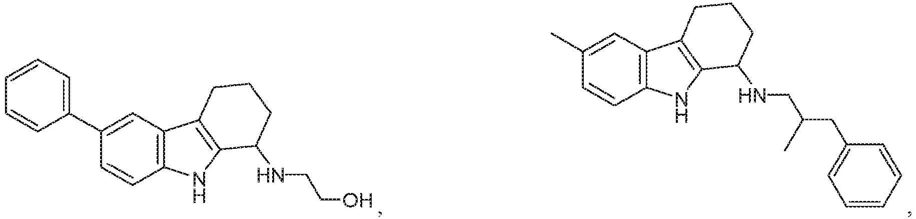

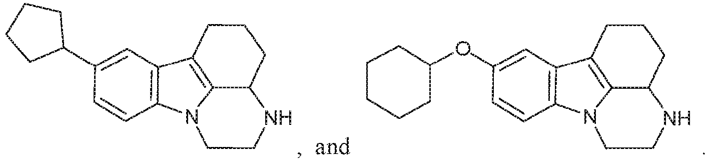

In some embodiments, the compound of formula (I) is CASIN: 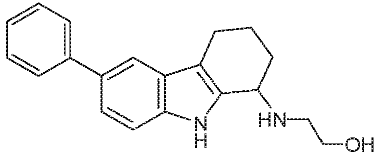 or a pharmaceutically acceptable salt thereof.

The term "ester" refers to a chemical moiety with formula -(**R**)**ₙ**-COO**R**', where **R** and **R'** are independently selected from the group consisting of alkyl, cycloalkyl, aryl, heteroaryl (bonded through a ring carbon) and heteroalicyclic (bonded through a ring carbon), and where n is 0 or 1.

An "amide" is a chemical moiety with formula -(**R**)**ₙ**-C(O)NH**R'** or -(**R**)**ₙ**-NHC(O)**R'**, where **R** and **R'** are independently selected from the group consisting of alkyl, cycloalkyl, aryl, heteroaryl (bonded through a ring carbon) and heteroalicyclic (bonded through a ring carbon), and where **n** is 0 or 1. An amide may be an amino acid or a peptide molecule attached to a molecule of the present invention, thereby forming a prodrug.

Any amine, hydroxy, or carboxyl side chain on the compounds of the present invention can be esterified or amidified. The procedures and specific groups to be used to achieve this end are known to those of skill in the art and can readily be found in reference sources such as Greene and Wuts, Protective Groups in Organic Synthesis, 3rd Ed., John Wiley & Sons, New York, NY, 1999, which is incorporated herein in its entirety.

The terms "protecting group" and "protecting groups" as used herein refer to any atom or group of atoms that is added to a molecule in order to prevent existing groups in the molecule from undergoing unwanted chemical reactions. Examples of protecting group moieties are described in T. W. Greene and P. G. M. Wuts, Protective Groups in Organic Synthesis, 3. Ed. John Wiley & Sons, 1999, and in J.F.W. McOmie, Protective Groups in Organic Chemistry Plenum Press, 1973, both of which are hereby incorporated by reference. The protecting group moiety may be chosen in such a way, that they are stable to the reaction conditions applied and readily removed at a convenient stage using methodology known from the art. A non-limiting list of protecting groups include benzyl; substituted benzyl; alkylcarbonyls (e.g., t-butoxycarbonyl (BOC)); arylalkylcarbonyls (e.g., benzyloxycarbonyl, benzoyl); substituted methyl ether (e.g. methoxymethyl ether); substituted ethyl ether; a substituted benzyl ether; tetrahydropyranyl ether; silyl ethers (e.g., trimethylsilyl, triethylsilyl, triisopropylsilyl, t-butyldimethylsilyl, or t-butyldiphenylsilyl); esters (e.g. benzoate ester); carbonates (e.g. methoxymethylcarbonate); sulfonates (e.g. tosylate, mesylate); acyclic ketal (e.g. dimethyl acetal); cyclic ketals (e.g., 1,3-dioxane or 1,3-dioxolanes); acyclic acetal; cyclic acetal; acyclic hemiacetal; cyclic hemiacetal; and cyclic dithioketals (e.g., 1,3-dithiane or 1,3-dithiolane).

A "prodrug" refers to an agent that is converted into the parent drug in vivo. Prodrugs are often useful because, in some situations, they may be easier to administer than the parent drug. They may, for instance, be bioavailable by oral administration whereas the parent is not. The prodrug may also have improved solubility in pharmaceutical compositions over the parent drug. An example, without limitation, of a prodrug would be a compound of the present invention which is administered as an ester (the "prodrug") to facilitate transmittal across a cell membrane where water solubility is detrimental to mobility but which then is metabolically hydrolyzed to the carboxylic acid, the active entity, once inside the cell where water-solubility is beneficial. A further example of a prodrug might be a short peptide (polyaminoacid) bonded to an acid group where the peptide is metabolized to reveal the active moiety.

The term "aromatic" refers to an aromatic group which has at least one ring having a conjugated pi electron system and includes both carbocyclic aryl (e.g., phenyl) and heterocyclic aryl groups (e.g., pyridine). The term includes monocyclic or fused-ring polycyclic (i.e., rings which share adjacent pairs of carbon atoms) groups. The term "carbocyclic" refers to a compound which contains one or more covalently closed ring structures, and that the atoms forming the backbone of the ring are all carbon atoms. The term thus distinguishes carbocyclic from heterocyclic rings in which the ring backbone contains at least one atom which is different from carbon. The term "heteroaromatic" refers to an aromatic group which contains at least one heterocyclic ring.

As used herein, the term "alkyl" refers to an aliphatic hydrocarbon group. The alkyl moiety may be a "saturated alkyl" group, which means that it does not contain any alkene or alkyne moieties. The alkyl moiety may also be an "unsaturated alkyl" moiety, which means that it contains at least one alkene or alkyne moiety. An "alkene" moiety refers to a group consisting of at least two carbon atoms and at least one carbon-carbon double bond, and an "alkyne" moiety refers to a group consisting of at least two carbon atoms and at least one carbon-carbon triple bond. The alkyl moiety, whether saturated or unsaturated, may be branched, straight chain, or cyclic.

The alkyl group may have 1 to 20 carbon atoms (whenever it appears herein, a numerical range such as "1 to 20" refers to each integer in the given range; *e.g.*, "1 to 20 carbon atoms" means that the alkyl group may consist of 1 carbon atom, 2 carbon atoms, 3 carbon atoms, *etc.*, up to and including 20 carbon atoms, although the present definition also covers the occurrence of the term "alkyl" where no numerical range is designated). The alkyl group may also be a medium size alkyl having 1 to 10 carbon atoms. The alkyl group could also be a lower alkyl having 1 to 5 carbon atoms. The alkyl group of the compounds of the invention may be designated as "C₁-C₄ alkyl" or similar designations. By way of example only, "C₁-C₄ alkyl" indicates that there are one to four carbon atoms in the alkyl chain, i.e., the alkyl chain is selected from the group consisting of methyl, ethyl, propyl, iso-propyl, n-butyl, iso-butyl, sec-butyl, and t-butyl.

The alkyl group may be substituted or unsubstituted. When substituted, the substituent group(s) is(are) one or more group(s) individually and independently selected from cycloalkyl, aryl, heteroaryl, heteroalicyclic, hydroxy, alkoxy, aryloxy, mercapto, alkylthio, arylthio, cyano, halo, carbonyl, thiocarbonyl, O-carbamyl, N-carbamyl, O-thiocarbamyl, N-thiocarbamyl, C-amido, N-amido, S-sulfonamido, N-sulfonamido, C-carboxy, O-carboxy, isocyanato, thiocyanato, isothiocyanato, nitro, silyl, trihalomethanesulfonyl, and amino, including mono- and di-substituted amino groups, and the protected derivatives thereof. Typical alkyl groups include, but are in no way limited to, methyl, ethyl, propyl, isopropyl, butyl, isobutyl, tertiary butyl, pentyl, hexyl, ethenyl, propenyl, butenyl, cyclopropyl, cyclobutyl, cyclopentyl, cyclohexyl, and the like. Wherever a substituent is described as being "optionally substituted" that substitutent may be substituted with one of the above substituents.

The substituent "R" appearing by itself and without a number designation refers to a substituent selected from the group consisting of hydrogen, alkyl, cycloalkyl, aryl, heteroaryl (bonded through a ring carbon) and heteroalicyclic (bonded through a ring carbon).

An "O-carboxy" group refers to a **R**C(=O)O- group, where **R** is as defined herein.

A "C-carboxy" group refers to a -C(=O)O**R** groups where **R** is as defined herein.

An "acetyl" group refers to a -C(=O)CH₃, group.

A "trihalomethanesulfonyl" group refers to a **X**₃CS(=O)₂- group where **X** is a halogen.

A "cyano" group refers to a -CN group.

An "isocyanato" group refers to a -NCO group.

A "thiocyanato" group refers to a -CNS group.

An "isothiocyanato" group refers to a -NCS group.

A "sulfinyl" group refers to a -S(=O)-**R** group, with **R** as defined herein.

A "S-sulfonamido" group refers to a -S(=O)₂N**R**, group, with **R** as defined herein.

A "N-sulfonamido" group refers to a **R**S(=O)₂NH- group with **R** as defined herein.

A "trihalomethanesulfonamido" group refers to a **X**₃CS(=O)₂N**R**- group with **X** and **R** as defined herein.

An "O-carbamyl" group refers to a -OC(=O)-N(**R**)₂, group-with **R** as defined herein.

An "N-carbamyl" group refers to a **R**OC(=O)NH- group, with **R** as defined herein.

An "O-thiocarbamyl" group refers to a -OC(=S)-N(**R**)₂, group with **R** as defined herein.

An "N-thiocarbamyl" group refers to an **R**OC(=S)NH- group, with **R** as defined herein.

A "C-amido" group refers to a -C(=O)-N(**R**)₂ group with **R** as defined herein.

An "N-amido" group refers to a **R**C(=O)NH- group, with **R** as defined herein.

The term "perhaloalkyl" refers to an alkyl group where all of the hydrogen atoms are replaced by halogen atoms.

The term "acylalkyl" refers to a **R**C(=O)**R**'- group, with **R** as defined herein, and R' being a diradical alkylene group. Examples of acylalkyl, without limitation, may include CH₃C(=O)CH₂-, CH₃C(=O)CH₂CH₂-, CH₃CH₂C(=O)CH₂CH₂-, CH₃C(=O)CH₂CH₂CH₂-, and the like.

Unless otherwise indicated, when a substituent is deemed to be "optionally substituted," it is meant that the substituent is a group that may be substituted with one or more group(s) individually and independently selected from cycloalkyl, aryl, heteroaryl, heteroalicyclic, hydroxy, alkoxy, aryloxy, mercapto, alkylthio, arylthio, cyano, halo, carbonyl, thiocarbonyl, O-carbamyl, N-carbamyl, O-thiocarbamyl, N-thiocarbamyl, C-amido, N-amido, S-sulfonamido, N-sulfonamido, C-carboxy, O-carboxy, isocyanato, thiocyanato, isothiocyanato, nitro, silyl, trihalomethanesulfonyl, and amino, including mono- and di-substituted amino groups, and the protected derivatives thereof. The protecting groups that may form the protective derivatives of the above substituents are known to those of skill in the art and may be found in references such as Greene and Wuts, above.

In the present context, the term "cycloalkyl" is intended to cover three-, four-, five-, six-, seven-, and eight- or more membered rings comprising carbon atoms only. A cycloalkyl can optionally contain one or more unsaturated bonds situated in such a way, however, that an aromatic pi-electron system does not arise. Some examples of "cycloalkyl" are the carbocycles cyclopropane, cyclobutane, cyclopentane, cyclopentene, cyclopentadiene, cyclohexane, cyclohexene, 1,3-cycloliexadiene, 1,4-cyclohexadiene, cycloheptane, or cycloheptene.

As used herein, "heterocyclyl" means a cyclic ring system comprising at least one heteroatom in the ring system backbone. The heteroatoms are independently selected from oxygen, sulfur, and nitrogen. Heterocyclyls may include multiple fused rings. Heterocyclyls may have any degree of saturation provided that at least one ring in the ring system is not aromatic. Heterocyclyls may be substituted or unsubstituted, and are attached to other groups via any available valence, preferably any available carbon or nitrogen. Preferred monocyclic heterocycles are of 5 or 6 members. In six membered monocyclic heterocycles, the heteroatom(s) are selected from one up to three of oxygen, sulfur, and nitrogen, and wherein when the heterocycle is five membered, preferably it has one or two heteroatoms selected from oxygen, sulfur, and nitrogen.

A heterocyclyl can further contain one or more carbonyl or thiocarbonyl functionalities, so as to make the definition include oxo-systems and thio-systems such as lactams, lactones, cyclic imides, cyclic thioimides, cyclic carbamates, and the like.

Some examples of "heterocyclyls" include, but are not limited to, tetrahydrothiopyran, 4*H*-pyran, tetrahydropyran, piperidine, 1,3-dioxin, 1,3-dioxane, 1,4-dioxin, 1,4-dioxane, piperazine, 1,3-oxathiane, 1,4-oxathiin, 1,4-oxathiane, tetrahydro-1,4-thiazine, 2*H*-1,2-oxazine, maleimide, succinimide, barbituric acid, thiobarbituric acid, dioxopiperazine, hydantoin, dihydrouracil, morpholine, trioxane, hexahydro-1,3,5-triazine, tetrahydrothiophene, tetrahydrofuran, pyrroline, pyrrolidine, pyrrolidone, pyrrolidione, pyrazoline, pyrazolidine, imidazoline, imidazolidine, 1,3-dioxole, 1,3-dioxolane, 1,3-dithiole, 1,3-dithiolane, isoxazoline, isoxazolidine, oxazoline, oxazolidine, oxazolidinone, thiazoline, thiazolidine, and 1,3-oxathiolane. The attachment point of a heterocycle radical can be at the position of a nitrogen heteroatom or via a carbon atom of the heterocycle.

In the present context the term "aryl" is intended to mean a carbocyclic aromatic ring or ring system. Moreover, the term "aryl" includes fused ring systems wherein at least two aryl rings, or at least one aryl and at least one C₃₋₈-cycloalkyl share at least one chemical bond. Some examples of "aryl" rings include optionally substituted phenyl, naphthalenyl, phenanthrenyl, anthracenyl, tetralinyl, fluorenyl, indenyl, and indanyl. The term "aryl" relates to aromatic, including, for example, benzenoid groups, connected via one of the ring-forming carbon atoms, and optionally carrying one or more substituents selected from heterocyclyl, heteroaryl, halo, hydroxy, amino, cyano, nitro, alkylamido, acyl, C₁₋₆ alkoxy, C₁₋₆ alkyl, C₁₋₆ hydroxyalkyl, C₁₋₆ aminoalkyl, C₁₋₆ alkylamino, alkylsulfenyl, alkylsulfinyl, alkylsulfonyl, sulfamoyl, or trifluoromethyl. The aryl group can be substituted at the *para* and/or *meta* positions. In other embodiments, the aryl group can be substituted at the *ortho* position. Representative examples of aryl groups include, but are not limited to, phenyl, 3-halophenyl, 4-halophenyl, 3-hydroxyphenyl, 4-hydroxyphenyl, 3-aminophenyl, 4-aminophenyl, 3-methylphenyl, 4-methylphenyl, 3-methoxyphenyl, 4-methoxyphenyl, 4-trifluoromethoxyphenyl 3-cyanophenyl, 4-cyanophenyl, dimethylphenyl, naphthyl, hydroxynaphthyl, hydroxymethylphenyl, trifluoromethylphenyl, alkoxyphenyl, 4-morpholin-4-ylphenyl, 4-pyrrolidin-1-ylphenyl, 4-pyrazolylphenyl, 4-triazolylphenyl, and 4-(2-oxopyrrolidin-1-yl)phenyl.

As used herein, the term "heteroaryl" means an aromatic radical having one or more heteroatom(s) (e.g., oxygen, sulfur, or nitrogen) in the ring backbone and may include a single ring (e.g., pyridine) or multiple condensed rings (e.g., quinoline). Heteroaryl groups can carry one or more substituents, each independently selected from halo, hydroxy, amino, cyano, nitro, cycloalkyl, haloalkyl, aryl, heterocyclyl, mercapto, alkylamido, acyl, C₁₋₆-alkoxy, C₁₋₆-alkyl, C₁₋₆-hydroxyalkyl, C₁₋₆-aminoalkyl, C₁₋₆-alkylamino, alkylsulfenyl, alkylsulfinyl, alkylsulfonyl, sulfamoyl, and trifluoromethyl. Representative examples of heteroaryl groups include, but are not limited to, optionally substituted derivatives of furan, benzofuran, thiophene, benzothiophene, pyrrole, pyridine, indole, oxazole, benzoxazole, isoxazole, benzisoxazole, thiazole, benzothiazole, isothiazole, imidazole, benzimidazole, pyrazole, indazole, tetrazole, quionoline, isoquinoline, pyridazine, pyrimidine, purine and pyrazine, furazan, 1,2,3-oxadiazole, 1,2,3-thiadiazole, 1,2,4-thiadiazole, triazole, benzotriazole, pteridine, phenoxazole, oxadiazole, benzopyrazole, quinolizine, cinnoline, phthalazine, quinazoline, and quinoxaline. In some embodiments, the substituents can be halo, hydroxy, cyano, O-C₁₋₆-alkyl, C₁₋₆-alkyl, hydroxy-C₁₋₆-alkyl, and amino-C₁₋₆-alkyl.

### Antisense Molecules

In some embodiments, the Cdc42-specific inhibitor can be an antisense molecule. The term "antisense" (AS) or "antisense fragment" refers to a polynucleotide fragment (comprising either deoxyribonucleotides, ribonucleotides or a mixture of both) having inhibitory antisense activity, which causes a decrease in the expression of the endogenous genomic copy of the corresponding gene. An AS polynucleotide refers to a polynucleotide which comprises consecutive nucleotides having a sequence of sufficient length and homology to a sequence present within the sequence of the target gene to permit hybridization of the AS to the gene. Many reviews have covered the main aspects of antisense (AS) technology and its enormous therapeutic potential (see, for example, Aboul-Fadl T., Curr Med. Chem. 2005; 12(19):2193-214; Crooke S T, Curr Mol. Med. 2004 August; 4(5):465-87; Crooke S T, Annu Rev Med. 2004; 55:61-95; Vacek M et al., Cell Mol Life Sci. 2003 May; 60(5):825-33; Cho-Chung Y S, Arch Pharm Res. 2003 March; 26(3):183-91; Moreira J N et al., Rev Recent Clin Trials 2006 Sep; 1(3):217-35). There are further reviews on the chemical (Crooke, 1995; Uhlmann et al, 1990), cellular (Wagner, 1994) and therapeutic (Hanania, et al, 1995; Scanlon, et al, 1995; Gewirtz, 1993) aspects of this technology. Antisense intervention in the expression of specific genes can be achieved by the use of synthetic AS oligonucleotide sequences (see, e.g., Lefebvre-d'Hellencourt et al, 1995; Agrawal, 1996; LevLehman et al, 1997).

AS oligonucleotide sequences may be short sequences of DNA, typically a 15-mer to a 30-mer but may be as small as a 7-mer (Wagner et al, 1996), designed to complement a target mRNA of interest and form an RNA:AS duplex. This duplex formation can prevent processing, splicing, transport or translation of the relevant mRNA. Moreover, certain AS nucleotide sequences can elicit cellular RNase H activity when hybridized with their target mRNA, resulting in mRNA degradation (Calabretta et al, 1996 Semin Oncol. 23(1):78-87). In that case, RNase H will cleave the RNA component of the duplex and can potentially release the AS to further hybridize with additional molecules of the target RNA An additional mode of action results from the interaction of AS with genomic DNA to form a triple helix, which can be transcriptionally inactive.

The sequence target segment for the antisense oligonucleotide is selected such that the sequence exhibits suitable energy related characteristics important for oligonucleotide duplex formation with their complementary templates, and shows a low potential for self-dimerization or self-complementation (Anazodo et al., 1996). For example, the computer program OLIGO^{®} (Primer Analysis Software, Version 3.4), can be used to determine antisense sequence melting temperature, free energy properties, and to estimate potential self-dimer formation and self-complimentary properties. The program allows the determination of a qualitative estimation of these two parameters (potential self-dimer formation and self-complimentary) and provides an indication of "no potential" or "some potential" or "essentially complete potential". Using this program target segments are generally selected that have estimates of no potential in these parameters. However, segments can be used that have "some potential" in one of the categories. A balance of the parameters is used in the selection as is known in the art. Further, the oligonucleotides are also selected as needed so that analogue substitutions do not substantially affect function.

Phosphorothioate antisense oligonucleotides do not normally show significant toxicity at concentrations that are effective and exhibit sufficient pharmacodynamic half-lives in animals (Agarwal et al., 1996) and are nuclease resistant. Antisense induced loss-of-function phenotypes related with cellular development were shown for the glial fibrillary acidic protein (GFAP), for the establishment of tectal plate formation in chick (Galileo et al., 1991) and for the N-myc protein, responsible for the maintenance of cellular heterogeneity in neuroectodermal cultures (ephithelial vs. neuroblastic cells, which differ in their colony forming abilities, tumorigenicity and adherence) (Rosolen et al., 1990; Whitesell et al, 1991). Antisense oligonucleotide inhibition of basic fibroblast growth factor (bFgF), having mitogenic and angiogenic properties, suppressed 80% of growth in glioma cells (Morrison, 1991) in a saturable and specific manner. Being hydrophobic, antisense oligonucleotides interact well with phospholipid membranes (Akhter et al., 1991). Following their interaction with the cellular plasma membrane, they are actively (or passively) transported into living cells (Loke et al., 1989), in a saturable mechanism predicted to involve specific receptors (Yakubov et al., 1989).

### siRNA

In other embodiments, the Cdc42-specific inhibitor can be a "small interfering RNA" (siRNA). siRNA refers to an RNA molecule which decreases or silences (prevents) the expression of a gene/mRNA (e.g., Cdc42) of its endogenous cellular counterpart. The term is understood to encompass "RNA interference" (RNAi). RNA interference (RNAi) refers to the process of sequence-specific post transcriptional gene silencing in mammals mediated by small interfering RNAs (siRNAs, e.g., short hairpin RNAs (shRNAs)) (Fire et al, 1998, Nature 391, 806). The corresponding process in plants is commonly referred to as specific post transcriptional gene silencing or RNA silencing and is also referred to as quelling in fungi. The RNA interference response may feature an endonuclease complex containing an siRNA, commonly referred to as an RNA-induced silencing complex (RISC), which mediates cleavage of single-stranded RNA having sequence complementary to the antisense strand of the siRNA duplex. Cleavage of the target RNA may take place in the middle of the region complementary to the antisense strand of the siRNA duplex (Elbashir et al 2001, Genes Dev., 15, 188). For recent information on these terms and proposed mechanisms, see Bernstein E., Denli A M., Hannon G J: The rest is silence. RNA. 2001 November; 7(11):1509-21; and Nishikura K.: A short primer on RNAi: RNA-directed RNA polymerase acts as a key catalyst. Cell. 2001 November 16; 107(4):415-8.

RNAi is an efficient method for the inactivation of genes (Nature Reviews, 2002, v. 3, p. 737-47; Nature, 2002, v. 418, p. 244-51). As a method, it is based on the ability of dsRNA species to enter a specific protein complex, where it is then targeted to the complementary cellular RNA and specifically degrades it. In more detail, dsRNAs are digested into short (17-29 bp) inhibitory RNAs (siRNAs) by type III RNAses (DICER, Drosha, etc) (Nature, 2001, v. 409, p. 363-6; Nature, 2003, 425, p. 415-9). These fragments and complementary mRNA are recognized by the specific RISC protein complex. The whole process is culminated by endonuclease cleavage of target mRNA (Nature Reviews, 2002, v. 3, p. 737-47; Curr Opin Mol. Ther. 2003 June; 5(3):217-24).

For disclosure on how to design and prepare siRNA to known genes see, for example, Chalk A M, Wahlestedt C, Sonnhammer E L. 2004 Jun. 18; 319(1):264-74; Sioud M, Leirdal M., Methods Mol. Biol. 2004; 252:457-69; Levenkova N, Gu Q, Rux J J. 2004 Feb. 12; 20(3):430-2; and Ui-Ter K, Naito Y, Takahashi F, Haraguchi T, Ohki-Hamazaki H, Juni A, Ueda R, Saigo K., Nucleic Acids Res. 2004 Feb. 9; 32(3):936--48. See also PCT publications WO 2004/015107 (Atugen) and WO 02/44321 (Tuschl et al), and also Chiu Y L, Rana T M. RNA 2003 September; 9(9):1034-48 and U.S. Pat. Nos. 5,898,031 and 6,107,094 (Crooke) for production of modified/more stable siRNAs.

DNA-based vectors capable of generating siRNA within cells have been developed. The method generally involves transcription of short hairpin RNAs that are efficiently processed to form siRNAs within cells. (see, e.g., Paddison et al. PNAS 2002, 99:1443-1448; Paddison et al. Genes & Dev 2002, 16:948-958; Sui et al. PNAS 2002, 8:5515-5520; and Brummelkamp et al. Science 2002, 296:550-553). These reports describe methods to generate siRNAs capable of specifically targeting numerous endogenously and exogenously expressed genes.

For methods related to the delivery of siRNAs, see, for example, Shen et al (FEBS letters 539: 111-114 (2003)), Xia et al., Nature Biotechnology 20: 1006-1010 (2002), Reich et al., Molecular Vision 9: 210-216 (2003), Sorensen et al. (J. Mol. Biol. 327: 761-766 (2003), Lewis et al., Nature Genetics 32: 107-108 (2002) and Simeoni et al., Nucleic Acids Research 31, 11: 2717-2724 (2003). siRNA has recently been successfully used for inhibition in primates; for further details, see, for example, Tolentino et al., Retina 24(1) February 2004 pp 132-138.

In some embodiments the oligoribonucleotide according to embodiments disclosed herein comprises modified siRNA. In various embodiments the siRNA comprises an RNA duplex comprising a first strand and a second strand, whereby the first strand comprises a ribonucleotide sequence at least partially complementary to about 18 to about 40 consecutive nucleotides of a target nucleic acid, and the second strand comprises ribonucleotide sequence at least partially complementary to the first strand and wherein said first strand and/or said second strand comprises a plurality of groups of modified ribonucleotides having a modification at the 2'-position of the sugar moiety whereby within each strand each group of modified ribonucleotides is flanked on one or both sides by a group of flanking ribonucleotides whereby each ribonucleotide forming the group of flanking ribonucleotides is selected from an unmodified ribonucleotide or a ribonucleotide having a modification different from the modification of the groups of modified ribonucleotides.

### Ribozymes

In some embodiments, the Cdc42-specific inhibitor can be a ribozyme. The term "ribozyme" refers to an RNA molecule that possesses RNA catalytic ability and cleaves a specific site in a target RNA. In accordance with the embodiments disclosed herein, ribozymes which cleave mRNA (e.g., Cdc42 mRNA) may be utilized as inhibitors. This may be necessary in cases where antisense therapy is limited by stoichiometric considerations (Sarver et al., 1990, Gene Regulation and Aids, pp. 305-325). Ribozymes can then be used that will target the a gene associated with a bone marrow disease. The number of RNA molecules that are cleaved by a ribozyme is greater than the number predicted by stochiochemistry. (Hampel and Tritz, 1989; Uhlenbeck, 1987).

Ribozymes catalyze the phosphodiester bond cleavage of RNA. Several ribozyme structural families have been identified including Group I introns, RNase P, the hepatitis delta virus ribozyme, hammerhead ribozymes and the hairpin ribozyme originally derived from the negative strand of the tobacco ringspot virus satellite RNA (sTRSV) (Sullivan, 1994; U.S. Pat. No. 5,225,347). The latter two families are derived from viroids and virusoids, in which the ribozyme is believed to separate monomers from oligomers created during rolling circle replication (Symons, 1989 and 1992). Hammerhead and hairpin ribozyme motifs are most commonly adapted for trans-cleavage of mRNAs for gene therapy (Sullivan, 1994). In general the ribozyme has a length of from about 30-100 nucleotides. Delivery of ribozymes is similar to that of AS fragments and/or siRNA molecules.

The term "nucleic acids," as used herein, may be DNA or RNA or modified versions thereof. Nucleic acids may also include modified nucleotides that permit correct read through by a polymerase and do not alter expression of a polypeptide encoded by that nucleic acid. The terms "nucleic acid" and "oligonucleotide" are used interchangeably to refer to a molecule comprising multiple nucleotides. As used herein, the terms refer to oligoribonucleotides as well as oligodeoxyribonucleotides. The terms shall also include polynucleosides (e.g., a polynucleotide minus the phosphate) and any other organic base containing polymer. Nucleic acids include vectors, e.g., plasmids, as well as oligonucleotides. Nucleic acid molecules can be obtained from existing nucleic acid sources, but are preferably synthetic (e.g., produced by oligonucleotide synthesis).

Polynucleotides to be used according to embodiments disclosed herein may undergo modifications so as to possess improved therapeutic properties. Modifications or analogs of nucleotides can be introduced to improve the therapeutic properties of polynucleotides. Improved properties include increased nuclease resistance and/or increased ability to permeate cell membranes. Nuclease resistance, where needed, is provided by any method known in the art that does not interfere with biological activity of the AS polynucleotide, siRNA, cDNA and/or ribozymes as needed for the method of use and delivery (Iyer et al., 1990; Eckstein, 1985; Spitzer and Eckstein, 1988; Woolf et al., 1990; Shaw et al., 1991). Modifications that can be made to oligonucleotides in order to enhance nuclease resistance include modifying the phosphorous or oxygen heteroatom in the phosphate backbone. These include preparing methyl phosphonates, phosphorothioates, phosphorodithioates and morpholino oligomers. In one embodiment it is provided by having phosphorothioate bonds linking between the four to six 3'-terminus nucleotide bases. Alternatively, phosphorothioate bonds link all the nucleotide bases. Other modifications known in the art may be used where the biological activity is retained, but the stability to nucleases is substantially increased.

All analogues of, or modifications to, a polynucleotide may be employed with the embodiments disclosed herein, provided that said analogue or modification does not substantially affect the function of the polynucleotide. The nucleotides can be selected from naturally occurring or synthetic modified bases. Naturally occurring bases include adenine, guanine, cytosine, thymine and uracil. Modified bases of nucleotides include inosine, xanthine, hypoxanthine, 2-aminoadenine, 6-methyl, 2-propyl and other alkyl adenines, 5-halo uracil, 5-halo cytosine, 6-aza cytosine and 6-aza thymine, psuedo uracil, 4-thiuracil, 8-halo adenine, 8-aminoadenine, 8-thiol adenine, 8-thiolalkyl adenines, 8-hydroxyl adenine and other 8-substituted adenines, 8-halo guanines, 8-amino guanine, 8-thiol guanine, 8-thioalkyl guanines, 8-hydroxyl guanine and other substituted guanines, other aza and deaza adenines, other aza and deaza guanines, 5-trifluoromethyl uracil and 5-trifluoro cytosine.

In addition, analogues of polynucleotides can be prepared wherein the structure of the nucleotide is fundamentally altered and that are better suited as therapeutic or experimental reagents. An example of a nucleotide analogue is a peptide nucleic acid (PNA) wherein the deoxyribose (or ribose) phosphate backbone in DNA (or RNA is replaced with a polyamide backbone which is similar to that found in peptides. PNA analogues have been shown to be resistant to degradation by enzymes and to have extended lives in vivo and in vitro. Further, PNAs have been shown to bind stronger to a complementary DNA sequence than a DNA molecule. This observation is attributed to the lack of charge repulsion between the PNA strand and the DNA strand. Other modifications that can be made to oligonucleotides include polymer backbones, cyclic backbones, or acyclic backbones, as well as LNA ("locked nucleic acid").

Embodiments disclosed herein also include nucleic acids (e.g., siRNA) that can have the following degrees of homology or identity to a Cdc42-specific inhibitory nucleic acid: 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or a range bounded by any two of the aforementioned percentages. Candidate Cdc42-specific inhibitory nucleic acids having greater than or equal to 35% homology or identity can be identified by methods known in the art and can be subsequently examined using functional assays, for example, the assays described herein and those known in the art.

The term "homology" refers to the percent of identity between two polynucleotide or two polypeptide moieties. The correspondence between the sequence from one moiety to another can be determined by techniques known in the art. For example, homology can be determined by a direct comparison of the sequence information between two polynucleotide or polypeptide molecules by aligning the sequence information and using readily available computer programs. Alternatively, homology can be determined by hybridization of polynucleotides under conditions, which form stable duplexes between homologous regions, followed by digestion with single-stranded-specific nuclease(s), and size determination of the digested fragments. Two DNA, or two polypeptide sequences are "substantially homologous" to each other when at least about 80%, preferably at least about 90%, and most preferably at least about 95% of the nucleotides or amino acids match over a defined length of the molecules, as determined using the methods above.

### Preparation of Peptides and Polypeptides

In some embodiments, the Cdc42-specific inhibitor can be a polypeptide (e.g., a dominant negative peptide, an antibody, or an affibody). Polypeptides may be produced, for example, via several methods known in the art (e.g., synthetically or via recombinant methods).

The terms "polypeptide," "peptide," and "protein" are used interchangeably herein to refer to a polymer of amino acid residues. The terms apply to amino acid polymers in which one or more amino acid residue is an analog or mimetic of a corresponding naturally occurring amino acid, as well as to naturally occurring amino acid polymers. Polypeptides can be modified, e.g., by the addition of carbohydrate residues to form glycoproteins. The terms "polypeptide," "peptide," and "protein" include glycoproteins, as well as non-glycoproteins. Polypeptide products can be biochemically synthesized such as by employing standard solid phase techniques. Such methods include but are not limited to exclusive solid phase synthesis, partial solid phase synthesis methods, fragment condensation, classical solution synthesis. These methods are preferably used when the peptide is relatively short (e.g., 10 kDa) and/or when it cannot be produced by recombinant techniques (e.g., not encoded by a nucleic acid sequence) and therefore involves different chemistry. Solid phase polypeptide synthesis procedures are well known in the art and further described by John Morrow Stewart and Janis Dillalia Young, Solid Phase Peptide Syntheses (2nd Ed., Pierce Chemical Company, 1984). Synthetic polypeptides can optionally be purified by preparative high performance liquid chromatography [Creighton T. (1983) Proteins, structures and molecular principles. WH Freeman and Co. N.Y.], after which their composition can be confirmed via amino acid sequencing. In cases where large amounts of a polypeptide are desired, it can be generated using recombinant techniques such as described by Bitter et al., (1987) Methods in Enzymol. 153:516-544, Studier et al. (1990) Methods in Enzymol. 185:60-89, Brisson et al. (1984) Nature 310:511-514, Takamatsu et al. (1987) EMBO J. 6:307-311, Coruzzi et al. (1984) EMBO J. 3:1671-1680 and Brogli et al., (1984) Science 224:838-843, Gurley et al. (1986) Mol. Cell. Biol. 6:559-565 and Weissbach & Weissbach, 1988, Methods for Plant Molecular Biology, Academic Press, NY, Section VIII, pp 421-463.

In some embodiments, the method of making the polypeptides or fragments thereof is to clone a polynucleotide comprising the cDNA of the gene into an expression vector and culture the cell harboring the vector so as to express the encoded polypeptide, and then purify the resulting polypeptide, all performed using methods known in the art as described in, for example, Marshak et al., "Strategies for Protein Purification and Characterization. A laboratory course manual." CSHL Press (1996). (in addition, see, e.g., Bibl Haematol. 1965; 23:1165-74 Appl Microbiol. 1967 July; 15(4):851-6; Can J. Biochem. 1968 May; 46(5):441-4; Biochemistry. 1968 July; 7(7):2574-80; Arch Biochem Biophys. 1968 Sep. 10; 126(3):746-72; Biochem Biophys Res Commun. 1970 Feb. 20; 38(4):825-30).).

The expression vector can include a promoter for controlling transcription of the heterologous material and can be either a constitutive or inducible promoter to allow selective transcription. Enhancers that can be required to obtain necessary transcription levels can optionally be included. The expression vehicle can also include a selection gene.

Vectors can be introduced into cells or tissues by any one of a variety of methods known within the art. Such methods can be found generally described in Sambrook et al., Molecular Cloning: A Laboratory Manual, Cold Springs Harbor Laboratory, New York (1989, 1992), in Ausubel et al., Current Protocols in Molecular Biology, John Wiley and Sons, Baltimore, Md. (1989), Vega et al., Gene Targeting, CRC Press, Ann Arbor, Mich. (1995), Vectors: A Survey of Molecular Cloning Vectors and Their Uses, Butterworths, Boston Mass. (1988) and Gilboa et al. (1986).

### Preparation of Anti- Cdc42 Antibodies

Antibodies that bind to Cdc42 or a fragment derived therefrom may be prepared using an intact polypeptide or fragments containing smaller polypeptides as the immunizing antigen. For example, it may be desirable to produce antibodies that specifically bind to the N- or C-terminal or any other suitable domains of Cdc42. The polypeptide used to immunize an animal can be derived from translated cDNA or chemical synthesis and can be conjugated to a carrier protein, if desired. Such commonly used carriers which are chemically coupled to the polypeptide include keyhole limpet hemocyanin (KLH), thyroglobulin, bovine serum albumin (BSA) and tetanus toxoid. The coupled polypeptide is then used to immunize the animal.

If desired, polyclonal or monoclonal antibodies can be further purified, for example by binding to and elution from a matrix to which the polypeptide or a peptide to which the antibodies were raised is bound. Those skilled in the art know various techniques common in immunology for purification and/or concentration of polyclonal as well as monoclonal antibodies (Coligan et al, Unit 9, Current Protocols in Immunology, Wiley Interscience, 1994).

Methods for making antibodies of all types, including fragments, are known in the art (See for example, Harlow and Lane, Antibodies: A Laboratory Manual, Cold Spring Harbor Laboratory, New York (1988)). Methods of immunization, including all necessary steps of preparing the immunogen in a suitable adjuvant, determining antibody binding, isolation of antibodies, methods for obtaining monoclonal antibodies, and humanization of monoclonal antibodies are all known to the skilled artisan

The antibodies may be humanized antibodies or human antibodies. Antibodies can be humanized using a variety of techniques known in the art including CDR-grafting (EP239,400: PCT publication WO0.91/09967; U.S. Pat. Nos. 5,225,539; 5,530,101; and 5,585,089, veneering or resurfacing (EP 592,1 06; EP 519,596; Padlan, Molecular Immunology 28(4/5):489-498 (1991); Studnicka et al., Protein Engineering 7(6):805-814 (1994); Roguska et al., PNAS 91:969-973 (1994)), and chain shuffling (U.S. Pat. No. 5,565,332).

The monoclonal antibodies as defined include antibodies derived from one species (such as murine, rabbit, goat., rat, human, etc.) as well as antibodies derived from two (or more) species, such as chimeric and humanized antibodies.

Completely human antibodies are particularly desirable for therapeutic treatment of human patients. Human antibodies can be made by a variety of methods known in the art including phage display methods using antibody libraries derived from human immunoglobulin sequences. See also U.S. Pat. Nos. 4,444,887 and 4,716,111; and PCT publications WO 98/46645, WO 98/50433, WO 98/24893, WO 98/16654, WO 96/34096, WO 96/33735, and WO 91/10741, each of which is incorporated herein by reference in its entirety.

Additional information regarding all types of antibodies, including humanized antibodies, human antibodies and antibody fragments can be found in WO 01/05998, which is incorporated herein by reference in its entirety.

Neutralizing antibodies can be prepared by the methods discussed above, possibly with an additional step of screening for neutralizing activity by, for example, a survival assay.

Embodiments disclosed herein also relate to the preparation and use of affibodies, binding proteins of non-Ig origin developed by combinatorial protein engineering principles, as described, for example, in Nygren PA 2008 FEBS Journal 275:2668-2676.

The polypeptides employed in embodiments disclosed herein may also be modified, optionally chemically modified, in order to improve their therapeutic activity. "Chemically modified"--when referring to the polypeptides, refers to a polypeptide where at least one of its amino acid residues is modified either by natural processes, such as processing or other post-translational modifications, or by chemical modification techniques which are well known in the art. Among the numerous known modifications typical, but not exclusive examples include: acetylation, acylation, amidation, ADP-ribosylation, glycosylation, GPI anchor formation, covalent attachment of a lipid or lipid derivative, methylation, myristylation, pegylation, prenylation, phosphorylation, ubiqutination, or any similar process.

Additional possible polypeptide modifications (such as those resulting from nucleic acid sequence alteration) include substitutions, deletions, and insertions.

A "conservative substitution" refers to the substitution of an amino acid in one class by an amino acid of the same class, where a class is defined by common physicochemical amino acid side chain properties and high substitution frequencies in homologous polypeptides found in nature, as determined, for example, by a standard Dayhoff frequency exchange matrix or BLOSUM matrix.

A "non-conservative substitution" refers to the substitution of an amino acid in one class with an amino acid from another class; for example, substitution of an Ala, a class II residue, with a class III residue such as Asp, Asn, Glu, or Gln.

A "deletion" refers to a change in either nucleotide or amino acid sequence in which one or more nucleotides or amino acid residues, respectively, are absent.

An "insertion" or "addition" refers to a change in a nucleotide or amino acid sequence which has resulted in the addition of one or more nucleotides or amino acid residues, respectively, as compared to the naturally occurring sequence.

A "substitution" refers to the replacement of one or more nucleotides or amino acids by different nucleotides or amino acids, respectively. As regards amino acid sequences the substitution may be conservative or non-conservative.

Embodiments disclosed herein also include polypeptides (e.g., dominant negative polypeptides or antibodies) that can have the following degrees of homology or identity to a Cdc42-specific inhibitory polypeptide: 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%,, 87%, 88%, 89%, 90%, 91%, 92%, 93%, 94%, 95%, 96%, 97%, 98%, 99%, or a range bounded by any two of the aforementioned percentages. Candidate Cdc42-specific inhibitory polypeptides having greater than or equal to 35% homology or identity can be identified by methods known in the art and can be subsequently examined using functional assays, for example, the assays described herein and those known in the art.

### Pharmaceutical Compositions and Administration

Also provided herein are pharmaceutical compositions for use with the methods provided herein. In some embodiments, the pharmaceutical compositions comprise a Cdc42-specific inhibitor and a pharmaceutically acceptable carrier.

Compounds, or mixtures of compounds described herein, can be synthetic, naturally-occurring, or a combination thereof. Compounds, or mixtures of compounds described herein can comprise amino acids, nucleotides, hydrocarbons, lipids, polysaccharides, etc. Compounds, or mixtures of compounds described herein preferably comprise a Cdc42-specific inhibitor (e.g., CASIN). Compounds, or mixtures of compounds described herein, can be formulated into pharmaceutical composition comprising a pharmaceutically acceptable carrier and other excipients as apparent to the skilled worker. Such composition can additionally contain effective amounts of other compounds, especially for the treatment of conditions, diseases, and/or disorders described herein.

Some embodiments comprise the administration of a pharmaceutically effective quantity of active agent or its pharmaceutically acceptable salts or esters, active agent analogs or their pharmaceutically acceptable salts or esters, or a combination thereof.

The compositions and preparations described preferably contain at least 0.1 % of active agent. The percentage of the compositions and preparations can, of course, be varied, and can contain between about 2% and 60% of the weight of the amount administered. Preferably, the percentage of the compositions and preparations can contain between about 2, 5, 10, or 15% and 30, 35, 40, 45, 50, 55, or 60% of the weight of the amount administered. The amount of active compounds in such pharmaceutically useful compositions and preparations is such that a suitable dosage will be obtained.

The active agent can form salts, which are also within the scope of the preferred embodiments. Reference to a compound of the active agent herein is understood to include reference to salts thereof, unless otherwise indicated. The term "salt(s)", as employed herein, denotes acidic and/or basic salts formed with inorganic and/or organic acids and bases. In addition, when an active agent contains both a basic moiety, such as, but not limited to an amine or a pyridine or imidazole ring, and an acidic moiety, such as, but not limited to a carboxylic acid, zwitterions ("inner salts") can be formed and are included within the term "salt(s)" as used herein. Pharmaceutically acceptable (e.g., non-toxic, physiologically acceptable) salts are preferred, although other salts are also useful, e.g., in isolation or purification steps, which can be employed during preparation. Salts of the compounds of the active agent can be formed, for example, by reacting a compound of the active agent with an amount of acid or base, such as an equivalent amount, in a medium such as one in which the salt precipitates or in an aqueous medium followed by lyophilization.

The active agents which contain a basic moiety, such as, but not limited to an amine or a pyridine or imidazole ring, can form salts with a variety of organic and inorganic acids. Exemplary acid addition salts include acetates (such as those formed with acetic acid or trilialoacetic acid, for example, trifluoroacetic acid), adipates, alginates, ascorbates, aspartates, benzoates, benzenesulfonates, bisulfates, borates, butyrates, citrates, camphorates, camphorsulfonates, cyclopentanepropionates, digluconates, dodecylsulfates, ethanesulfonates, fumarates, glucoheptanoates, glycerophosphates, hemisulfates, heptanoates, hexanoates, hydrochlorides (formed with hydrochloric acid), hydrobromides (formed with hydrogen bromide), hydroiodides, 2-hydroxyethanesulfonates, lactates, maleates (formed with maleic acid), methanesulfonates (formed with methanesulfonic acid), 2-naphthalenesulfonates, nicotinates, nitrates, oxalates, pectinates, persulfates, 3-phenylpropionates, phosphates, picrates, pivalates, propionates, salicylates, succinates, sulfates (such as those formed with sulfuric acid), sulfonates (such as those mentioned herein), tartrates, thiocyanates, toluenesulfonates such as tosylates, undecanoates, and the like.

The active agents which contain an acidic moiety, such as, but not limited to a carboxylic acid, can form salts with a variety of organic and inorganic bases. Exemplary basic salts include ammonium salts, alkali metal salts such as sodium, lithium, and potassium salts, alkaline earth metal salts such as calcium and magnesium salts, salts with organic bases (for example, organic amines) such as benzathines, dicyclohexylamines, hydrabamines [formed with N,N-bis(dehydro-abietyl)ethylenediamine], N-methyl-D-glucamines, N-methyl-D-glucamides, t-butyl amines, and salts with amino acids such as arginine, lysine and the like. Basic nitrogen-containing groups can be quaternized with agents such as lower alkyl halides (e.g., methyl, ethyl, propyl, and butyl chlorides, bromides and iodides), dialkyl sulfates (e.g., dimethyl, diethyl, dibutyl, and diamyl sulfates), long chain halides (e.g., decyl, lauryl, myristyl and stearyl chlorides, bromides and iodides), aralkyl halides (e.g., benzyl and phenethyl bromides), and others.

Prodrugs and solvates of the compounds of the preferred embodiments are also contemplated herein. The term "prodrug", as employed herein, denotes a compound which, upon administration to a subject, undergoes chemical conversion by metabolic or chemical processes to yield a compound of the active agent, and/or a salt and/or solvate thereof. Solvates of the active agent are preferably hydrates.

Active agent, and salts thereof, can exist in their tautomeric form (for example, as an amide or imino ether). All such tautomeric forms are contemplated herein as part of the preferred embodiments.

All stereoisomers of the present compounds, such as those, for example, which can exist due to asymmetric carbons on any of the substituents, including enantiomeric forms (which can exist even in the absence of asymmetric carbons) and diastereomeric forms, are contemplated and within the scope of the preferred embodiments. Individual stereoisomers of the compounds of the preferred embodiments can, for example, be substantially free of other isomers, or can be admixed, for example, as racemates or with all other or other selected, stereoisomers. The chiral centers of the preferred embodiments can have the S or R configuration as defined by the IUPAC 1974 Recommendations.

When the compounds according to the preferred embodiments are in the forms of salts, they are preferably pharmaceutically acceptable salts. Such salts include pharmaceutically acceptable acid addition salts, pharmaceutically acceptable base addition salts, pharmaceutically acceptable metal salts, ammonium and alkylated ammonium salts. Acid addition salts include salts of inorganic acids as well as organic acids. Representative examples of suitable inorganic acids include hydrochloric, hydrobromic, hydroiodic, phosphoric, sulfuric, nitric acids and the like. Representative examples of suitable organic acids include formic, acetic, trichloroacetic, trifluoroacetic, propionic, benzoic, cinnamic, citric, fumaric, glycolic, lactic, maleic, malic, malonic, mandelic, oxalic, picric, pyruvic, salicylic, succinic, methanesulfonic, ethanesulfonic, tartaric, ascorbic, pamoic, bismethylene salicylic, ethanedisulfonic, gluconic, citraconic, aspartic, stearic, palmitic, EDTA, glycolic, p-aminobenzoic, glutamic, benzenesulfonic, p-toluenesulfonic acids, sulphates, nitrates, phosphates, perchlorates, borates, acetates, benzoates, hydroxynaphthoates, glycerophosphates, ketoglutarates and the like. Examples of metal salts include lithium, sodium, potassium, magnesium salts and the like. Examples of ammonium and alkylated ammonium salts include ammonium, methylammonium, dimethylammonium, trimethylammonium, ethylammonium, hydroxyethylammonium, diethylammonium, butylammonium, tetramethylammonium salts and the like. Examples of organic bases include lysine, arginine, guanidine, diethanolamine, choline and the like.

The pharmaceutically acceptable salts can be prepared by reacting the active agent with 1 to 4 equivalents of a base such as sodium hydroxide, sodium methoxide, sodium hydride, potassium t-butoxide, calcium hydroxide, magnesium hydroxide and the like, in solvents like ether, THF, methanol, t-butanol, dioxane, isopropanol, ethanol, etc. Mixture of solvents can be used. Organic bases like lysine, arginine, diethanolamine, choline, guandine and their derivatives etc. can also be used. Alternatively, acid addition salts wherever applicable are prepared by treatment with acids such as hydrochloric acid, hydrobromic acid, nitric acid, sulfuric acid, phosphoric acid, p-toluenesulphonic acid, methanesulfonic acid, fonic acid, acetic acid, citric acid, maleic acid salicylic acid, hydroxynaphthoic acid, ascorbic acid, palmitic acid, succinic acid, benzoic acid, benzenesulfonic acid, tartaric acid and the like in solvents like ethyl acetate, ether, alcohols, acetone, TFIF, dioxane, etc. Mixture of solvents can also be used.

The compounds can be formulated in various forms, including solid and liquid forms, such as tablets, gel, syrup, powder, aerosol, creams, lotions, tinctures, foams, etc.

The compositions of the preferred embodiments can contain physiologically acceptable diluents, fillers, lubricants, excipients, solvents, binders, stabilizers, and the like. Diluents that can be used in the compositions include but are not limited to dicalcium phosphate, calcium sulphate, lactose, cellulose, kaolin, mannitol, sodium chloride, dry starch, powdered sugar and for prolonged release tablet-hydroxy propyl methyl cellulose (HPMC). The binders that can be used in the compositions include but are not limited to starch, gelatin and fillers such as sucrose, glucose, dextrose and lactose.

Natural and synthetic gums that can be used in the compositions include but are not limited to sodium alginate, ghatti gum, carboxymethyl cellulose, methyl cellulose, polyvinyl pyrrolidone and veegum. Excipients that can be used in the compositions include but are not limited to microcrystalline cellulose, calcium sulfate, dicalcium phosphate, starch, magnesium stearate, lactose, and sucrose. Stabilizers that can be used include but are not limited to polysaccharides such as acacia, agar, alginic acid, guar gum and tragacanth, amphotsics such as gelatin and synthetic and semi-synthetic polymers such as carbomer resins, cellulose ethers and carboxymethyl chitin.

Solvents that can be used include but are not limited to Ringers solution, water, distilled water, dimethyl sulfoxide to 50% in water, propylene glycol (neat or in water), phosphate buffered saline, balanced salt solution, glycol and other conventional fluids.

The dosages and dosage regimen in which the compounds are administered will vary according to the dosage form, mode of administration, the condition being treated and particulars of the patient being treated. Accordingly, optimal therapeutic concentrations will be best determined at the time and place through routine experimentation.

The compounds according to the preferred embodiments can also be used enterally. Orally, the compounds according to the preferred embodiments are suitable administered at the rate of 100 µg to 100 mg per day per kg of body weight. Preferably, orally, the compounds according to the preferred embodiments are suitable administered at the rate of about 100, 150, 200, 250, 300, 350, 400, ,450, or 500 µg to about 1, 5, 10, 25, 50, 75, 100 mg per day per kg of body weight. The required dose can be administered in one or more portions. For oral administration, suitable forms are, for example, tablets, gel, aerosols, pills, dragees, syrups, suspensions, emulsions, solutions, powders and granules; a preferred method of administration consists in using a suitable form containing from 1 mg to about 500 mg of active substance. Preferably, a method of administration consists in using a suitable form containing from about 1, 2, 5, 10, 25, or 50 mg to about 100, 200, 300, 400, 500 mg of active substance.

The compounds according to the preferred embodiments can also be administered parenterally in the form of solutions or suspensions for intravenous or intramuscular perfusions or injections. In that case, the compounds according to the preferred embodiments are generally administered at the rate of about 10 µg to 10 mg per day per kg of body weight; a preferred method of administration consists of using solutions or suspensions containing approximately from 0.01 mg to 1 mg of active substance per ml. Preferably, the compounds according to the preferred embodiments are generally administered at the rate of about 10, 20, 30, 40, 50, 60, 70, 80, 90, or 100 µg to 1, 2, 3, 4, 5, 6, 7, 8, 9, or 10 mg per day per kg of body weight; a preferred method of administration consists of using solutions or suspensions containing approximately from 0.01, 0.02, 0.03, 0.04, or 0.5 mg to 0.1, 0.2, 0.3, 0.4, 0.5, 0.6, 0.7, 0.8, 0.9, or 1 mg of active substance per ml.

The active compounds and/or pharmaceutical compositions of the embodiments disclosed herein can be administered according to various routes, typically by injection or oral administration, including local or systemic administration(s). Furthermore, repeated administrations can be performed, if needed.

For ex vivo administration, the active agent can be administered by any standard method that would maintain viability of the cells, such as by adding it to culture medium (appropriate for the target cells) and adding this medium directly to the cells. As is known in the art, any medium used in this method can be aqueous and non-toxic so as not to render the cells non-viable. In addition, it can contain standard nutrients for maintaining viability of cells, if desired. For in vivo administration, the complex can be added to, for example, to a pharmaceutically acceptable carrier, e.g., saline and buffered saline, and administered by any of several means known in the art. Examples of administration include parenteral administration, e.g., by intravenous injection including regional perfusion through a blood vessel supplying the tissues(s) or organ(s) having the target cell(s), or by inhalation of an aerosol, subcutaneous or intramuscular injection, topical administration such as to skin wounds and lesions, direct transfection into, e.g., bone marrow cells prepared for transplantation and subsequent transplantation into the subject, and direct transfection into an organ that is subsequently transplanted into the subject. Further administration methods include oral administration, particularly when the active agent is encapsulated, or rectal administration, particularly when the active agent is in suppository form.

It is contemplated that such target cells can be located within a subject or human patient, in which case a safe and effective amount of the active agent, in pharmacologically acceptable form, would be administered to the patient. Generally speaking, it is contemplated that useful pharmaceutical compositions of the preferred embodiments will include the selected active compound derivative in a convenient amount, e.g., from about 0.001% to about 10% (w/w) that is diluted in a pharmacologically or physiologically acceptable carrier, such as, for example, phosphate buffered saline. The route of administration and ultimate amount of material that is administered to the subject under such circumstances will depend upon the intended application and will be apparent to those of skill in the art in light of the examples which follow.

Any composition chosen should be of low or non-toxicity to the cell. Toxicity for any given compound can vary with the concentration of compound used. It is also beneficial if the compound chosen is metabolized or eliminated by the body and if this metabolism or elimination is done in a manner that will not be harmfully toxic.

The examples are illustrative of the types of compounds to be used in the method claimed herein; the list is not exhaustive. Derivatives of the above compounds that fit the criteria of the claims are preferably also be considered when choosing an active compound.

The compound is preferably administered such that a therapeutically effective concentration of the compound is in contact with the affected cells of the body. The dose administered to a subject, particularly a human, in the context of the preferred embodiments is preferably sufficient to effect a therapeutic response in the subject over a reasonable period of time. The dose will be determined by the strength of the particular compound employed and the condition of the subject, as well as the body weight of the subject to be treated. The existence, nature, and extent of any adverse side effects that might accompany the administration of a particular compound also will determine the size of the dose and the particular route of administration employed with a particular patient. In general, the compounds of the preferred embodiments are therapeutically effective at low doses. The generally useful dose range is from about 0.001 mM, or less, to about 100 mM, or more. Preferably, the effective dose range is from about 0.01, 0.05, 0.1, 0.5, 0.6, 0.7, 0.8, or 0.9 mM, to about 1, 2, 3, 4, 5, 6, 7, 8, 9, or 10 mM. Accordingly, the compounds will be generally administered in low doses.

The compound can be administered in a pharmaceutically acceptable carrier. Pharmaceutically acceptable carriers are well-known to those who are skilled in the art. The choice of carrier will be determined in part by the particular compound, as well as by the particular method used to administer the composition. Accordingly, there is a wide variety of suitable formulations of the pharmaceutical composition of the preferred embodiments.

The compounds can be administered orally, topically, parenterally, by inhalation or spray, vaginally, rectally or sublingually in dosage unit formulations. The term "administration by injection" includes but is not limited to: intravenous, intraarticular, intramuscular, subcutaneous and parenteral injections, as well as use of infusion techniques. Dermal administration can include topical application or transdermal administration. One or more compounds can be present in association with one or more non-toxic pharmaceutically acceptable carriers and if desired other active ingredients.

Compositions intended for oral use can be prepared according to any suitable method known to the art for the manufacture of pharmaceutical compositions. Such compositions can contain one or more agents selected from the group consisting of diluents, sweetening agents, flavoring agents, coloring agents and preserving agents in order to provide palatable preparations. Tablets contain the active ingredient in admixture with nontoxic pharmaceutically acceptable excipients that are suitable for the manufacture of tablets. These excipients can be, for example, inert diluents, such as calcium carbonate, sodium carbonate, lactose, calcium phosphate or sodium phosphate; granulating and disintegrating agents, for example, corn starch, or alginic acid; and binding agents, for example magnesium stearate, stearic acid or talc. The tablets can be uncoated or they can be coated by known techniques to delay disintegration and adsorption in the gastrointestinal tract and thereby provide a sustained action over a longer period. For example, a time delay material such as glyceryl monostearate or glyceryl distearate can be employed. These compounds can also be prepared in solid, rapidly released form.

Formulations for oral use can also be presented as hard gelatin capsules wherein the active ingredient is mixed with an inert solid diluent, for example, calcium carbonate, calcium phosphate or kaolin, or as soft gelatin capsules wherein the active ingredient is mixed with water or an oil medium, for example peanut oil, liquid paraffin or olive oil.

Aqueous suspensions containing the active materials in admixture with excipients suitable for the manufacture of aqueous suspensions can also be used. Such excipients are suspending agents, for example sodium carboxymethylcellulose, methylcellulose, hydroxypropyl-methylcellulose, sodium alginate, polyvinylpyrrolidone, gum tragacanth and gum acacia; dispersing or wetting agents can be a naturally-occurring phosphatide, for example, lecithin, or condensation products of an alkylene oxide with fatty acids, for example polyoxyethylene stearate, or condensation products of ethylene oxide with long chain aliphatic alcohols, for example heptadecaethylene oxycetanol, or condensation products of ethylene oxide with partial esters derived from fatty acids and hexitol such as polyoxyethylene sorbitol monooleate, or condensation products of ethylene oxide with partial esters derived from fatty acids and hexitol anhydrides, for example polyethylene sorbitan monooleate. The aqueous suspensions can also contain one or more preservatives, for example ethyl, or n-propyl p-hydroxybenzoate, one or more coloring agents, one or more flavoring agents, and one or more sweetening agents, such as sucrose or saccharin.

Dispersible powders and granules suitable for preparation of an aqueous suspension by the addition of water provide the active ingredient in admixture with a dispersing or wetting agent, suspending agent and one or more preservatives. Suitable dispersing or wetting agents and suspending agents are exemplified by those already mentioned above. Additional excipients, for example, sweetening, flavoring and coloring agents, can also be present.

The compounds can also be in the form of non-aqueous liquid formulations, e.g., oily suspensions which can be formulated by suspending the active ingredients in a vegetable oil, for example arachis oil, olive oil, sesame oil or peanut oil, or in a mineral oil such as liquid paraffin. The oily suspensions can contain a thickening agent, for example beeswax, hard paraffin or cetyl alcohol. Sweetening agents such as those set forth above, and flavoring agents can be added to provide palatable oral preparations. These compositions can be preserved by the addition of an anti-oxidant such as ascorbic acid.

Compounds of the preferred embodiments can also be administrated transdermally using methods known to those skilled in the art. For example, a solution or suspension of an active agent in a suitable volatile solvent optionally containing penetration enhancing agents can be combined with additional additives known to those skilled in the art, such as matrix materials and bacteriocides. After sterilization, the resulting mixture can be formulated following known procedures into dosage forms. In addition, on treatment with emulsifying agents and water, a solution or suspension of an active agent can be formulated into a lotion or salve.

Suitable solvents for processing transdermal delivery systems are known to those skilled in the art, and include lower alcohols such as ethanol or isopropyl alcohol, lower ketones such as acetone, lower carboxylic acid esters such as ethyl acetate, polar ethers such as tetrahydrofuran, lower hydrocarbons such as hexane, cyclohexane or benzene, or halogenated hydrocarbons such as dichloromethane, chloroform, trichlorotrifluoroethane, or trichlorofluoroethane. Suitable solvents can also include mixtures of one or more materials selected from lower alcohols, lower ketones, lower carboxylic acid esters, polar ethers, lower hydrocarbons, halogenated hydrocarbons.

Suitable penetration enhancing materials for transdermal delivery system are known to those skilled in the art, and include, for example, monohydroxy or polyhydroxy alcohols such as ethanol, propylene glycol or benzyl alcohol, saturated or unsaturated C8-C18 fatty alcohols such as lauryl alcohol or cetyl alcohol, saturated or unsaturated C8-C18 fatty acids such as stearic acid, saturated or unsaturated fatty esters with up to 24 carbons such as methyl, ethyl, propyl, isopropyl, n-butyl, sec-butyl, isobutyl, tertbutyl or monoglycerin esters of acetic acid, capronic acid, lauric acid, myristinic acid, stearic acid, or palmitic acid, or diesters of saturated or unsaturated dicarboxylic acids with a total of up to about 24 carbons such as diisopropyl adipate, diisobutyl adipate, diisopropyl sebacate, diisopropyl maleate, or diisopropyl fumarate. Additional penetration enhancing materials include phosphatidyl derivatives such as lecithin or cephalin, terpenes, amides, ketones, ureas and their derivatives, and ethers such as dimethyl isosorbid and diethyleneglycol monoethyl ether. Suitable penetration enhancing formulations can also include mixtures of one or more materials selected from monohydroxy or polyhydroxy alcohols, saturated or unsaturated C8-C18 fatty alcohols, saturated or unsaturated C8-C18 fatty acids, saturated or unsaturated fatty esters with up to 24 carbons, diesters of saturated or unsaturated discarboxylic acids with a total of up to 24 carbons, phosphatidyl derivatives, terpenes, amides, ketones, ureas and their derivatives, and ethers.

Suitable binding materials for transdermal delivery systems are known to those skilled in the art and include polyacrylates, silicones, polyurethanes, block polymers, styrenebutadiene copolymers, and natural and synthetic rubbers. Cellulose ethers, derivatized polyethylenes, and silicates can also be used as matrix components. Additional additives, such as viscous resins or oils can be added to increase the viscosity of the matrix.

In some embodiments the composition can comprise, for example a topical formulation. In some embodiments, the topical formulation is a non-transdermal composition, formulated so as to not penetrate beyond the dermal layer. Non-transdermal formulations are known in the art, and include matrical or micellar solutions, bandages, wound dressings, aerosol sprays, foams, non-transdermal topical patches, tinctures, topical administrative agents and the like.

Pharmaceutical compositions of the preferred embodiments can also be in the form of oil-in-water emulsions. The oil phase can be a vegetable oil, for example olive oil or arachis oil, or a mineral oil, for example, liquid paraffin or mixtures of these. Suitable emulsifying agents can be naturally-occurring gums, for example, gum acacia or gum tragacanth, naturally-occurring phosphatides, for example, soy bean, lecithin, and esters or partial esters derived from fatty acids and hexitol anhydrides, for example, sorbitan monooleate, and condensation products of the said partial esters with ethylene oxide, for example, polyoxyethylene sorbitan monooleate. The emulsions can also contain sweetening and flavoring agents. Syrups and elixirs can be formulated with sweetening agents, for example glycerol, propylene glycol, sorbitol or sucrose. Such formulations can also contain a demulcent, a preservative and flavoring and coloring agents.

The compounds can also be administered in the form of suppositories for rectal or vaginal administration of the drug. These compositions can be prepared by mixing the drug with a suitable nonirritating excipient which is solid at ordinary temperatures but liquid at the rectal temperature or vaginal temperature and will therefore melt in the rectum or vagina to release the drug. Such materials include cocoa butter and polyethylene glycols.

For all regimens of use disclosed herein for active agent, the daily oral dosage regimen will preferably be from about 0.01 to about 200 mg/Kg of total body weight. Preferably, the daily oral dosage regimen will preferably be from about 0.01, 0.05, 0.1, 0.5, 1, 2, 3, 4, or 5 to about 10, 50, 100, 110, 120, 130, 140, 150, 160, 170, 180, 190, or200 mg/Kg of total body weight. The daily dosage for administration by injection, including intravenous, intramuscular, subcutaneous and parenteral injections, and use of infusion techniques will preferably be from 0.01 to 200 mg/Kg of total body weight. Preferably, the daily dosage for administration by injection, including intravenous, intramuscular, subcutaneous and parenteral injections, and use of infusion techniques will preferably be from about 0.01, 0.05, 0.1, 0.5, 1, 2, 3, 4, or 5 to about 10, 50, 100, 110, 120, 130, 140, 150, 160, 170, 180, 190, or200 mg/Kg of total body weight. The daily vaginal dosage regime will preferably be from 0.01 to 200 mg/Kg of total body weight. The daily topical dosage regimen will preferably be from 0.01 to 200 mg administered between one to four times daily. The concentration for vaginal dosage and topical dosage will preferably be that required to maintain a daily dose is of from 0.1 to 200 mg/Kg. Preferably, the daily oral dosage regimen will preferably be from about 0.01, 0.05, 0.1, 0.5, 1, 2, 3, 4, or 5 to about 10, 50, 100, 110, 120, 130, 140, 150, 160, 170, 180, 190, or200 mg/Kg of total body weight. The daily inhalation dosage regimen will preferably be from 0.01 to 10 mg/Kg of total body weight. Preferably, the daily inhalation dosage regimen will preferably be from about 0.01, 0.05, 0.1, 0.5, to about 1, 2, 3, 4, 5, or 10, mg/Kg of total body weight.

It will be appreciated by those skilled in the art that the particular method of administration will depend on a variety of factors, all of which are considered routinely when administering therapeutics. It will also be understood, however, that the specific dose level for any given patient will depend upon a variety of factors, including, the activity of the specific compound employed, the age of the patient, the body weight of the patient, the general health of the patient, the gender of the patient, the diet of the patient, time of administration, route of administration, rate of excretion, drug combinations, and the severity of the condition undergoing therapy. It will be further appreciated by one skilled in the art that the optimal course of treatment, i.e., the mode of treatment and the daily number of doses of an active agent or a pharmaceutically acceptable salt thereof given for a defined number of days, can be ascertained by those skilled in the art using conventional treatment tests.

The active compounds can be incorporated into pharmaceutical compositions suitable for administration to a subject, e.g., a human. Such compositions typically comprise the nucleic acid molecule, protein, modulator, or antibody and a pharmaceutically acceptable carrier.

As used herein the language "pharmaceutically acceptable carrier" is intended to include any and all solvents, dispersion media, coatings, antibacterial and antifungal agents, isotonic and absorption delaying agents, and the like, compatible with pharmaceutical administration. The use of such media and agents for pharmaceutically active substances is well known in the art. Except insofar as any conventional media or agent is incompatible with the active compound, such media can be used in the compositions of the preferred embodiments. Supplementary active compounds can also be incorporated into the compositions. A pharmaceutical composition of the preferred embodiments is formulated to be compatible with its intended route of administration. Examples of routes of administration include parenteral, e.g., intravenous, intradermal, subcutaneous, oral (e.g., inhalation), transdermal, non-transdermal (topical), transmucosal, and rectal administration. Solutions or suspensions used for parenteral, intradermal, or subcutaneous application can include the following components: a sterile diluent such as water for injection, saline solution, fixed oils, polyethylene glycols, glycerine, propylene glycol or other synthetic solvents; antibacterial agents such as benzyl alcohol or methyl parabens; antioxidants such as ascorbic acid or sodium bisulfite; chelating agents such as ethylenediaminetetraacetic acid, buffers such as acetates, citrates or phosphates and agents for the adjustment of tonicity such as sodium chloride or dextrose. pH can be adjusted with acids or bases, such as hydrochloric acid or sodium hydroxide. The parenteral preparation can be enclosed in ampoules, disposable syringes or multiple dose vials made of glass or plastic.

In some embodiments, the active compounds are prepared with carriers that will protect the compound against rapid elimination from the body, such as a controlled release formulation, including implants and microencapsulated delivery systems. Biodegradable, biocompatible polymers can be used, such as ethylene vinyl acetate, polyanhydrides, polyglycolic acid, collagen, polyorthoesters, and polylactic acid. Methods for preparation of such formulations will be apparent to those skilled in the art. The materials can also be obtained commercially from Alza Corporation and Nova Pharmaceuticals, Inc. Liposomal suspensions (including liposomes) can also be used as pharmaceutically acceptable carriers. These can be prepared according to methods known to those skilled in the art.

It is especially advantageous to formulate oral or parenteral compositions in dosage unit form for ease of administration and uniformity of dosage. "Dosage unit form" as used herein refers to physically discrete units suited as unitary dosages for the subject to be treated, each unit containing a predetermined quantity of active compound calculated to produce the desired therapeutic effect in association with the required pharmaceutical carrier. The specification for the dosage unit forms of the preferred embodiments are dictated by and directly dependent on the unique characteristics of the active compound and the particular therapeutic effect to be achieved, and the limitations inherent in the art of compounding such an active compound for the treatment of individuals.

As used herein, the terms "effective amount" or "therapeutically effective amount" means the total amount of each active component of the pharmaceutical composition or method that is sufficient to show a meaningful patient benefit, e.g., healing of chronic conditions or in an increase in rate of healing of such conditions, or in a reduction in aberrant conditions. This includes both therapeutic and prophylactic treatments. Accordingly, the compounds can be used at very early stages of a disease, or before early onset, or after significant progression. When applied to an individual active ingredient, administered alone, the term refers to that ingredient alone. When applied to a combination, the term refers to combined amounts of the active ingredients that result in the therapeutic effect, whether administered in combination, serially or simultaneously.

In practicing the method of treatment or use of the preferred embodiments, a therapeutically effective amount of one, two, or more of the active agents of the preferred embodiments is administered to a subject. The active agents of the preferred embodiments can be administered in accordance with the method of the preferred embodiments either alone of in combination with other known therapies. When co-administered with one or more other therapies, the active agents of the preferred embodiments can be administered either simultaneously with the other treatment(s), or sequentially. If administered sequentially, the attending physician will decide on the appropriate sequence of administering the active agents of the preferred embodiments in combination with the other therapy.

Generally, a therapeutically effective amount of active agent (i.e., an effective dosage) ranges from about 0.001 to 5000 mg/kg body weight, more preferably about 0.01 to 1000 mg/kg body weight, more preferably about 0.01 to 500 mg/kg body weight, more preferably about 0.01 to 250 mg/kg body weight, more preferably about 0.01 to 100 mg/kg body weight, more preferably about 0.001 to 60 mg/kg body weight, more preferably about 0.01 to 25 mg/kg body weight, more preferably about 0. 1 to 20 mg/kg body weight, and even more preferably about 1 to 10 mg/kg, 2 to 9 mg/kg, 3 to 8 mg/kg, 4 to 7 mg/kg, or 5 to 6 mg/kg body weight.

The skilled artisan will appreciate that certain factors can influence the dosage required to effectively treat a subject, including but not limited to the severity of the disease or disorder, previous treatments, the general health and/or age of the subject, and other diseases present. Moreover, treatment of a subject with a therapeutically effective amount can include a single treatment or, preferably, can include a series of treatments. In a preferred example, a subject is treated in the range of between about 0. 1 to 20 mg/kg body weight, one time per week for between about 1 to 10 weeks, preferably between 2 to 8 weeks, more preferably between about 3 to 7 weeks, and even more preferably for about 4, 5, or 6 weeks. It will also be appreciated that the effective dosage used for treatment can increase or decrease over the course of a particular treatment. Changes in dosage can result and become apparent from the results of diagnostic assays as described herein.

The preferred embodiments encompass one or more additional agents that modulate expression or activity of Cdc42 GTPase. An agent can, for example, be a small molecule. For example, such small molecules include, but are not limited to, peptides, peptidomimetics, amino acids, amino acid analogs, polynucleotides, polynucleotide analogs, nucleotides, nucleotide analogs, organic or inorganic compounds (i.e., including heteroorganic and organometallic compounds) having a molecular weight less than about 10,000 grams per mole, organic or inorganic compounds having a molecular weight less than about 5,000 grams per mole, organic or inorganic compounds having a molecular weight less than about 1,000 grams per mole, organic or inorganic compounds having a molecular weight less than about 500 grams per mole, and salts, esters, and other pharmaceutically acceptable forms of such compounds.

In one embodiment, the additional agent can be a prenylation inhibitor, such as disclosed by U.S. Pat. Nos. 6,649,638, 5,420,245; 5,574,025; 5,523,430; 5,602,098; 5,631,401; 5,705,686; 5,238,922; 5,470,832; and 6,191,147, all of which are incorporated herein by reference in their entirety.

In another embodiment, the additional agent comprises one or more inhibitor of farnesyl protein transferase (FPTase), prenyl-protein transferase or geranylgeranyl-protein transferase as described in U.S. Pat. Nos. 6,572,850; 6,458,783; 6,423,751; 6,387,926; 6,242,433; 6,191,147; 6,166,067; 6,156,746; 6,083,979; 6,011,029; 5,929,077; 5,928,924; 5,843,941; 5,786,193; 5,629,302; 5,618,964; 5,574,025; 5,567,841; 5,523,430; 5,510,510; 5,470,832; 5,447,922, 6,596,735; 6,586,461; 6,586,447; 6,579,887; 6,576,639; 6,545,020; 6,539,309; 6,535,820; 6,528,523: 6,511,800; 6,500,841; 6,495,564; 6,492,381; 6,458,935; 6,451,812; 6,441,017; 6,440,989; 6,440,974; 6,432,959; 6,426,352; 6,410,541; 6,403,581; 6,399,615; 6,387,948; 6,387,905; 6,387,903; 6,376,496; 6,372,747; 6,362,188; 6,358,968; 6,329,376; 6,316,462; 6,294,552; 6,277,854; 6,268,394; 6,265,382; 6,262,110; 6,258,824: 6,248,756; 6,242,458; 6,239,140; 6,228,865; 6,228,856; 6,225,322; 6,218,401; 6,214,828; 6,214,827; 6,211,193; 6,194,438, which are specifically incorporated herein by reference in their entirety.

A "farnesyl protein transferase inhibitor" or "FPT inhibitor" or "FTI" is defined herein as a compound which: (i) potently inhibits FPT (but generally not geranylgeranyl protein transferase I) and (ii) blocks intracellular farnesylation of ras. FPT catalyzes the addition of an isoprenyl lipid moiety onto a cysteine residue present near the carboxy-terminus of the Ras protein. This is the first step in a post-translational processing pathway that is essential for both Ras membrane-association and Ras-induced oncogenic transformation. A number of FPT inhibitors have been reported, including a variety of peptidomimetic inhibitors as well as other small molecule inhibitors.

Farnesyl transferase inhibitors generally fall into two classes: analogs of farnesyl diphosphate; and protein substrates for farnesyl transferase. Farnesyl transferase inhibitors have been described in U.S. Pat. No. 5,756,528, U.S. Pat. No. 5,141,851, U.S. Pat. No. 5,817,678, U.S. Pat. No. 5,830,868, U.S. Pat. No. 5,834,434, and U.S. Pat. No. 5,773,455, all of which are incorporated herein by reference in their entirety. Among the farnesyl transferase inhibitors shown to be effective for inhibiting the transfer of the farnesyl moiety to Ras-related proteins are L-739,749 (a peptidomimetic analog of the C-A-A-X sequence), L-74-4,832 (a peptidomimetic analog of the C-A-A-X sequence), SCH 44342 (1-(4-pyridylacetyl)-4-(8-chloro-5,6 dihydro-IIH benzo [5,6] cyclohepta [1,2-b]pyridin-11-yhdene)piperidine), BZA-5B (a benzodiazepine peptidomimetic), FTI-276 (a C-A-A-X peptidomimetic), and B1086 (a C-A-A-X peptidomimetic). Administration of farnesyl transferase inhibitors (FTIs) is accomplished by standard methods known to those of skill in the art, most preferably by administration of tablets containing the FTI, and is expected to fall approximately within a range of about 0.1 mg/kg of body to weight to about 20 mg/kg of body weight per day.

In another embodiment, the additional agent comprises one or more inhibitor of geranylgeranyl-protein transferase (GGT) as have been described in U.S. Pat. No. 5,470,832 (Gibbs & Graham), which is incorporated herein by reference in its entirety. These compounds can be administered to an individual in dosage amounts of between 0.5 mg/kg of body weight to about 20 mg/kg of body weight. Alternatively, one or more inhibitors of isoprenylation, including farnesyl transferase (FT) inhibitors and/or geranylgeranyl transferase inhibitors (GGT) are administered to a patient.

In another embodiment, the additional agent comprises one or more toxins such as toxins A and B from C. difficile and C. sordellii lethal toxin (LT). In addition, Rac 1 and Rac2 can be inhibited when Rho is specifically ADP ribosylated by C3 enzyme, which is one of the botulinum toxins, and Staphylococcal toxin EDIN (Narumiya, S. and Morii, S., Cell Signal, 5, 9-19, 1993; Sekine, A. et al., J. Biol. Chem., 264, 8602-8605, 1989, all of which are incorporated herein by reference in their entirety).

It is understood that appropriate doses of small molecule agents depends upon a number of factors within the ken of the ordinarily skilled physician, veterinarian, or researcher. The dose(s) of the small molecule will vary, for example, depending upon the identity, size, and condition of the subject or sample being treated, further depending upon the route by which the composition is to be administered, if applicable, and the effect which the practitioner desires the small molecule to have upon the nucleic acid or polypeptide of the preferred embodiments. Exemplary doses include milligram or microgram amounts of the small molecule per kilogram of subject or sample weight (e.g., about 1 microgram per kilogram to about 500 milligrams per kilogram, about 100 micrograms per kilogram to about 5 milligrams per kilogram, or about 1 microgram per kilogram to about 50 micrograms per kilogram. It is furthermore understood that appropriate doses of a small molecule depend upon the potency of the small molecule with respect to the expression or activity to be modulated. Such appropriate doses can be determined using the assays described herein. When one or more of these small molecules is to be administered to a subject (e.g., a human) in order to modulate expression or activity of a polypeptide or nucleic acid of the preferred embodiments, a physician, veterinarian, or researcher can, for example, prescribe a relatively low dose at first, subsequently increasing the dose until an appropriate response is obtained. In addition, it is understood that the specific dose level for any particular subject will depend upon a variety of factors including the activity of the specific compound employed, the age, body weight, general health, gender, and diet of the subject, the time of administration, the route of administration, the rate of excretion, any drug combination, and the degree of expression or activity to be modulated.

Suitable dosage ranges for the active compound can vary according to these considerations, but in general, the compounds are administered in the range of about 0.1 µg/kg-5 mg/kg of body weight; preferably the range is about 1 µg/kg-300 µg/kg of body weight; more preferably about 10 µg/kg-100 µg/kg of body weight. For a typical 70-kg human subject, thus, the dosage range is from about 0.7 µg-350 mg; preferably about 700 µg-21 mg; most preferably about 700 µg-7 mg. Dosages can be higher when the compounds are administered orally or transdermally as compared to, for example, i.v. administration. The compounds can be administered as a single bolus dose, a dose over time, as in i.v. or transdermal administration, or in multiple dosages.

The amount of active compound to be administered can vary according to the discretion of the skilled artisan. The amount of active compound to be administered to the recipient is within the ranges described herein. However, the administration of such amounts will vary according to the standards set forth by clinicians.

The dosage regimen for rejuvenation of immune system, blood cell, T-cell, or regulatory T-cell with the active compounds is based on a variety of factors, including the type of injury, the age, weight, sex, medical condition of the individual, the severity of the condition, the route of administration, and the particular compound employed. Thus, the dosage regimen can vary widely, but can be determined routinely by a physician using standard methods. Dosage levels of the order of between 0.1 ng/kg and 10 mg/kg body weight of the active compounds per body weight are useful for all methods of use disclosed herein.

The treatment regime will also vary depending on the condition being treated, based on a variety of factors, including the type of injury, the age, weight, sex, medical condition of the individual, the severity of the condition, the route of administration, and the particular compound employed.

In a preferred embodiment, the active compound is administered subcutaneously. A suitable subcutaneous dose of the active compound is preferably between about 0.1 ng/kg and about 10 mg/kg administered twice daily for a time sufficient to increase rejuvenation of immune system, blood cell, T-cell, or regulatory T-cell. This dosage regimen maximizes the therapeutic benefits of the treatments while minimizing the amount of agent needed. Such an application minimizes costs as well as possible deleterious side effects.

For subcutaneous administration, the active ingredient can comprise from 0.0001% to 10% w/w, e.g. from 1% to 2% by weight of the formulation, although it can comprise as much as 10% w/w, but preferably not more than 5% w/w, and more preferably from 0.1% to 1% of the formulation. In a most preferred embodiment, subcutaneous administration of between about 1 to 1000 µg/kg/day of the active compounds is initiated at between one week before to one week after administration of a cancer therapy (e.g., a chemotherapeutic agent).

In all of these embodiments, the compounds can be administered prior to, simultaneously with, or subsequent to any other therapeutic exposure.

The active compounds can be administered by any suitable route, including orally, parentally, by inhalation spray, rectally, or topically in dosage unit formulations containing conventional pharmaceutically acceptable carriers, adjuvants, and vehicles. The term parenteral as used herein includes, subcutaneous, intravenous, intraarterial, intramuscular, intrasternal, intratendinous, intraspinal, intracranial, intrathoracic, infusion techniques or intraperitoneally. In some embodiments, the active compounds are administered as a depot comprising a bio-compatible matrix formulated for continuous delivery of the agent in vivo. In some embodiments, the depot is formulated to degrade over time, thereby releasing the agent in a continuous or near-continuous manner. In some embodiments, the depot is formulated for release of the agent over the range of about 1 day to about 1, 2, 3, 4, 5, 6 months or more. In some embodiments, the depot can be an injectable depot for local administration. In some embodiments, the injectable depot is formulated for subcutaneous, intravenous, intraarterial, intramuscular, intrasternal, intratendinous, intraspinal, intracranial, intrathoracic, infusion techniques or intraperitoneallysubcutaneous, intravenous, intraarterial, intramuscular, intrasternal, intratendinous, intraspinal, intracranial, intrathoracic, infusion techniques or intraperitoneal injection. In some embodiments, the injectable depot is formulated for local injection at or near the stroma of the intestinal tract.

The active compounds can be made up in a solid form (including granules, powders or suppositories) or in a liquid form (e.g., solutions, suspensions, or emulsions). The compounds can be applied in a variety of solutions. Suitable solutions for use in accordance with the preferred embodiments are sterile, dissolve sufficient amounts of the peptide, and are not harmful for the proposed application. In this regard, the compounds disclosed herein are very stable but are hydrolyzed by strong acids and bases. The compounds are soluble in organic solvents and in aqueous solutions at pH 5-8.

The active compounds can be subjected to conventional pharmaceutical operations such as sterilization and/or can contain conventional adjuvants, such as preservatives, stabilizers, wetting agents, emulsifiers, buffers etc.

For administration, the active compounds are ordinarily combined with one or more adjuvants appropriate for the indicated route of administration. The compounds can be admixed with lactose, sucrose, starch powder, cellulose esters of alkanoic acids, stearic acid, talc, magnesium stearate, magnesium oxide, sodium and calcium salts of phosphoric and sulphuric acids, acacia, gelatin, sodium alginate, polyvinylpyrrolidine, and/or polyvinyl alcohol, and tableted or encapsulated for conventional administration. Alternatively, the compounds disclosed herein can be dissolved in saline, water, polyethylene glycol, propylene glycol, carboxymethyl cellulose colloidal solutions, ethanol, corn oil, peanut oil, cottonseed oil, sesame oil, tragacanth gum, and/or various buffers. Other adjuvants and modes of administration are well known in the pharmaceutical art. The carrier or diluent can include time delay material, such as glyceryl monostearate or glyceryl distearate alone or with a wax, or other materials well known in the art.

In some embodiments, the pharmaceutical composition comprises a Cdc42-specific inhibitor in a dosage formulated in an amount that is less than or equivalent to the amount that is sufficient to reduce GTP-bound Cdc42 levels in an aged immune system, blood cell, T-cell, or regulatory T-cell cell to the about the levels of GTP-bound Cdc42 in a normal, non-aged immune system, blood cell, T-cell, or regulatory T-cell. In some embodiments, the pharmaceutical composition comprises a Cdc42-specific inhibitor in a dosage formulated in an amount that is less than the amount that is sufficient to mobilize hematopoietic stem cells and progenitor cells from bone marrow into peripheral blood.

### Additional Administration and Regimens

Some details regarding the administration of the Cdc42-specific inhibitor are provided supra. Additional information regarding administration and regimens for treatment are provided herein.

In some embodiments, only a single administration of the Cdc42-specific inhibitor is necessary for treating a subject. As discussed supra, this can be a factor determined by subject specific characteristics, such as age, health, or Cdc42 activity. Often, however, a subject may need more than one administration of the Cdc42-specific inhibitor to obtain the desired therapeutic result. Thus, in some embodiments, the administering of the Cdc42-specific inhibitor to said subject occurs once and in other embodiments, the administering of the Cdc42-specific inhibitor occurs more than once. Administering of the Cdc42-specific inhibitor can occur as often as needed for treatment, e.g. in some embodiments administering occurs 1 time, 2 times, 3 times, 4 times, 5 times, 6 times, 7 times, 8 times, 9 times, 10 times, 1-10 times, 1-9 times, 1-8 times, 1-7 times, 1-6 times, 1-5 times, 1-4 times, 1-3 times, 2-10 times, 2-9 times, 2-8 times, 2-7 times, 2-6 times, 2-5 times, 2-4 times, 2-3 times, 3-10 times, 3-9 times, 3-8 times, 3-7 times, 3-6 times, 3-5 times, 3-4 times, 4-10 times, 4-9 times, 4-8 times, 4-7 times, 4-6 times, 4-5 times, 5-10 times, 5-9 times, 5-8 times, 5-7 times, 5-6 times, 6-10 times, 6-9 times, 6-8 times, 6-7 times, 7-10 times, 7-9 times, 7-8 times, 8-10 times, 8-9 times, 9-10 times, about any of the aforementioned times of administration (e.g., about 3 times or about 1-3 times), or at least any of the aforementioned times of administration (e.g., at least 3 times, at least about 3 times, or at least about 1-3 times).

When more than one administration of a Cdc42-specific inhibitor is given to a subject, each administration may be of the same Cdc42-specific inhibitor or the administrations may be of different Cdc42-specific inhibitors. For example, a sample from the subject may be taken (e.g. blood sample or biopsy) and screened to determine the best or most active Cdc42-specific inhibitor at a given time of administration (e.g., a subject may respond better to a different Cdc42-specific inhibitor as treatment progresses). Doses of the Cdc42-specific inhibitor may be adjusted during the course of treatment, resulting in a subject receiving the same or different doses of the same or different Cdc42-specific inhibitor during the course of treating the subject. Thus, in some embodiments, principles of personalized medicine are utilized to determine the Cdc42-specific inhibitor to be administered to a subject.

Administering the Cdc42-specific inhibitor to the subject in need of treatment may occur as a regimen. In some embodiments, the administering occurs as an everyday regimen occurring on 1 day, 2 days, 3 days, 4 days, 5 days, 6 days, 7 days, 8 days, 10 days, 11 days, 12 days, 13 days, 14 days, or a range bounded by any of the aforementioned days (e.g., 1-5 days or 3-7 days), or about any of the aforementioned days (e.g., about 2 days, about 1-5 days, or about 3-7 days). In some embodiments, the administering occurs as a non-consecutive day regimen. In some embodiments, the administering occurs on non-consecutive days for 1 day, 2 days, 3 days, 4 days, 5 days, 6 days, 7 days, 8 days, 10 days, 11 days, 12 days, 13 days, 14 days, 1 week, 2 weeks, 3 weeks, 1 month, 2 months, 3 months, 6 months, 9 months, 1 year, 5 years, 10 years, for the remaining life of the subject, or a range bounded by any of the aforementioned days, weeks, or months, or about any of the aforementioned days, weeks, or months.

A subject may require more than one administration or even more than one regimen of administration as outlined above. Accordingly, in some embodiments the everyday regimen or non-consecutive day regimen is repeated every day, every week, every month, every 6 months, every 9 months, every 12 months, every 2 years, every 5 years, a range bounded by any of the aforementioned time periods (e.g., every day to every week or every month to every 12 months), or about any of the aforementioned time periods (e.g., about every day to every week or about every month to every 12 months).

In some embodiments, a subject's Cdc42 activity is determined prior to the first administration, or prior to any subsequent administration, of a Cdc42-specific inhibitor (e.g., determining activity before a first administration but not before a second administration or determining activity before a first administration and before a second administration or determining activity before a first administration and not before a second administration but determining activity before a third administration). It may be beneficial, in some embodiments, to determine the subject's Cdc42 activity prior to each administration of a Cdc42-specific inhibitor (e.g., determining activity before a first administration and before a second administration or determining activity before a first administration and before a second administration and before a third administration). It may be beneficial for a physician to utilize the information gleaned from determining the subject's Cdc42 activity to prepare a patient-specific regimen or afford patient-specific treatment. Thus, in some embodiments, the regimen or administration of the Cdc42-specific inhibitor is determined, or administration is repeated, based upon the Cde-42 activity in the subject.

A threshold for Cdc42 activity may be utilized in order to decide an appropriate regimen or administration of Cdc42-specific inhibitor(s). In some embodiments, the regimen or administering of the Cdc42-specific inhibitor is repeated when the Cdc42 activity in the subject is 1%, 2%, 3%, 4%, 5%, 6%, 7%, 8%, 9%, 10%, 11%, 12%, 13%, 14%, 15 %, 16%, 17%, 18%, 19%, 20%, 21%, 22%, 23%, 24%, 25%, 26%, 27%, 28%, 29%, 30%, 35%, 40%, 45%, 50%, 55%, 60%, 65%, 70%, 75%, 80%, 85%, 90%, 95%, 100%, 105%, 110%, 115%, 120%, 125%, 130%, 135%, 140%, 145%, 150%, 160%, 170%, 180%, 190% or 200%, about any of the aforementioned percentages, or a range bounded by any of the aforementioned percentages (e.g., about 1%-30%, about 5%-25%, about 5%-20%, about 5%-15% or 1%-30%, 5%-25%, 5%-20%, 5%-15%), 1%-100%, 1%-90%, 1%-80%, 1%-70%, 1%-60%, 1%-50%, 1%-40%, 1%-30%, 1%-20%, 1%-10%, 10%-100%, 10%-90%, 10%-80%, 10%-70%, 10%-70%, 10%-60%, 10%-50%, 10%-40%, 10%-30%, 10%-20%, 20%-100%, 20%-90%, 20%-80%, 20%-70%, 20%-60%, 20%-50%, 20%-40%, 20%-30%, 30%-100%, 30%-90%, 30%-80%, 30%-70%, 30%-60%, 30%-50%, 30%-40%, 40%-100%, 40%-90%, 40%-80%, 40%-70%, 40%-60%, 40%-50%, 50%-100%, 50%-90%, 50%-80%, 50%-70%, 50%-60%, 60%-100%, 60%-90%, 60%-80%, 60%-70%, 70%-100%, 70%-90%, 70%-80%, 80%-100%, 80%-90%, 90%-100%, about any of the aforementioned range of percentages (e.g., about 10%-70%, about 30%-60%, or about 50%-70%), or about 25%, about 30%, about 40%, about 50%, about 55%, about 60%, about 70%, about 80%, about 90%, about 100%, about 105%, about 110%, about 115%, about 120%, or about 125% of the Cdc42 activity in the subject prior to first administering the Cdc42-specific inhibitor to said subject.

In addition to relying on a subject's Cdc42 activity to determine the regimen or administration of a Cdc42-specific inhibitor, or as an alternative to such reliance, the regimen or administration may be determined by the ratio of Cdc42-GTP to total Cdc42 levels in the subject. In some embodiments, the ratio of Cdc42-GTP to total Cdc42 levels in the subject is 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, or 3.0 prior to administering, about 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, or 3.0 prior to administering, greater than 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, or 3.0 prior to administering, or greater than about 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, or 3.0 prior to administering. In some embodiments, at least 1%, 2%, 3%, 4%, 5%, 6%, 7%, 8%, 9%, 10%, 11%, 12%, 13%, 14%, 15%, 16%, 17%, 1 8%, 19%, 20%, 21%, 22%, 23%, 24%, 25%, 26%, 27%, 28%, 29%, 30%, 35%, 40%, 45%, 50%, 55%, 60%, 65%, 70%, 75%, 80%, 85%, 90%; at least about 1%, 2%, 3%, 4%, 5%, 6%, 7%, 8%, 9%, 10%, 11%, 12%, 13%, 14%, 1 5%, 16%, 17%, 18%, 19%, 20%, 21%, 22%, 23%, 24%, 25%, 26%, 27%, 28%, 29%, 30%, 35%, 40%, 45%, 50%, 55%, 60%, 65%, 70%, 75%, 80%, 85%, 90%; at least 10%-90%, 10%-80%, 10%-70%, 10%-70%, 10%-60%, 10%-50%, 10%-40%, 10%-30%, 10%-20%, 20%-100%, 20%-90%, 20%-80%, 20%-70%, 20%-60%, 20%-50%, 20%-40%, 20%-30%, 30%-100%, 30%-90%, 30%-80%, 30%-70%, 30%-60%, 30%-50%, 30%-40%, 40%-100%, 40%-90%, 40%-80%, 40%-70%, 40%-60%, 40%-50%, 50%-100%, 50%-90%, 50%-80%, 50%-70%, 50%-60%, 60%-100%, 60%-90%, 60%-80%, 60%-70%, 70%-100%, 70%-90%, 70%-80%, 80%-100%, 80%--90%; or at least 10%, 20%, 30%, 40%, 50%, 60%, 70%, 80%, 90% of blood cells, T-cells, or regulatory T-cells in the subject comprise the aforementioned ratio(s) of Cdc42-GTP to total Cdc42 levels prior to administration of a Cdc42-specific inhibitor. In some embodiments, the ratio of Cdc42-GTP to total Cdc42 levels in a subject's blood cells, T-cells, or regulatory T-cells is reduced after administration of a Cdc42-specific inhibitor. In some embodiments, the ratio of Cdc42-GTP to total Cdc42 levels in said blood cells, T-cells, or regulatory T-cells is less than 1.0, 1.1, 1.2, 1.3, 1.4, or 1.5 after said administering or less than about 1.0, 1.1, 1.2, 1.3, 1.4, or 1.5 after administration of a Cdc42-specific inhibitor. In some embodiments, the ratio of Cdc42-GTP to total Cdc42 levels in the blood cells, T-cells, or regulatory T-cells is at least 0.8, 0.9, 1.0, 1.1, 1.2 or greater or at least about 0.8, 0.9, 1.0, 1.1, 1.2 or greater after administration of a Cdc42-specific inhibitor.

Some subjects may benefit from repeated administration of a Cdc42-specific inhibitor for the remainder of the subject's life in order to maintain the above mentioned levels and ratios of CDC42 activity. Other subjects may benefit from repeated administration of a Cdc42-specific inhibitor during the course of a medical treatment plan in order to maintain the above-mentioned levels and ratios of CDC42 activity. However, some subjects may not require continuing exposure to a Cdc42-specific inhibitor in order to maintain the above-mentioned levels and ratios of CDC42 activity. Presented herein is the discovery that treatment of a subject with a Cdc42-specific inhibitor can be performed transiently and the rejuvenating effect of the treatment can be maintained long after the treatment with the Cdc42-specific inhibitor. Accordingly, provided herein are methods of treating a subject, further comprising discontinuing exposure of the subject to the Cdc42-specific inhibitor, wherein the Cdc42-specific inhibitor-mediated change in the subject is maintained after discontinuing exposure. In some embodiments, the inhibitor-mediated change is maintained for 1, 2, 3, 4, 5, 6, 7, 8, 9, 10, 11, 12, 13, 14, 15, 16, 17, 18, 19, 20, 21 days or longer, 3, 4, 5, 6, 7, 8, 9, 10, 11, 12, 13, 14, 15, 16 weeks or longer, 2, 3, 4, 5, 6, 7, 8, 9, 10, 11, 12, 13, 14, 15, 16, 17, 18, 19, 20, 21, 22, 23, 24 months or longer re, or 2, 3, 4, 5, 6, 7, 8, 9, 10, 11, 12 years or longer after discontinuing exposure to the Cdc42-specific inhibitor.

Presented herein is also the surprising discovery that the administering of the Cdc42-specific inhibitor promotes immune tolerance in said subject. Thus, the subject is conferred an increased tolerance to developing a neoplastic disease, or in situations where the subject receives a Cdc42-specific inhibitor as part of treatment for a neoplastic disease, the subject is conferred an increased tolerance to developing a subsequent neoplastic disease. The increased tolerance in the previously described embodiments is relative to a similarly situated subject that does not receive a Cdc42-specific inhibitor. In some embodiments, the increased tolerance is 1%, 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, 35%, 40%, 45%, 50%, 55%, 60%, 65%, 70%, 75%, 80%, 85%, 90%, 95%, 100%, 150%, 200%, 250%, 300%, 350%, 400%, 450%, 500%, about any of the aforementioned percentages, or a range bounded by any of the aforementioned percentages (e.g., about 1%-30%, about 5%-25%, about 5%-20%, about 5%-15% or 1%-30%, 5%-25%, 5%-20%, 5%-15%), 1%-100%, 1%-90%, 1%-80%, 1%-70%, 1%-60%, 1%-50%., 1%-40%, 1%-30%, 1%-20%, 1%-10%, 10%-100%, 10%-90%, 10%-80%, 10%-70%, 10%-70%, 10%-60%, 10%-50%, 10%-40%, 10%-30%, 10%-20%, 20%-100%, 20%-90%, 20%-80%, 20%-70%, 20%-60%, 20%-50%, 20%-40%, 20%-30%, 30%-100%, 30%-90%, 30%-80%, 30%-70%, 30%-60%, 30%-50%, 30%-40%, 40%-100%, 40%-90%, 40%-80%, 40%-70%, 40%-60%, 40%-50%, 50%-100%, 50%-90%, 50%-80%, 50%-70%, 50%-60%, 60%-100%, 60%-90%, 60%-80%, 60%-70%, 70%-100%, 70%-90%, 70%-80%, 80%-100%, 80%-90%, 90%-100%, about any of the aforementioned range of percentages (e.g., about 10%-70%, about 30%-60%, or about 50%-70%), at least any of the aforementioned percentages or ranges of percentages (e.g., at least 10%, at least 20%, at least 30%, at least 40%, at least 50%, at least 60%, at least 70%, at least 80%, at least 90%, at least 100%, at least 150%, at least 200%, at least 250%, at least 300%, at least 350%, at least 400%, at least 450%, at least 500%, at least 50%-100%, at least 50%-300%), or at least about any of the aforementioned percentages or ranges of percentages (e.g., at least about 10%, at least about 20%, at least about 30%, at least about 40%, at least about 50%, at least about 60%, at least about 70%, at least about 80%, at least about 90%, at least about 100%, at least about 150%, at least about 200%, at least about 250%, at least about 300%, at least about 350%, at least about 400%, at least about 450%, at least about 500%, at least about 50%-100%, at least about 50%-300%) over the tolerance a subject would have conferred in the absence of the Cdc42-specific inhibitor.

Accordingly, in some embodiments, the subject is administered a Cdc42-specific inhibitor for the remainder of the subject's life in order to maintain the above-mentioned levels and ratios of CDC42 activity. In other embodiments, the subject is administered a Cdc42-specific inhibitor during the course of a medical treatment plan in order to maintain the above-mentioned levels and ratios of CDC42 activity. In still other embodiments, a subject is discontinued exposure to a Cdc42-specific inhibitor at the end of a course of medical treatment. In some embodiments, a subject is discontinued exposure to a Cdc42-specific inhibitor and the Cdc42-specific inhibitor-mediated change in said subject is maintained after discontinuing exposure.

### Kits

In a further aspect, kits are provided for enhancing the immune system, blood cells, T-cells, or regulatory T-cells, wherein the kits comprise an effective amount of the active compounds for enhancing the immune system, blood cells, T-cells, or regulatory T-cells, and instructions for using the amount effective of active compound as a therapeutic. In a preferred embodiment, the kit further comprises a pharmaceutically acceptable carrier, such as those adjuvants described above. In another preferred embodiment, the kit further comprises a means for delivery of the active compound to a subject. Such devices include, but are not limited to syringes, matrical or micellar solutions, bandages, wound dressings, aerosol sprays, lipid foams, transdermal patches, topical administrative agents, polyethylene glycol polymers, carboxymethyl cellulose preparations, crystalloid preparations (e.g., saline, Ringer's lactate solution, phosphate-buffered saline, etc.), viscoelastics, polyethylene glycols, and polypropylene glycols. The means for delivery can either contain the effective amount of the active compounds, or can be separate from the compounds, that are then applied to the means for delivery at the time of use.

Information regarding procedural or other details supplementary to those set forth herein, are described in cited references specifically incorporated herein by reference.

The various methods and techniques described above provide a number of ways to carry out the invention. Of course, it is to be understood that not necessarily all objectives or advantages described can be achieved in accordance with any particular embodiment described herein. Thus, for example, those skilled in the art will recognize that the methods may be performed in a manner that achieves or optimizes one advantage or group of advantages as taught herein without necessarily achieving other objectives or advantages as may be taught or suggested herein.

Furthermore, the skilled artisan will recognize the interchangeability of various features from different embodiments. Similarly, the various features and steps discussed above, as well as other known equivalents for each such feature or step, can be mixed and matched by one of ordinary skill in this art to perform methods in accordance with principles described herein.

Although the invention has been disclosed in the context of certain embodiments and examples, it will be understood by those skilled in the art that the invention extends beyond the specifically disclosed embodiments to other alternative embodiments and/or uses and obvious modifications and equivalents thereof. Accordingly, the invention is not intended to be limited by the specific disclosures of preferred embodiments herein, but instead by reference to claims attached hereto.

The following examples provide illustrations of some of the embodiments described herein but are not intended to limit the invention.

### General Experimental Methods

The Cdc42 conditional knockout mice and CASIN were developed in CCHMC by Dr. Yi Zheng lab) (Yang L, Wang L, Zheng Y. Gene targeting of Cdc42 and Cdc42GAP affirms the critical involvement of Cdc42 in filopodia induction, directed migration, and proliferation in primary mouse embryonic fibroblasts. Mol Biol Cell. 2006 Nov;17(11):4675-85) (Liu W, Du W, Shang X, Wang L, Evelyn C, Florian MC, A Ryan M, Rayes A, Zhao X, Setchell K, Meller J, Guo F, Nassar N, Geiger H, Pang Q, Zheng Y. Rational identification of a Cdc42 inhibitor presents a new regimen for long-term hematopoietic stem cell mobilization. Leukemia. 2019 Mar;33(3):749-761). In the examples below, the effects of Cdc42 deletion and CASIN on T-cell immunity against tumor growth of colorectal cancer cells and/or pancreatic cancer cells was evaluated. A syngeneic mouse model of tumor growth by subcutaneous injection of MC38, a mouse colon cancer cell line, or KPC, a mouse pancreatic cancer cell line, was also developed. CASIN was given by intraperitoneal injection at 20 mg/kg body weight twice a day for one week and then 30 mg/kg body weight once a day until the end of the experiments. CASIN was administered either before cancer cell injection (e.g., prophylactic treatment) or upon tumor onset (e.g., therapeutic treatment). Anti-PD-1 (150 µg) was given by intraperitoneal injection once every other day. For examples involving Anti-PD-1 (150 µg), four experimental groups were set up: vehicle + isotype control antibody, CASIN + isotype control antibody, vehicle + Anti-PD-1, and CASIN + Anti-PD-1.

### EXAMPLE 1

Cdc42 GTPase is a regulator of T cell function. Gene targeting (heterozygous deletion) of Cdc42 specifically in regulatory T-cells (Tregs), a type of T-cell that promotes immune tolerance and tumor immune evasion through suppressing effector T-cells, was achieved by crossbreeding Cdc42^{loxp/loxp} (Cdc42^{fl/fl}) mice with Foxp3^{YFP-Cre} mutant mice expressing a knocked-in yellow fluorescent protein/iCre-recombinase fusion protein from the Foxp3 locus. The resultant Cdc42^{f1/+}Foxp3^{YFP-Cre} mice (Cd42^{+/-}) and control Cdc42^{+/+}Foxp3^{YFP-Cre} mice (wild type, WT) were anesthetized and splenocytes from the mice were analyzed by flow cytometry. The loss of Cdc42 had no effect on the homeostasis of Tregs (Fig. 1A) but destabilized Tregs, as evidenced by decreased Foxp3 expression, a Treg signature transcription factor, (Fig. 1B) and increased effector T-cell cytokine IL-4 (Fig. 1C) and IFN-γ (Fig. 1D) expression in Tregs. Concomitantly, Cdc42-deficient mice exhibited increased CD4⁺ effector T cells, as evidenced by increased IFN-γ and IL-4 expression in CD4⁺Foxp3⁻ cells (Fig. 1E, F). Steady state Cdc42-deficient mice did not show alterations in CD8⁺ effector T cells (Fig. 1G, H).

### EXAMPLE 2

Cdc42^{fl/+}Foxp3^{YFP-Cre} and Cdc42^{+/+}Foxp3^{YFP-Cre} mice were inoculated (s.c.) with MC38 mouse colon cancer cells (8 x 10⁵) or KPC mouse pancreatic cancer cells (8 x 10⁵). Tumor volume was monitored. The mice were anesthetized and tumors were dissected and subjected to flow cytometry analysis. Treg-specific heterozygous deletion of Cdc42 inhibited tumor growth of colon cancer cells (Fig. 2A). In line with this, tumor infiltrating Tregs in Cdc42-deficient mice were unstable, as reflected by increased Tregs expressing effector T-cell cytokine IFN-γ (Fig. 2B), and tumor infiltrating effector T-cells in Cdc42-deficient mice were increased, as evidenced by increased IFN-γ-producing CD4⁺Foxp3⁻ cells (Fig. 2C) and CD8⁺ cells (Fig. 2D). Tumor-bearing Cdc42-deficient mice did not show alterations in IL-4⁺ Tregs and IL-4⁺CD4⁺ effector T cells (data not shown). Furthermore, in addition to suppression of tumor growth of colon cancer cells, mice bearing Treg-specific heterozygous deletion of Cdc42 demonstrated a suppression of tumor growth of pancreatic cancer cells (Fig. 2E). Mechanistically, RNA-seq found that Cdc42^{+/-} Tregs had about 400-fold increase in the expression of carbonic anhydrase I (CAI) (data not shown) that functions to modulate cellular pH by catalyzing the hydration of intracellular CO₂ to HCO₃⁻and H⁺. Quantitative real-time RT-PCR (Q-PCR) confirmed the upregulation of CAI in Cdc42^{+/-} Tregs (Fig. 3A). As a result, while extracellular (medium) pH (pHe) of wild type (WT) Treg culture was maintained at 7.4, pHe of Cdc42^{+/-} Treg culture was increased to 7.56 (Fig. 3B). WT Tregs were destabilized upon incubation with culture medium of pH 7.56 (Fig. 3C) or with conditional medium from Cdc42^{+/-} Treg culture (Fig. 3D). These data suggest that extracellular alkalization leads to the instability of Cdc42^{+/-} Tregs. Furthermore, incubation of Cdc42^{+/-} Tregs with a CA inhibitor, acetazolamide, rescued the instability of Cdc42 Tregs (Fig. 3E) and tumor growth of MC38 cells (Fig. 3F).

### EXAMPLE 3

C57BL/6 mice were injected (i.p.) with vehicle or 30 mg/kg body weight of CASIN twice a day for one week and then 40 mg/kg body weight of CASIN once a day until the end of the experiment. One day after the first CASIN treatment, the mice were injected (s.c.) with MC38 (8 x 10⁵). Tumor growth was monitored. Upon euthanasia, tumors were dissected and subjected to flow cytometry analysis. Prophylactic CASIN treatment of C57BL/6 mice mimicked Cdc42 deficiency in suppressing tumor growth (Fig. 4A), destabilizing Tregs (Fig. 4B), and increasing effector T cells (Fig. 4C, D). Thus, pharmacological targeting of Cdc42 in C57BL/6 mice with a Cdc-42-specific small molecule inhibitor, CASIN, exhibited prophylactic benefits.

### EXAMPLE 4

C57BL/6 mice were injected (s.c.) with MC38 (8 x 10⁵) at day 1 and injected (i.p.) with Anti-CD4/CD8 neutralizing antibodies (5 mg/kg body weight for each antibody) or isotype control antibody once every 4 days starting at day 1. Starting from day 10 when tumor onset was observed, the mice were treated with vehicle or 30 mg/kg body weight of CASIN twice a day for one week and then 40 mg/kg body weight of CASIN once a day until the end of the experiment. Tumor volume was monitored. At day 14, depletion of T-cells in peripheral blood from the mice treated with Anti-CD4/CD8 neutralizing antibodies was confirmed by flow cytometry analysis. Upon euthanasia, tumors were dissected from isotype control antibody-treated mice and analyzed by flow cytometry and immunohistochemistry using Anti-CD3 antibody. Therapeutic CASIN treatment inhibited tumor growth (Fig. 5A) to a surprising extent that was associated with Treg instability (Fig. 5B) and increased effector T-cells (CD3⁺ T-cells) (Fig. 5C), IFN-γ-producing CD4⁺Foxp3⁻ (Fig. 5D) and CD8⁺ cells (Fig. 5E). Depletion of T-cells by Anti-CD4/CD8 neutralizing antibodies completely restored tumor growth (Fig. 5A), suggesting that the tumor suppression by CASIN is attributed to the increased effector T-cells. Thus, pharmacological targeting of Cdc42 in C57BL/6 mice with a Cdc-42-specific small molecule inhibitor, CASIN, exhibited therapeutic benefits.

### EXAMPLE 5

C57BL/6 mice were injected (s.c.) with MC38 (8 x 10⁵) at day 1. Starting from day 8 when tumor onset was observed, the mice were treated (i.p.) with vehicle or 30 mg/kg body weight of CASIN twice a day for one week and then 40 mg/kg body weight of CASIN once a day until the end of the experiment. Anti-PD-1 (150 µg) or isotype control antibody was injected (i.p.) once every other day starting from day 8 until day 16. Tumor volume was monitored. Combined CASIN and immune checkpoint inhibitor (Anti-PD-1) caused more drastic (e.g., synergistic) suppression of tumor growth than CASIN or immune checkpoint inhibitor alone. The combined therapy further demonstrated tumor regression (Fig. 6).

## Claims

1. A Cdc42-specific inhibitor having the structure of formula (I): 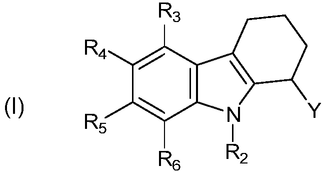 as a single enantiomer, a mixture of enantiomers, pharmaceutically acceptable salt, a solvate, or polymorph thereof, wherein:
Y is selected from the group consisting of -O**R₇**, **-NR₈R₉,** and -NN**R₈R₉**;
**R₇** is selected from the group consisting of C₁₋₆ alkyl, -(CH₂)ᵤC₃₋₇cycloalkyl, C₂₋₆ alkenyl, C₁₋₆ alkoxy, hydroxy-C₁₋₆ alkyl, phenyl, C₁₋₆ alkyl substituted with up to 5 fluoro, and C₁₋₆ alkoxy substituted with up to 5 fluoro, said C₁₋₆ alkyl, -(CH₂)ᵤC₃₋₇cycloalkyl, C₂₋₆ alkenyl, C₁₋₆ alkoxy, hydroxy-C₁₋₆ alkyl, phenyl are each optionally substituted with one or more substitutents each independently selected from the group consisting of halo, -CN, -OH, C₁₋₆ alkoxyl, heteroaryl, **R₁₉**, and -O**R₂₀**;
**R₈** and **R₉** are each separately a hydrogen or **R₂₀**; or **R₈** and **R₉** are optionallly taken together with the nitrogen to which they are attached to form indolinyl, pyrrolidinyl, piperidinyl, piperazinyl, or morpholinyl, each optionally substituted with one or more substituents each independently selected from the group consisting of halo, cyano, nitro, hydroxy, C₁₋₆ alkyl, (CH₂)ᵤC₃₋₇cycloalkyl, C₂₋₆ alkenyl, C₁₋₆ alkoxy, hydroxy-C₁₋₆ alkyl, phenyl, C₁₋₆ alkyl substituted with up to 5 fluoro, and C₁₋₆ alkoxy substituted with up to 5 fluoro; or **R₈** and **R₂** come together to be C₁₋₃ alkyl linking together as a ring;
each **R₂₀** separately selected from the group consisting of C₁₋₆ alkyl, C₃₋₇ cycloalkyl, and phenyl, said C₁₋₆ alkyl, C₃₋₇ cycloalkyl, and phenyl, each optionally substituted with one or more substituents each independently selected from the group consisting of **R₂₁** and **R₂₂,**
each **R₂₁** is separately selected from the group consisting of halo, cyano, nitro, and hydroxy,
each **R₂₂** is separately selected from the group consisting of C₁₋₆ alkyl, C₁₋₆ alkoxy -(CH₂)ᵤC₃₋₇cycloalkyl, C₂₋₆ alkenyl, hydroxy-C₁₋₆ alkyl, **R₁₉**, and -O**R₂₀**, each optionally substituted with one or more substituents each independently selected from the group consisting of halo, cyano, nitro, hydroxy, C₁₋₆ alkyl, and C₁₋₆ alkoxy;
each **u** is independently 0, 1, 2, 3, or 4;
**R₂** is a hydrogen, or selected from the group consisting of C₁₋₆ alkyl, C₃₋₇ cycloalkyl, and phenyl, said C₁₋₆ alkyl, C₃₋₇ cycloalkyl, and phenyl, each optionally substituted with one or more substituents each independently selected from the group consisting of halo, cyano, nitro, hydroxy, C₁₋₆ alkyl, -(CH₂)ᵤC₃₋₇cycloalkyl, C₂₋₆ alkenyl, C₁₋₆ alkoxy, hydroxy-C₁₋₆ alkyl, phenyl, C₁₋₆ alkyl substituted with up to 5 fluoro, C₁₋₆ alkoxy substituted with up to 5 fluoro, and -O(CH₂)ᵤphenyl optionally substituted with one or more substituents each independently selected from the group consisting of halo, cyano, nitro, hydroxy, C₁₋₆ alkyl, and C₁₋₆ alkoxy; or **R₈** and **R₂** come together to be C₁₋₃ alkyl linking together as a ring;
**R₃, R₄, R₅** and **R₆** are each independently selected from the group consisting of hydrogen, halo, cyano, nitro, hydroxy, C₁₋₆ alkyl,
(CH₂)ᵤC₃₋₇cycloalkyl, -O(CH₂)ᵤC₃₋₇cycloalkyl, C₂₋₆ alkenyl, C₁₋₆ alkoxy, hydroxy-C₁₋₆ alkyl, phenyl, C₁₋₆ alkyl substituted with up to 5 fluoro, and C₁₋₆ alkoxy substituted with up to 5 fluoro, said C₁₋₆ alkyl, (CH₂)ᵤC₃₋₇cycloalkyl, -O(CH₂)ᵤC₃₋₇cycloalkyl, C₂₋₆ alkenyl, C₁₋₆ alkoxy, hydroxy-C₁₋₆ alkyl, and phenyl, each optionally substituted with one or more **R₂₃,**
each **R₂₃** is independently selected from the group consisting of halo, cyano, nitro, hydroxy, C₁₋₆ alkyl, -(CH₂)ᵤC₃₋₇cycloalkyl, C₂₋₆ alkenyl, C₁₋₆ alkoxy, hydroxy-C₁₋₆ alkyl, phenyl, C₁₋₆ alkyl substituted with up to 5 fluoro, and C₁₋₆ alkoxy substituted with up to 5 fluoro, said phenyl optionally substituted with one or more substituents each independently selected from the group consisting of halo, cyano, nitro, hydroxy, C₁₋₆ alkyl, -(CH₂)ᵤC₃₋₇cycloalkyl, C₂₋₆ alkenyl, C₁₋₆ alkoxy, hydroxy-C₁₋₆ alkyl, phenyl, C₁₋₆ alkyl substituted with up to 5 fluoro, and C₁₋₆ alkoxy substituted with up to 5 fluoro;
each **R₁₉** is independently aryl optionally substituted with one or more substituents each independently selected from the group consisting of halo, cyano, nitro, hydroxy, C₁₋₆ alkyl optionally substituted with up to 5 fluoro, and C₁₋₆ alkoxy optionally substituted with up to 5 fluoro;
each **R₂₀** is independently hydrogen or aryl optionally substituted with one or more substituents each independently selected from the group consisting of halo, cyano, nitro, hydroxy, C₁₋₆ alkyl optionally substituted with up to 5 fluoro, and C₁₋₆ alkoxy optionally substituted with up to 5 fluoro; and
wherein when **Y** is **NR₈R₉** then **R₈** and **R₂** optionally come together to be C₁₋₃ alkyl linking together as a ring,
with the proviso when **R₈** comes together with **R₂** to be C₁₋₃ alkyl linking together as a ring then **R₄** is not substituted with hydroxyl;
for use in treating a neoplastic disease in a subject.

2. The compound for use according to claim 1, wherein one, two or three of **R₃, R₄**, **R₅** and **R₆** are not hydrogen.

3. The compound for use according to claim 1 or claim 2, wherein **R₄** is selected from the group consisting of C₁₋₆ alkyl, -(CH₂)ᵤC₃₋₇cycloalkyl, -O(CH₂)ᵤC₃₋₇cycloalkyl, C₂₋₆ alkenyl, C₁₋₆ alkoxy, phenyl, C₁₋₆ alkyl substituted with up to 5 fluoro, and C₁₋₆ alkoxy substituted with up to 5 fluoro, said C₁₋₆ alkyl, - (CH₂)ᵤC₃₋₇cycloalkyl, -O(CH₂)ᵤC₃₋₇ cycloalkyl, C₂₋₆ alkenyl, C₁₋₆ alkoxy, and phenyl, each optionally substituted with one or more substituents each independently selected from the group consisting of haloC₁₋₆ alkyl, -(CH₂)ᵤC₃₋₇cycloalkyl, C₂₋₆ alkenyl, C₁₋₆ alkoxy, hydroxy-C₁₋₆ alkyl, phenyl, C₁₋₆ alkyl substituted with up to 5 fluoro, and C₁₋₆ alkoxy substituted with up to 5 fluoro.

4. The compound for use according to any of claims 1-3,
wherein:
**Y** is -N**R₈R₉**,
**R₈** is hydrogen; and **R₉** is C₁₋₆ alkyl optionally substituted with one or more substituents each independently selected from the group consisting of hydroxy, **R₁₉** and -O**R₂₀**;
each **R₁₉** is independently phenyl optionally substituted with one or more substituents each independently selected from the group consisting of halo, cyano, C₁₋₆ alkyl optionally substituted with up to 5 fluoro, and C₁₋₆ alkoxy optionally substituted with up to 5 fluoro, or each **R₁₉** is independently phenyl optionally substituted with one or more substituents each independently selected from the group consisting of halo, C₁₋₆ alkyl, and C₁₋₆ alkoxy; and
each **R₂₀** is independently hydrogen or phenyl optionally substituted with one or more substituents each independently selected from the group consisting of halo, cyano, nitro, hydroxy, C₁₋₆ alkyl optionally substituted with up to 5 fluoro, and C₁₋₆ alkoxy optionally substituted with up to 5 fluoro, or each **R₂₀** is independently phenyl optionally substituted with one or more substituents each independently selected from the group consisting of halo, C₁₋₆ alkyl, and C₁₋₆ alkoxy.

5. The compound for use according to any of claims 1-4, wherein **R₂** and **R₈** are hydrogen, or optionally when **Y** is -N**R₈R₉** and **R₈** and **R₂** come together to be C₁₋₃ alkyl linking together as a ring.

6. The compound for use according to any of claims 1-5, wherein **R₉** is hydrogen,
or **R₉** is C₁₋₆ alkyl optionally substituted with one or more substituents each independently selected from the group consisting of hydroxy, **R₁₉** or -O**R₂₀** where each **R₁₉** is independently phenyl optionally substituted with one or more substituents each independently selected from the group consisting of halo, cyano, C₁₋₆ alkyl optionally substituted with up to 5 fluoro, and C₁₋₆ alkoxy optionally substituted with up to 5 fluoro; and where each **R₂₀** is independently hydrogen or phenyl optionally substituted with one or more substituents each independently selected from the group consisting of halo, cyano, nitro, hydroxy, C₁₋₆ alkyl optionally substituted with up to 5 fluoro, and C₁₋₆ alkoxy optionally substituted with up to 5 fluoro,
or **R₉** is hydrogen or C₁₋₆ alkyl, optionally substituted with one or more substituents each independently selected from the group consisting of hydroxyl, **R₁₉** and -O**R₂₀** where each **R₁₉** is independently phenyl optionally substituted with one or more substituents each independently selected from the group consisting of halo, cyano, C₁₋₆ alkyl optionally substituted with up to 5 fluoro, and C₁₋₆ alkoxy optionally substituted with up to 5 fluoro; and where each **R₂₀** is independently hydrogen or phenyl optionally substituted with one or more substituents each independently selected from the group consisting of halo, cyano, nitro, hydroxy, C₁₋₆ alkyl optionally substituted with up to 5 fluoro, and C₁₋₆ alkoxy optionally substituted with up to 5 fluoro.

7. The compound for use according to any of claims 1-6,
wherein **R₄** is selected from the group consisting of C₁₋₆ alkyl, -(CH₂)ᵤC₃₋₇cycloalkyl, -O(CH₂)ᵤC₃₋₇cycloalkyl, C₂₋₆ alkenyl, C₁₋₆ alkoxy, phenyl, C₁₋₆ alkyl substituted with up to 5 fluoro, and C₁₋₆ alkoxy substituted with up to 5 fluoro, said C₁₋₆ alkyl, -(CH₂)ᵤC₃₋₇cycloalkyl, -O(CH₂)ᵤC₃₋₇cycloalkyl, C₂₋₆ alkenyl, C₁₋₆ alkoxy, and phenyl, each optionally substituted with one or more **R₂₃,**
each **R₂₃** is independently selected from the group consisting of halo, C₁₋₆ alkyl, -(CH₂)ᵤC₃₋₇cycloalkyl, C₂₋₆ alkenyl, C₁₋₆ alkoxy, phenyl, C₁₋₆ alkyl substituted with up to 5 fluoro, and C₁₋₆ alkoxy substituted with up to 5 fluoro, said phenyl optionally substituted with one or more substituents each independently selected from the group consisting of halo, C₁₋₆ alkyl, -(CH₂)ᵤC₃₋₇cycloalkyl, C₂₋₆ alkenyl, C₁₋₆ alkoxy, C₁₋₆ alkyl substituted with up to 5 fluoro, and C₁₋₆ alkoxy substituted with up to 5 fluoro,
or wherein **R₄** is selected from the group consisting of C₁₋₆ alkyl, C₃₋₇cycloalkyl, -OC₃₋₇cycloalkyl, phenyl, C₁₋₆ alkyl substituted with up to 5 fluoro, and C₁₋₆ alkoxy substituted with up to 5 fluoro, said phenyl optionally substituted with one or more substituents each independently selected from the group consisting of halo, C₁₋₆ alkyl, C₁₋₆ alkoxy, C₁₋₆ alkyl substituted with up to 5 fluoro, and C₁₋₆ alkoxy substituted with up to 5 fluoro.

8. The compound for use according to any of claims 1-7, wherein Y is -N**R₈R₉** and **R₈** and **R₂** come together to be C₁₋₃ alkyl linking together as a ring.

9. The compound for use according to any of claims 1-8, wherein **R₂** is a hydrogen or selected from the group consisting of C₁₋₆ alkyl, C₃₋₇ cycloalkyl, and phenyl, said C₁₋₆ alkyl optionally substituted with one or more halo.

10. The compound for use according to any of claims 1-9, wherein **R₉** is hydrogen, or C₁₋₆ alkyl, optionally substituted with one or more substituents each independently selected from the group consisting of hydroxyl, **R₁₉** and -O**R₂₀**;
each **R₁₉** is independently phenyl optionally substituted with one or more substituents each independently selected from the group consisting of halo, cyano, C₁₋₆ alkyl optionally substituted with up to 5 fluoro, and C₁₋₆ alkoxy optionally substituted with up to 5 fluoro; and
each **R₂₀** is independently hydrogen or phenyl optionally substituted with one or more substituents each independently selected from the group consisting of halo, cyano, nitro, hydroxy, C₁₋₆ alkyl optionally substituted with up to 5 fluoro, and C₁₋₆ alkoxy optionally substituted with up to 5 fluoro.

11. The compound for use according to any of claims 1-10, wherein compound of formula (I) is selected from the group consisting of: 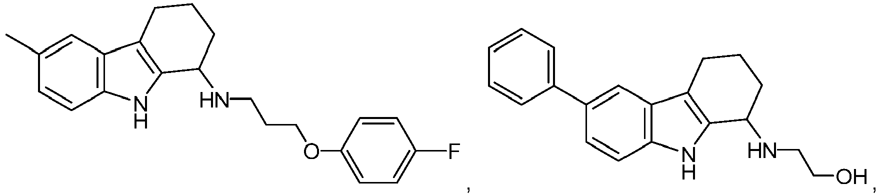 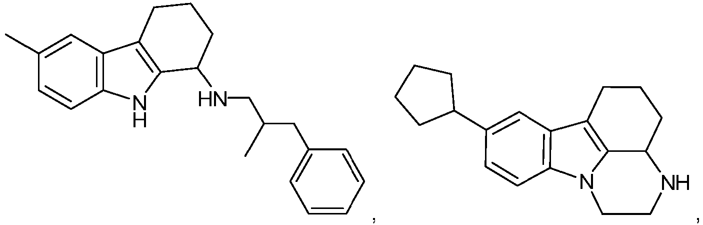 and

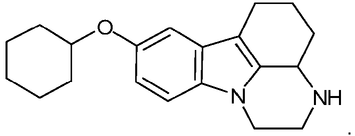

12. The compound for use according to claims 1 to 11, wherein the Cdc42-specific inhibitor is CASIN.

13. The compound for use according to any of claims 1-12, wherein the neoplastic disease is selected from the group consisting of colon cancer and pancreatic cancer.

14. The compound for use according to any of claims 1-12, wherein the use further comprises administering one or more of an anticancer agent, an antineoplastic agent, or an apoptosis modulating agent that is an immune checkpoint inhibitor that targets PD-1, PD-L1, or CTLA-4, a chemotherapeutic compound, a radiation therapy, or a surgical intervention.

15. The compound for use according to any of claims 1-12, wherein the immune checkpoint inhibitor that targets PD-1, PD-L1, or CTLA-4 is an antibody that is selected from the group consisting of pembrolizumab, nivolumad, cemiplimab, atezolizumab, avelumab, durvalumab, and ipilimumab.

## Patentansprüche

1. Cdc42-spezifischer Inhibitor mit der Struktur von Formel (I): 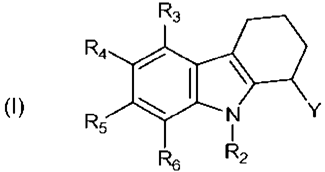 als einzelnes Enantiomer, ein Gemisch von Enantiomeren, ein pharmazeutisch verträgliches Salz, ein Solvat oder ein Polymorph davon, wobei:
Y ausgewählt ist aus der Gruppe, bestehend aus **-OR₇, -NR₈R₉,** und -NN**R₈R₉**;
**R₇** ausgewählt ist aus der Gruppe bestehend aus C₁₋₆-Alkyl, -(CH₂)ᵤC₃₋₇-Cycloalkyl, C₂₋₆-Alkenyl, C₁₋₆-Alkoxy, Hydroxy-C₁₋₆-alkyl, Phenyl, C₁₋₆-Alkyl, substituiert mit bis zu 5 Fluoratomen und C₁₋₆-Alkoxy, substituiert mit bis zu 5 Fluoratomen, wobei C₁₋₆-Alkyl, -(CH₂)ᵤC₃₋₇-Cycloalkyl, C₂₋₆-Alkenyl, C₁₋₆-Alkoxy, Hydroxy-C₁₋₆-alkyl, Phenyl jeweils optional mit einem oder mehreren Substituenten substituiert sind, die jeweils unabhängig voneinander ausgewählt sind aus der Gruppe bestehend aus Halogen, -CN, -OH, C₁₋₆-Alkoxyl, Heteroaryl, **R₁₉**, und -O**R₂₀**;
**R₈** und **R₉** jeweils getrennt ein Wasserstoff oder **R₂₀** sind; oder **R₈** und **R₉** optional zusammengenommen werden mit dem Stickstoff, an den sie angehängt sind, um Indolinyl, Pyrrolidinyl, Piperidinyl, Piperazinyl oder Morpholinyl zu bilden, die jeweils optional mit einem oder mehreren Substituenten substituiert sind, die jeweils unabhängig voneinander ausgewählt sind aus der Gruppe bestehend aus Halogen, Cyano, Nitro, Hydroxy, C₁₋₆-Alkyl, (CH₂)ᵤC₃₋₇-Cycloalkyl, C₂₋₆-Alkenyl, C₁₋₆-Alkoxy, Hydroxy-C₁₋₆-alkyl, Phenyl, C₁₋₆-Alkyl, substituiert mit bis zu 5 Fluoratomen und C₁₋₆-Alkoxy, substituiert mit bis zu 5 Fluoratomen; oder **R₈** und **R₂** sich zusammentun, um C₁₋₃-Alkyl zu sein, das sie zu einem Ring verknüpft;
jedes **R₂₀** separat ausgewählt ist aus der Gruppe bestehend aus C₁₋₆-Alkyl, C₃₋₇-Cycloalkyl und Phenyl, wobei das C₁₋₆-Alkyl, C₃₋₇-Cycloalkyl und Phenyl jeweils optional substituiert sind mit einem oder mehreren Substituenten, die jeweils unabhängig voneinander ausgewählt sind aus der Gruppe bestehend aus **R₂₁** und **R₂₂**,
jedes **R₂₁** separat ausgewählt ist aus der Gruppe bestehend aus Halogen, Cyano, Nitro und Hydroxy,
jedes **R₂₂** separat ausgewählt ist aus der Gruppe bestehend aus C₁₋₆-Alkyl, C₁₋₆-Alkoxy -(CH₂)ᵤC₃₋₇-Cycloalkyl, C₂₋₆-Alkenyl, Hydroxy-C₁₋₆-alkyl, **R₁₉**, und -O**R₂₀**, jeweils optional substituiert mit einem oder mehreren Substituenten, die jeweils unabhängig voneinander ausgewählt sind aus der Gruppe bestehend aus Halogen, Cyano, Nitro, Hydroxy, C₁₋₆-Alkyl und C₁₋₆-Alkoxy;
jedes u unabhängig 0, 1, 2, 3 oder 4 ist;
**R₂** ein Wasserstoff ist, oder ausgewählt ist aus der Gruppe bestehend aus C₁₋₆-Alkyl, C₃₋₇-Cycloalkyl und Phenyl, wobei das C₁₋₆-Alkyl, C₃₋₇-Cycloalkyl und Phenyl jeweils optional substituiert sind mit einem oder mehreren Substituenten, die jeweils unabhängig voneinander ausgewählt sind aus der Gruppe bestehend aus Halogen, Cyano, Nitro, Hydroxy, C₁₋₆-Alkyl, -(CH₂)ᵤC₃₋₇-Cycloalkyl, C₂₋₆-Alkenyl, C₁₋₆-Alkoxy, Hydroxy-C₁₋₆-alkyl, Phenyl, C₁₋₆-Alkyl, substituiert mit bis zu 5 Fluoratomen, C₁₋₆-Alkoxy, substituiert mit bis zu 5 Fluoratomen und -O(CH₂)ᵤ-Phenyl, optional substituiert mit einem oder mehreren Substituenten, die jeweils unabhängig voneinander ausgewählt sind aus der Gruppe bestehend aus Halogen, Cyano, Nitro, Hydroxy, C₁₋₆-Alkyl und C₁₋₆-Alkoxy; oder **R₈** und **R₂** sich zusammentun, um C₁₋₃-Alkyl zu sein, das sie zu einem Ring verknüpft;
**R₃, R₄**, **R₅** und **R₆** jeweils unabhängig voneinander ausgewählt sind aus der Gruppe bestehend aus Wasserstoff, Halogen, Cyano, Nitro, Hydroxy, C₁₋₆-Alkyl, (CH₂)ᵤC₃₋₇-Cycloalkyl, -O(CH₂)ᵤC₃₋₇-Cycloalkyl, C₂₋₆-Alkenyl, C₁₋₆-Alkoxy, Hydroxy-C₁₋₆-alkyl, Phenyl, C₁₋₆-Alkyl, substituiert mit bis zu 5 Fluoratomen und C₁₋₆-Alkoxy, substituiert mit bis zu 5 Fluoratomen, wobei das C₁₋₆-Alkyl, (CH₂)ᵤC₃₋₇-Cycloalkyl, -O(CH₂)ᵤC₃₋₇-Cycloalkyl, C₂₋₆-Alkenyl, C₁₋₆-Alkoxy, Hydroxy-C₁₋₆-alkyl und Phenyl jeweils optional substituiert sind mit einem oder mehreren **R₂₃,**
jedes **R₂₃** unabhängig ausgewählt ist aus der Gruppe bestehend aus Halogen, Cyano, Nitro, Hydroxy, C₁₋₆-Alkyl, -(CH₂)ᵤC₃₋₇-Cycloalkyl, C₂₋₆-Alkenyl, C₁₋₆-Alkoxy, Hydroxy-C₁₋₆-alkyl, Phenyl, C₁₋₆-Alkyl, substituiert mit bis zu 5 Fluoratomen und C₁₋₆-Alkoxy, substituiert mit bis zu 5 Fluoratomen, wobei das Phenyl optional mit einem oder mehreren Substituenten substituiert ist, die jeweils unabhängig voneinander ausgewählt sind aus der Gruppe bestehend aus Halogen, Cyano, Nitro, Hydroxy, C₁₋₆-Alkyl, -(CH₂)ᵤC₃₋₇-Cycloalkyl, C₂₋₆-Alkenyl, C₁₋₆-Alkoxy, Hydroxy-C₁₋₆-alkyl, Phenyl, C ₁₋₆-Alkyl, substituiert mit bis zu 5 Fluoratomen und C₁₋₆-Alkoxy, substituiert mit bis zu 5 Fluoratomen;
jedes **R₁₉** unabhängig ausgewählt ist aus Aryl, optional substituiert mit einem oder mehreren Substituenten, die jeweils unabhängig ausgewählt sind aus der Gruppe bestehend aus Halogen, Cyano, Nitro, Hydroxy, C₁₋₆-Alkyl, optional substituiert mit bis zu 5 Fluoratomen, und C₁₋₆-Alkoxy, optional substituiert mit bis zu 5 Fluoratomen;
jedes **R₂₀** unabhängig Wasserstoff oder Aryl ist, optional substituiert mit einem oder mehreren Substituenten, die jeweils unabhängig ausgewählt sind aus der Gruppe bestehend aus Halogen, Cyano, Nitro, Hydroxy, C₁₋₆-Alkyl, optional substituiert mit bis zu 5 Fluoratomen, und C₁₋₆-Alkoxy, optional substituiert mit bis zu 5 Fluoratomen; und
wobei, wenn **Y** N**R₈R₉** ist, **R₈** und **R₂** optional zusammenkommen, um C₁₋₃-Alkyl zu sein, das sie zu einem Ring verknüpft,
mit der Maßgabe, dass wenn **R₈** mit **R₂** zusammenkommt, um C₁₋₃-Alkyl zu sein, das sie zu einem Ring verknüpft, dann **R₄** nicht mit Hydroxyl substituiert wird;
zur Verwendung beim Behandeln einer neoplastischen Erkrankung bei einem Subjekt.

2. Verbindung zur Verwendung nach Anspruch 1, wobei ein, zwei oder drei von **R₃, R₄**, **R₅** und **R₆** kein Wasserstoff sind.

3. Verbindung zur Verwendung nach Anspruch 1 oder Anspruch 2, wobei **R₄** ausgewählt ist aus der Gruppe bestehend aus C₁₋₆-Alkyl, -(CH₂)ᵤC₃₋₇-Cycloalkyl, -O(CH₂)ᵤC₃₋₇-Cycloalkyl, C₂₋₆-Alkenyl, C₁₋₆-Alkoxy, Phenyl, C₁₋₆-Alkyl, substituiert mit bis zu 5 Fluoratomen, und C₁₋₆-Alkoxy, substituiert mit bis zu 5 Fluoratomen, wobei das C₁₋₆-Alkyl, -(CH₂)ᵤC₃₋₇-Cycloalkyl, -O(CH₂)ᵤC₃₋₇-Cycloalkyl, C₂₋₆-Alkenyl, C₁₋₆-Alkoxy, und Phenyl jeweils optional substituiert sind mit einem oder mehreren Substituenten, die jeweils unabhängig ausgewählt sind aus der Gruppe bestehend aus Halogen, C₁₋₆-Alkyl, -(CH₂)ᵤC₃₋₇-Cycloalkyl, C₂₋₆-Alkenyl, C₁₋₆-Alkoxy, Hydroxy-C₁₋₆-alkyl, Phenyl, C₁₋₆-Alkyl, substituiert mit bis zu 5 Fluoratomen, und C₁₋₆-Alkoxy, substituiert mit bis zu 5 Fluoratomen.

4. Verbindung zur Verwendung nach einem der Ansprüche 1 bis 3,
wobei:
**Y** -N**R₈R₉** ist,
**R₈** Wasserstoff ist; und **R₉** C₁₋₆-Alkyl ist, optional substituiert mit einem oder mehreren Substituenten, die jeweils unabhängig voneinander ausgewählt sind aus der Gruppe bestehend aus Hydroxy, **R₁₉** und -O**R₂₀**;
jedes **R₁₉** unabhängig Phenyl ist, optional substituiert mit einem oder mehreren Substituenten, die jeweils unabhängig ausgewählt sind aus der Gruppe bestehend aus Halogen, Cyano, C₁₋₆-Alkyl, optional substituiert mit bis zu 5 Fluoratomen, und C₁₋₆-Alkoxy, optional substituiert mit bis zu 5 Fluoratomen, oder jedes **R₁₉** unabhängig Phenyl ist, optional substituiert mit einem oder mehreren Substituenten, die jeweils unabhängig ausgewählt sind aus der Gruppe bestehend aus Halogen, C₁₋₆-Alkyl und C₁₋₆-Alkoxy; und
jedes **R₂₀** unabhängig Wasserstoff oder Phenyl ist, optional substituiert mit einem oder mehreren Substituenten, die jeweils unabhängig ausgewählt sind aus der Gruppe bestehend aus Halogen, Cyano, Nitro, Hydroxy, C₁₋₆-Alkyl, optional substituiert mit bis zu 5 Fluoratomen, und C₁₋₆-Alkoxy, optional substituiert mit bis zu 5 Fluoratomen, oder jedes **R₂₀** unabhängig Phenyl ist, optional substituiert mit einem oder mehreren Substituenten, die jeweils unabhängig ausgewählt sind aus der Gruppe bestehend aus Halogen, C₁₋₆-Alkyl und C₁₋₆-Alkoxy.

5. Verbindung zur Verwendung nach einem der Ansprüche 1 bis 4, wobei **R₂** und **R₈** Wasserstoff sind, oder optional, wenn **Y -NR₈R₉** ist, **R₈** und **R₂** zusammenkommen, um C₁₋₃-Alkyl zu sein, das sie als einen Ring verknüpft.

6. Verbindung zur Verwendung nach einem der Ansprüche 1 bis 5, wobei **R₉** Wasserstoff ist,
oder **R₉** C₁₋₆-Alkyl ist, optional substituiert mit einem oder mehreren Substituenten, die jeweils unabhängig voneinander ausgewählt sind aus der Gruppe bestehend aus Hydroxy, **R₁₉** oder -O**R₂₀**, wobei jedes **R₁₉** unabhängig Phenyl ist, optional substituiert mit einem oder mehreren Substituenten, die jeweils unabhängig ausgewählt sind aus der Gruppe bestehend aus Halogen, Cyano, C₁₋₆-Alkyl, optional substituiert mit bis zu 5 Fluoratomen, und C₁₋₆-Alkoxy, optional substituiert mit bis zu 5 Fluoratomen; und wobei jedes **R₂₀** unabhängig Wasserstoff oder Phenyl ist, optional substituiert mit einem oder mehreren Substituenten, die jeweils unabhängig ausgewählt sind aus der Gruppe bestehend aus Halogen, Cyano, Nitro, Hydroxy, C₁₋₆-Alkyl, optional substituiert mit bis zu 5 Fluoratomen, und C₁₋₆-Alkoxy, optional substituiert mit bis zu 5 Fluoratomen,
oder **R₉** Wasserstoff oder C₁₋₆-Alkyl ist, optional substituiert mit einem oder mehreren Substituenten, die jeweils unabhängig voneinander ausgewählt sind aus der Gruppe bestehend aus Hydroxyl, **R₁₉** und -O**R₂₀**, wobei jedes **R₁₉** unabhängig Phenyl ist, optional substituiert mit einem oder mehreren Substituenten, die jeweils unabhängig ausgewählt sind aus der Gruppe bestehend aus Halogen, Cyano, C₁₋₆-Alkyl, optional substituiert mit bis zu 5 Fluoratomen, und C₁₋₆-Alkoxy, optional substituiert mit bis zu 5 Fluoratomen; und wobei jedes **R₂₀** unabhängig Wasserstoff oder Phenyl ist, optional substituiert mit einem oder mehreren Substituenten, die jeweils unabhängig ausgewählt sind aus der Gruppe bestehend aus Halogen, Cyano, Nitro, Hydroxy, C₁₋₆-Alkyl, optional substituiert mit bis zu 5 Fluoratomen, und C₁₋₆-Alkoxy, optional substituiert mit bis zu 5 Fluoratomen.

7. Verbindung zur Verwendung nach einem der Ansprüche 1 bis 6,
wobei **R₄** ausgewählt ist aus der Gruppe bestehend aus C₁₋₆-Alkyl, - (CH₂)ᵤC₃₋₇-Cycloalkyl, -O(CH₂)ᵤC₃₋₇-Cycloalkyl, C₂₋₆-Alkenyl, C₁₋₆-Alkoxy, Phenyl, C₁₋₆-Alkyl, substituiert mit bis zu 5 Fluoratomen, und C₁₋₆-Alkoxy, substituiert mit bis zu 5 Fluoratomen, das C₁₋₆-Alkyl, -(CH₂)ᵤC₃₋₇-Cycloalkyl, -O(CH₂)ᵤC₃₋₇-Cycloalkyl, C₂₋₆-Alkenyl, C₁₋₆-Alkoxy und Phenyl, jedes optional substituiert mit einem oder mehreren **R₂₃,**
jedes **R₂₃** unabhängig ausgewählt ist aus der Gruppe bestehend aus Halogen, C₁₋₆-Alkyl, -(CH₂)ᵤC₃₋₇-Cycloalkyl, C₂₋₆-Alkenyl, C₁₋₆-Alkoxy, Phenyl, C₁₋₆-Alkyl, substituiert mit bis zu 5 Fluoratomen, und C₁₋₆-Alkoxy, substituiert mit bis zu 5 Fluoratomen, wobei das Phenyl optional substituiert ist mit einem oder mehreren Substituenten, die jeweils unabhängig ausgewählt sind aus der Gruppe bestehend aus Halogen, C₁₋₆-Alkyl, -(CH₂)ᵤC₃₋₇-Cycloalkyl, C₂₋₆-Alkenyl, C₁₋₆-Alkoxy, C₁₋₆-Alkyl, substituiert mit bis zu 5 Fluoratomen, und C₁₋₆-Alkoxy, substituiert mit bis zu 5 Fluoratomen,
oder wobei **R₄** ausgewählt ist aus der Gruppe bestehend aus C₁₋₆-Alkyl, C ₃₋₇-Cycloalkyl, -OC₃₋₇-Cycloalkyl, Phenyl, C₁₋₆-Alkyl, substituiert mit bis zu 5 Fluoratomen, und C₁₋₆-Alkoxy substituiert mit bis zu 5 Fluoratomen, wobei das Phenyl optional mit einem oder mehreren Substituenten substituiert ist, die jeweils unabhängig voneinander ausgewählt sind aus der Gruppe bestehend aus Halogen, C₁₋₆-Alkyl, C₁₋₆-Alkoxy, C₁₋₆-Alkyl, substituiert mit bis zu 5 Fluoratomen, und C₁₋₆-Alkoxy, substituiert mit bis zu 5 Fluoratomen.

8. Verbindung zur Verwendung nach einem der Ansprüche 1 bis 7, wobei, Y -N**R₈R₉** ist, **R₈** und **R₂** zusammenkommen, um C₁₋₃-Alkyl zu sein, das sie zu einem Ring verknüpft.

9. Verbindung zur Verwendung nach einem der Ansprüche 1 bis 8, wobei **R₂** ein Wasserstoff ist oder ausgewählt ist aus der Gruppe bestehend aus C₁₋₆-Alkyl, C₃₋₇-Cycloalkyl und Phenyl, wobei das C₁₋₆-Alkyl optional substituiert ist mit einem oder mehreren Halogenen.

10. Verbindung zur Verwendung nach einem der Ansprüche 1 bis 9, wobei **R₉** Wasserstoff oder C₁₋₆-Alkyl ist, optional substituiert mit einem oder mehreren Substituenten, die jeweils unabhängig voneinander ausgewählt sind aus der Gruppe bestehend aus Hydroxyl, **R₁₉** und -O**R₂₀**;
jedes **R₁₉** unabhängig Phenyl ist, optional substituiert mit einem oder mehreren Substituenten, die jeweils unabhängig ausgewählt sind aus der Gruppe bestehend aus Halogen, Cyano, C₁₋₆-Alkyl, optional substituiert mit bis zu 5 Fluoratomen, und C₁₋₆-Alkoxy, optional substituiert mit bis zu 5 Fluoratomen; und
jedes **R₂₀** unabhängig Wasserstoff oder Phenyl ist, optional substituiert mit einem oder mehreren Substituenten, die jeweils unabhängig ausgewählt sind aus der Gruppe bestehend aus Halogen, Cyano, Nitro, Hydroxy, C₁₋₆-Alkyl, optional substituiert mit bis zu 5 Fluoratomen, und C₁₋₆-Alkoxy, optional substituiert mit bis zu 5 Fluoratomen.

11. Verbindung zur Verwendung nach einem der Ansprüche 1 bis 10, wobei die Verbindung der Formel (I) ausgewählt ist aus der Gruppe bestehend aus: 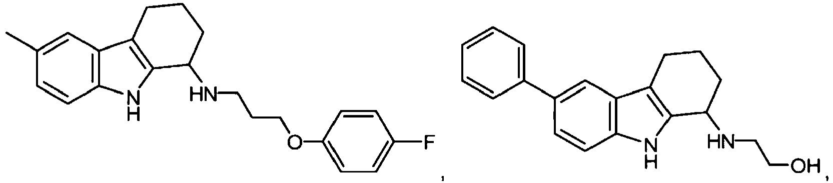 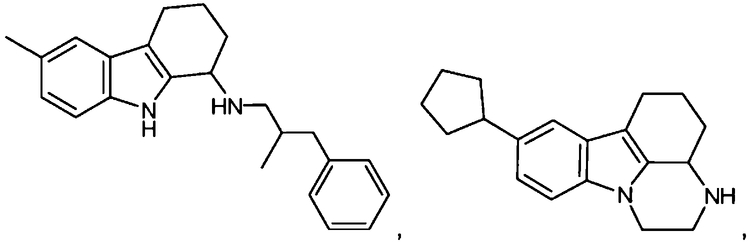 und

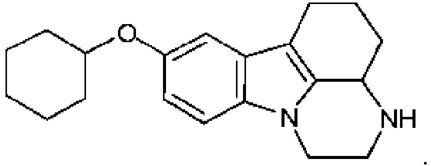

12. Verbindung zur Verwendung nach den Ansprüchen 1 bis 11, wobei der Cdc42-spezifische Inhibitor CASIN ist.

13. Verbindung zur Verwendung nach einem der Ansprüche 1 bis 12, wobei die neoplastische Erkrankung ausgewählt ist aus der Gruppe bestehend aus Dickdarmkrebs und Bauchspeicheldrüsenkrebs.

14. Verbindung zur Verwendung nach einem der Ansprüche 1 bis 12, wobei die Verwendung ferner ein Verabreichen eines oder mehrerer von einem Antikrebsmittel, einem Antineoplastikum oder einem Apoptose-modulierenden Wirkstoff umfasst, der ein Immun-Checkpoint-Inhibitor ist, der auf PD-1, PD-L1 oder CTLA-4 abzielt, einer chemotherapeutischen Verbindung, einer Strahlentherapie oder einem chirurgischen Eingriff.

15. Verbindung zur Verwendung nach einem der Ansprüche 1 bis 12, wobei der Immun-Checkpoint-Inhibitor, der auf PD-1, PD-L1 oder CTLA-4 abzielt, ein Antikörper ist, der ausgewählt ist aus der Gruppe bestehend aus Pembrolizumab, Nivolumab, Cemiplimab, Atezolizumab, Avelumab, Durvalumab und Ipilimumab.

## Revendications

1. Inhibiteur spécifique de Cdc42 ayant la structure de formule (I) : 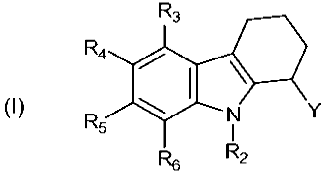 en tant qu'énantiomère unique, mélange d'énantiomères, sel pharmaceutiquement acceptable, solvate ou polymorphe de celui-ci, dans lequel :
Y est choisi dans le groupe constitué de -O**R₇**, -N**R₈R₉**, et -NN**R₈R₉** ;
**R₇** est choisi dans le groupe constitué d'alkyle en C₁₋₆, -(CH₂)ᵤ-cycloalkyle en C₃₋₇, alcényle en C₂₋₆, alcoxy en C₁₋₆, alkyl hydroxy en C₁₋₆, phényle, alkyle en C₁₋₆ substitué par jusqu'à 5 fluoro et alcoxy en C₁₋₆ substitué par jusqu'à 5 fluoro, lesdits alkyle en C₁₋₆, -(CH₂)ᵤ-cycloalkyle en C₃₋₇, alcényle en C₂₋₆, alcoxy en C₁₋₆, alkyl hydroxy en C₁₋₆, phényl sont chacun éventuellement substitués par un ou plusieurs substituants choisis chacun indépendamment dans le groupe constitué d'halo, -CN, -OH, alcoxyle en C₁₋₆, hétéroaryle, **R₁₉**, et -O**R₂₀** ;
**R₈** et **R₉** représentent chacun séparément un hydrogène ou **R₂₀** ; ou **R₈** et **R₉** sont éventuellement pris ensemble avec l'azote auquel ils sont attachés pour former indolinyle, pyrrolidinyle, pipéridinyle, pipérazinyle ou morpholinyle, chacun étant éventuellement substitué par un ou plusieurs substituants choisis chacun indépendamment dans le groupe constitué d'halo, cyano, nitro, hydroxy, alkyle en C₁₋₆, -(CH₂)ᵤ-cycloalkyle en C₃₋₇, alcényle en C₂₋₆, alcoxy en C₁₋₆, alkyl hydroxy en C₁₋₆, phényle, alkyle en C₁₋₆ substitué par jusqu'à 5 fluoro et alcoxy en C₁₋₆ substitué par jusqu'à 5 fluoro ; ou **R₈** et **R₂** s'assemblent pour former un alkyle en C₁₋₃ se liant ensemble en tant que cycle ;
chaque **R₂₀** étant choisi séparément dans le groupe constitué d'alkyle en C₁₋₆, cycloalkyle en C₃₋₇ et phényle, lesdits alkyle en C₁₋₆, cycloalkyle en C₃₋₇ et phényle, étant chacun éventuellement substitués par un ou plusieurs substituants choisis chacun indépendamment dans le groupe constitué de **R₂₁** et **R₂₂,**
chaque **R₂₁** est choisi séparément dans le groupe constitué d'halo, cyano, nitro et hydroxy,
chaque **R₂₂** est choisi séparément dans le groupe constitué d'alkyle en C₁₋₆, alcoxy en C₁₋₆, -(CH₂)ᵤ-cycloalkyle en C₃₋₇, alcényle en C₂₋₆, alkyl hydroxy en C₁₋₆, **R₁₉** et **-OR₂₀**, chacun étant éventuellement substitué par un ou plusieurs substituants choisis chacun indépendamment dans le groupe constitué d'halo, cyano, nitro, hydroxy, alkyle en C₁₋₆ et alcoxy en C₁₋₆ ;
chaque u représente indépendamment 0, 1, 2, 3 ou 4 ;
**R₂** est un hydrogène ou est choisi dans le groupe constitué d'alkyle en C₁₋₆, cycloalkyle en C₃₋₇ et phényle, lesdits alkyle en C₁₋₆, cycloalkyle en C₃₋₇ et phényle étant chacun éventuellement substitués par un ou plusieurs substituants choisis chacun indépendamment dans le groupe constitué d'halo, cyano, nitro, hydroxy, alkyle en C₁₋₆, -(CH₂)ᵤ-cycloalkyle en C₃₋₇, alcényle en C₂₋₆, alcoxy en C₁₋₆, alkyl hydroxy en C₁₋₆, phényle, alkyle en C₁₋₆ substitué par jusqu'à 5 fluoro, alcoxy en C₁₋₆ substitué par jusqu'à 5 fluoro, et -O(CH₂)ᵤ-phényle éventuellement substitué par un ou plusieurs substituants choisis chacun indépendamment dans le groupe constitué d'halo, cyano, nitro, hydroxy, alkyle en C₁₋₆ et alcoxy en C₁₋₆ ; ou **R₈** et **R₂** s'assemblent pour former un alkyle en C₁₋₃ se liant ensemble en tant que cycle ;
**R₃**, **R₄**, **R₅** et **R₆** sont chacun indépendamment choisis dans le groupe constitué d'hydrogène, halo, cyano, nitro, hydroxy, alkyle en C₁₋₆, -(CH₂)ᵤ-cycloalkyle en C₃₋₇, -O(CH₂)ᵤ-cycloalkyle en C₃₋₇, alcényle en C₂₋₆, alcoxy en C₁₋₆, alkyl hydroxy en C₁₋₆, phényle, alkyle en C₁₋₆ substitué par jusqu'à 5 fluoro et alcoxy en C₁₋₆ substitué par jusqu'à 5 fluoro, lesdits alkyle en C₁₋₆, -(CH₂)ᵤ-cycloalkyle en C₃₋₇, -O(CH₂)ᵤ-cycloalkyle en C₃₋₇, alcényle en C₂₋₆, alcoxy en C₁₋₆, alkyl hydroxy en C₁₋₆ et phényle étant chacun éventuellement substitués par un ou plusieurs **R₂₃**,
chaque **R₂₃** est choisi indépendamment dans le groupe constitué d'halo, cyano, nitro, hydroxy, alkyle en C₁₋₆, -(CH₂)ᵤ-cycloalkyle en C₃₋₇, alcényle en C₂₋₆, alcoxy en C₁₋₆ , alkyl hydroxy en C₁₋₆, phényle, alkyle en C₁₋₆ substitué par jusqu'à 5 fluoro et alcoxy en C₁₋₆ substitué par jusqu'à 5 fluoro, ledit phényle étant éventuellement substitué par un ou plusieurs substituants choisis chacun indépendamment dans le groupe constitué d'halo, cyano, nitro, hydroxy, alkyle en C₁₋₆, -(CH₂)ᵤ-cycloalkyle en C₃₋₇, alcényle en C₂₋₆, alcoxy en C₁₋₆, alkyl hydroxy en C₁₋₆, phényle, alkyle en C₁₋₆ substitué par jusqu'à 5 fluoro et alcoxy en C₁₋₆ substitué par jusqu'à 5 fluoro ;
chaque **R₁₉** représente indépendamment aryle éventuellement substitué par un ou plusieurs substituants choisis chacun indépendamment dans le groupe constitué d'halo, cyano, nitro, hydroxy, alkyle en C₁₋₆ éventuellement substitué par jusqu'à 5 fluoro et alcoxy en C₁₋₆ éventuellement substitué par jusqu'à 5 fluoro ;
chaque **R₂₀** représente indépendamment hydrogène ou aryle éventuellement substitué par un ou plusieurs substituants choisis chacun indépendamment dans le groupe constitué d'halo, cyano, nitro, hydroxy, alkyle en C₁₋₆ éventuellement substitué par jusqu'à 5 fluoro et alcoxy en C₁₋₆ éventuellement substitué par jusqu'à 5 fluoro ; et
dans lequel, lorsque Y représente N**R₈R₉,** alors **R₈** et **R₂** s'assemblent éventuellement pour former un alkyle en C₁₋₃ se liant ensemble en tant que cycle,
à condition que, lorsque **R₈** s'assemble avec **R₂** pour former un alkyle en C₁₋₃ se liant ensemble en tant que cycle, **R₄** ne soit pas substitué par hydroxyle ;
destiné à être utilisé dans le traitement d'une maladie néoplasique chez un sujet.

2. Composé destiné à être utilisé selon la revendication 1, dans lequel un, deux ou trois parmi **R₃, R₄**, **R₅** et **R₆** ne représentent pas hydrogène.

3. Composé destiné à être utilisé selon la revendication 1 ou la revendication 2, dans lequel **R₄** est choisi dans le groupe constitué d'alkyle en C₁₋₆, -(CH₂)ᵤ-cycloalkyle en C₃₋₇, -O(CH₂)ᵤ-cycloalkyle en C₃₋₇, alcényle en C₂₋₆, alcoxy en C₁₋₆, phényle, alkyle en C₁₋₆ substitué par jusqu'à 5 fluoro et alcoxy en C₁₋₆ substitué par jusqu'à 5 fluoro, lesdits alkyle en C₁₋₆, -(CH₂)ᵤ-cycloalkyle en C₃₋₇, -O(CH₂)ᵤ-cycloalkyle en C₃₋₇, alcényle en C₂₋₆, alcoxy en C₁₋₆ et phényle étant chacun éventuellement substitué par un ou plusieurs substituants choisis chacun indépendamment dans le groupe constitué d'alkyl halo en C₁₋₆, -(CH₂)ᵤ-cycloalkyle en C₃₋₇, alcényle en C₂₋₆, alcoxy en C₁₋₆, alkyl hydroxy en C₁₋₆, phényle, alkyle en C₁₋₆ substitué par jusqu'à 5 fluoro et alcoxy en C₁₋₆ substitué par jusqu'à 5 fluoro.

4. Composé destiné à être utilisé selon l'une quelconque des revendications 1 à 3,
dans lequel :
**Y** représente -N**R₈R₉**,
**R₈** représente hydrogène ; et **R₉** représente alkyle en C₁₋₆ éventuellement substitué par un ou plusieurs substituants choisis chacun indépendamment dans le groupe constitué d'hydroxy, **R₁₉** et -O**R₂₀** ;
chaque **R₁₉** représente indépendamment phényle éventuellement substitué par un ou plusieurs substituants choisis chacun indépendamment dans le groupe constitué d'halo, cyano, alkyle en C₁₋₆ éventuellement substitué par jusqu'à 5 fluoro et alcoxy en C₁₋₆ éventuellement substitué par jusqu'à 5 fluoro, ou chaque **R₁₉** représente indépendamment phényle éventuellement substitué par un ou plusieurs substituants choisis chacun indépendamment dans le groupe constitué d'halo, alkyle en C₁₋₆, et alcoxy en C₁₋₆ ; et
chaque **R₂₀** représente indépendamment hydrogène ou phényle éventuellement substitué par un ou plusieurs substituants choisis chacun indépendamment dans le groupe constitué d'halo, cyano, nitro, hydroxy, alkyle en C₁₋₆ éventuellement substitué par jusqu'à 5 fluoro et alcoxy en C₁₋₆ éventuellement substitué par jusqu'à 5 fluoro, ou chaque **R₂₀** représente indépendamment phényle éventuellement substitué par un ou plusieurs substituants choisis chacun indépendamment dans le groupe constitué d'halo, alkyle en C₁₋₆ et alcoxy en C₁₋₆.

5. Composé destiné à être utilisé selon l'une quelconque des revendications 1 à 4, dans lequel **R₂** et **R₈** représentent hydrogène, ou éventuellement lorsque **Y** représente -N**R₈R₉** et que **R₈** et **R₂** s'assemblent pour former un alkyle en C₁₋₃ se liant ensemble en tant que cycle.

6. Composé destiné à être utilisé selon l'une quelconque des revendications 1 à 5, dans lequel **R₉** représente hydrogène,
ou **R₉** représente un alkyle en C₁₋₆ éventuellement substitué par un ou plusieurs substituants choisis chacun indépendamment dans le groupe constitué d'hydroxy, **R₁₉** ou -O**R₂₀** où chaque **R₁₉** représente indépendamment phényle éventuellement substitué par un ou plusieurs substituants choisis chacun indépendamment dans le groupe constitué d'halo, cyano, alkyle en C₁₋₆ éventuellement substitué par jusqu'à 5 fluoro et alcoxy en C₁₋₆ éventuellement substitué par jusqu'à 5 fluoro ; et où chaque **R₂₀** représente indépendamment hydrogène ou phényle éventuellement substitué par un ou plusieurs substituants choisis chacun indépendamment dans le groupe constitué d'halo, cyano, nitro, hydroxy, alkyle en C₁₋₆ éventuellement substitué par jusqu'à 5 fluoro et alcoxy en C₁₋₆ éventuellement substitué par jusqu'à 5 fluoro,
ou **R₉** représente hydrogène ou alkyle en C₁₋₆ éventuellement substitué par un ou plusieurs substituants choisis chacun indépendamment dans le groupe constitué d'hydroxyle, **R₁₉** et -O**R₂₀** où chaque **R₁₉** représente indépendamment phényle éventuellement substitué par un ou plusieurs substituants choisis chacun indépendamment dans le groupe constitué d'halo, cyano, alkyle en C₁₋₆ éventuellement substitué par jusqu'à 5 fluoro et alcoxy en C₁₋₆ éventuellement substitué par jusqu'à 5 fluoro ; et où chaque **R₂₀** représente indépendamment hydrogène ou phényle éventuellement substitué par un ou plusieurs substituants choisis chacun indépendamment dans le groupe constitué d'halo, cyano, nitro, hydroxy, alkyle en C₁₋₆ éventuellement substitué par jusqu'à 5 fluoro et alcoxy en C₁₋₆ éventuellement substitué par jusqu'à 5 fluoro.

7. Composé destiné à être utilisé selon l'une quelconque des revendications 1 à 6,
dans lequel **R₄** est choisi dans le groupe constitué d'alkyle en C₁₋₆, - (CH₂)ᵤ-cycloalkyle en C₃₋₇, -O(CH₂)ᵤ-cycloalkyle en C₃₋₇, alcényle en C₂₋₆, alcoxy en C₁₋₆, phényle, alkyle en C₁₋₆ substitué par jusqu'à 5 fluoro et alcoxy en C₁₋₆ substitué par jusqu'à 5 fluoro, lesdits alkyle en C₁₋₆, -(CH₂)ᵤ-cycloalkyle en C₃₋₇, - O(CH₂)ᵤ-cycloalkyle en C₃₋₇, alcényle en C₂₋₆, alcoxy en C₁₋₆ et phényle, chacun étant éventuellement substitué par un ou plusieurs **R₂₃**,
chaque **R₂₃** est choisi indépendamment dans le groupe constitué d'halo, alkyle en C₁₋₆, -(CH₂)ᵤ-cycloalkyle en C₃₋₇, alcényle en C₂₋₆, alcoxy en C₁₋₆, phényle, alkyle en C₁₋₆ substitué par jusqu'à 5 fluoro et alcoxy en C₁₋₆ substitué par jusqu'à 5 fluoro, ledit phényle étant éventuellement substitué par un ou plusieurs substituants choisis chacun indépendamment dans le groupe constitué d'halo, alkyle en C₁₋₆, -(CH₂)ᵤ-cycloalkyle en C₃₋₇, alcényle en C₂₋₆, alcoxy en C₁₋₆, alkyle en C₁₋₆ substitué par jusqu'à 5 fluoro et alcoxy en C₁₋₆ substitué par jusqu'à 5 fluoro,
ou dans lequel **R₄** est choisi dans le groupe constitué d'alkyle en C₁₋₆, cycloalkyle en C₃₋₇, -O-cycloalkyle en C₃₋₇, phényle, alkyle en C₁₋₆ substitué par jusqu'à 5 fluoro et alcoxy en C₁₋₆ substitué par jusqu'à 5 fluoro, ledit phényle étant éventuellement substitué par un ou plusieurs substituants choisis chacun indépendamment dans le groupe constitué d'halo, alkyle en C₁₋₆, alcoxy en C₁₋₆, alkyle en C₁₋₆ substitué par jusqu'à 5 fluoro et alcoxy en C₁₋₆ substitué par jusqu'à 5 fluoro.

8. Composé destiné à être utilisé selon l'une quelconque des revendications 1 à 7, dans lequel **Y** représente -N**R₈R₉** et **R₈** et **R₂** s'assemblent pour former un alkyle en C₁₋₃ se liant ensemble en tant que cycle.

9. Composé destiné à être utilisé selon l'une quelconque des revendications 1 à 8, dans lequel **R₂** représente hydrogène ou est choisi dans le groupe constitué d'alkyle en C₁₋₆, cycloalkyle en C₃₋₇ et phényle, ledit alkyle en C₁₋₆ étant éventuellement substitué par un ou plusieurs halo.

10. Composé destiné à être utilisé selon l'une quelconque des revendications 1 à 9, dans lequel **R₉** représente hydrogène ou alkyle en C₁₋₆ éventuellement substitué par un ou plusieurs substituants choisis chacun indépendamment dans le groupe constitué d'hydroxyle, **R₁₉** et -O**R₂₀** ;
chaque **R₁₉** représente indépendamment phényle éventuellement substitué par un ou plusieurs substituants choisis chacun indépendamment dans le groupe constitué d'halo, cyano, alkyle en C₁₋₆ éventuellement substitué par jusqu'à 5 fluoro et alcoxy en C₁₋₆ éventuellement substitué par jusqu'à 5 fluoro ; et
chaque **R₂₀** représente indépendamment hydrogène ou phényle éventuellement substitué par un ou plusieurs substituants choisis chacun indépendamment dans le groupe constitué d'halo, cyano, nitro, hydroxy, alkyle en C₁₋₆ éventuellement substitué par jusqu'à 5 fluoro et alcoxy en C₁₋₆ éventuellement substitué par jusqu'à 5 fluoro.

11. Composé destiné à être utilisé selon l'une quelconque des revendications 1 à 10, dans lequel le composé de formule (I) est choisi dans le groupe constitué de : 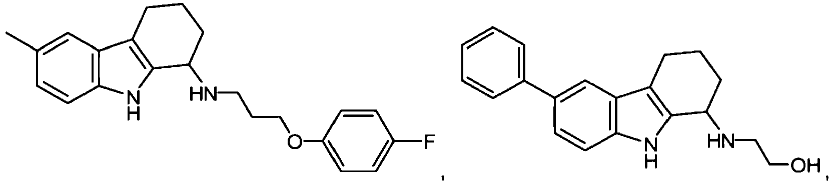 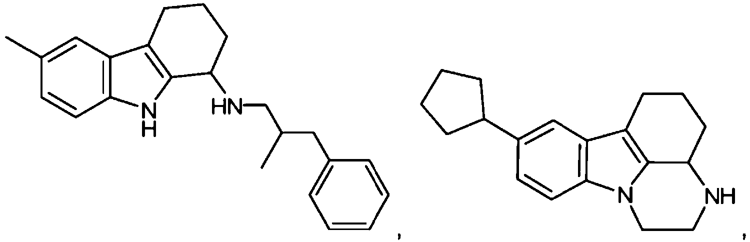 et

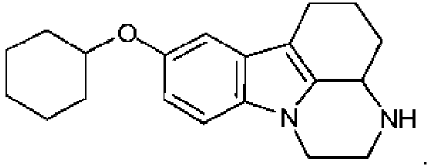

12. Composé destiné à être utilisé selon les revendications 1 à 11, dans lequel l'inhibiteur spécifique de Cdc42 est CASIN.

13. Composé destiné à être utilisé selon l'une quelconque des revendications 1 à 12, dans lequel la maladie néoplasique est choisie dans le groupe constitué de cancer du côlon et cancer du pancréas.

14. Composé destiné à être utilisé selon l'une quelconque des revendications 1 à 12, dans lequel l'utilisation comprend en outre l'administration d'un ou de plusieurs parmi agent anticancéreux, agent antinéoplasique ou agent modulant l'apoptose qui est un inhibiteur de point de contrôle immunitaire qui cible PD-1, PD-L1 ou CTLA-4, un composé chimiothérapeutique, une radiothérapie ou une intervention chirurgicale.

15. Composé destiné à être utilisé selon l'une quelconque des revendications 1 à 12, dans lequel l'inhibiteur de point de contrôle immunitaire qui cible PD-1, PD-L1 ou CTLA-4 est un anticorps qui est choisi dans le groupe constitué de pembrolizumab, nivolumab, cémiplimab, atézolizumab, avélumab, durvalumab et ipilimumab.
